(19) Europäisches Patentamt
European Patent Office
Office européen des brevets

(11) EP 0 515 264 B1

# (12) FASCICULE DE BREVET EUROPEEN

(45) Date de publication et mention de la délivrance du brevet:
**11.09.1996 Bulletin 1996/37**

(51) Int Cl.[6]: **C07J 33/00**, C07J 21/00, C07J 13/00

(21) Numéro de dépôt: **92401362.6**

(22) Date de dépôt: **20.05.1992**

(54) **Nouveaux dérivés stéroides du pregna-4,9(11),17(20)-triène-3-one, leur préparation, leur préparation, leur application à la préparation de composés stéroides de type pregna-4,9(11),16-trièn-3,20-dione et nouveaux intermédiaires**

Neue Steroidderivate von Pregna-4,9(11),17(20)-trien-3-on, ihre Herstellung, ihre Verwendung zur Herstellung von Derivaten des Typs Pregna-4,9(11),16-trien-3,20-dion und neue Zwischenprodukte

New steroid derivatives of pregna-4,9(11),17(20)-trien-3-on, their preparation, their use in the preparation of derivatives of pregna-4,9(11),16-trien-3,20-dione and intermediates therefor

(84) Etats contractants désignés:
**AT BE CH DE DK ES FR GB GR IT LI LU NL PT SE**

(30) Priorité: **23.05.1991 FR 9106202**

(43) Date de publication de la demande:
**25.11.1992 Bulletin 1992/48**

(73) Titulaire: **ROUSSEL UCLAF**
**93230 Romainville (FR)**

(72) Inventeurs:
- **Brion, Francis**
  **F-93220 Gagny (FR)**
- **Buendia, Jean**
  **F-94170 Le Perreux S/ Marne (FR)**
- **Diolez, Christian**
  **F-91000 Palaiseau (FR)**
- **Vivat, Michel**
  **F-77400 Lagny S/ Marne (FR)**

(74) Mandataire: **Vieillefosse, Jean-Claude et al**
**ROUSSEL UCLAF,**
**Département des Brevets,**
**111, Route de Noisy**
**93235 Romainville Cédex (FR)**

(56) Documents cités:

| | |
|---|---|
| **EP-A- 0 061 416** | **EP-A- 0 127 216** |
| **EP-A- 0 127 217** | **EP-A- 0 306 969** |
| **EP-A- 0 336 521** | **DE-A- 2 148 631** |
| **FR-A- 2 451 381** | **GB-A- 2 022 590** |
| **US-A- 4 189 430** | |

Remarques:
Le dossier contient des informations techniques présentées postérieurement au dépôt de la demande et ne figurant pas dans le présent fascicule.

EP 0 515 264 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

## Description

Nouveaux dérivés stéroïdes du pregna-4,9(11), 17(20)-trièn-3-one, leur préparation, leur application à la préparation du composés stéroïdes du type pregna-4,9(11), 16-triène-3,20-dione et nouveaux intermédiaires.

La présente invention a pour objet de nouveaux dérivés stéroïdes du pregna-4,9(11),17(20)-trièn-3-one, leur préparation, leur application à la préparation de composés stéroïdes de type pregna-4,9(11),16-triène-3,20-dione et nouveaux intermédiaires.

L'invention a ainsi pour objet les composés de formule (I) :

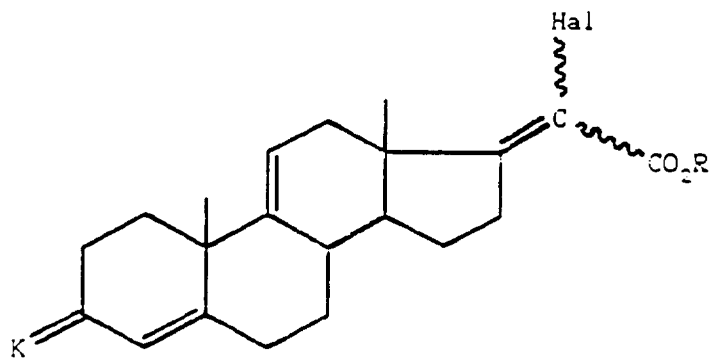

(I)

dans laquelle Hal représente un atome de chlore ou de brome, R représente un radical alkyle renfermant de 1 à 6 atomes de carbone, un radical aralkyle renfermant de 7 à 15 atomes de carbone ou un reste silylé, K représente un groupement protecteur du radical oxo de formule :

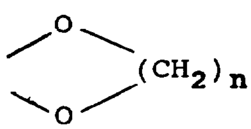 , 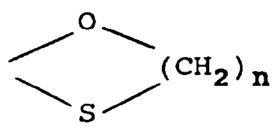 ou 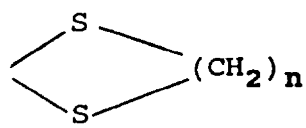

dans laquelle n est égal à 2 ou 3 et les traits ondulés symbolisent l'une quelconque des formes isomères ou leurs mélanges.

Lorsque R représente un radical alkyle, il s'agit de préférence d'un radical méthyle, éthyle, propyle, isopropyle, butyle, sec-butyle, tert-butyle, pentyle ou hexyle.

Lorsque R représente un radical aralkyle, il s'agit de préférence d'un radical benzyle ou phénéthyle.

Lorsque R représente un reste silyle, il s'agit d'un reste trialkylsilyle, tel que triméthylsilyle, tert-butyldiméthylsilyle ou encore d'un reste triphénylsilyle ou diphényltert-butylsilyle.

L'invention a plus particulièrement pour objet les composés de formule (I) telle que définie ci-dessus, dans laquelle Hal représente un atome de chlore, R représente un radical alkyle renfermant de 1 à 3 atomes de carbone et K représente un groupement de formule

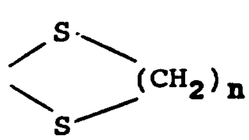

dans laquelle n est égal à 2 ou 3,

et tout particulièrement le 20-chloro 3,3-[(1,2-éthanediyl) bis(thio)] pregna-4,9(11),17(20)-trièn-21-oate de méthyle.

L'invention a également pour objet un procédé de préparation des composés de formule (I) telle que définie plus haut, caractérisé en ce que l'on traite le composé de formule (II) :

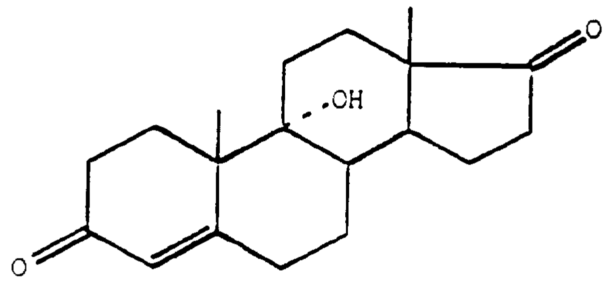

(II)

par l'anhydride acétique en présence d'un acide fort puis par un agent d'hydrolyse acide, pour obtenir le composé de formule (III) :

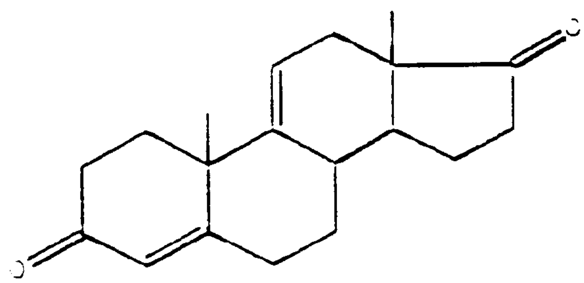

(III)

dont on bloque sélectivement la fonction 3-oxo par action d'un diol, d'un thiol ou d'un dithiol de formule $HO-(CH_2)_n-OH$, $HO-(CH_2)_n-SH$ ou $HS-(CH_2)_n-SH$ dans laquelle n est défini comme précédemment pour obtenir un composé de formule (IV) :

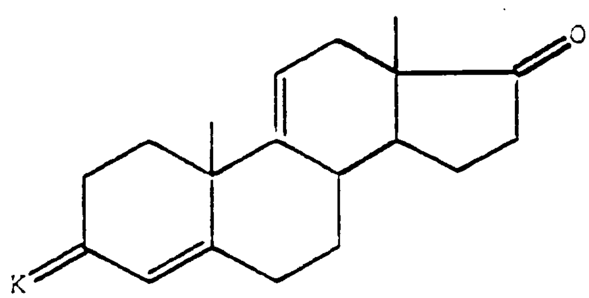

(IV)

dans laquelle K est défini comme précédemment sur lequel l'on fait réagir un composé de formule :

$$Hal_3C\text{-}CO_2R$$

dans laquelle Hal et R sont définis comme précédemment, en présence du zinc et d'un acide de Lewis pour obtenir le composé de formule (I) attendu.

L'acide fort en présence duquel on fait réagir l'anhydride acétique sur le produit de formule (II) est notamment l'acide paratoluène sulfonique, l'acide méthane sulfonique, l'acide perchlorique ou encore l'acide chlorhydrique, l'acide bromhydrique ou l'acide sulfurique. Il est de préférence utilisé en quantité catalytique.

L'agent d'hydrolyse de l'acétate intermédiaire formé in situ est un acide aqueux, notamment l'acide chlorhydrique, l'acide bromhydrique ou l'acide sulfurique ou, plus particulièrement, l'acide formique.

Le blocage de la cétone en position 3 est réalisé par action du diol, du thiol ou du dithiol en milieu acide, plus particulièrement de l'éthane dithiol en présence d'acide chlorhydrique ou bromhydrique concentré, en quantité catalytique, ou encore en présence d'un acide de Lewis tel que le chlorure de zinc, le tétrachlorure de titane ou le trifluorure de bore de préférence sous forme d'éthérate.

L'acide de Lewis utilisé dans la réaction du composé de formule (IV) avec le trihaloacétate est par exemple, le chlorure de zinc, le chlorure d'aluminium, le chlorure de diéthylaluminium, ou, de préférence, le tétrachlorure de titane.

On opère de préférence au sein d'un éther cyclique tel que le tétrahydrofuranne ou le dioxanne.

L'invention a aussi pour objet l'application des composés de formule (I) telle que définie précédemment, à la préparation des composés de formule (A) :

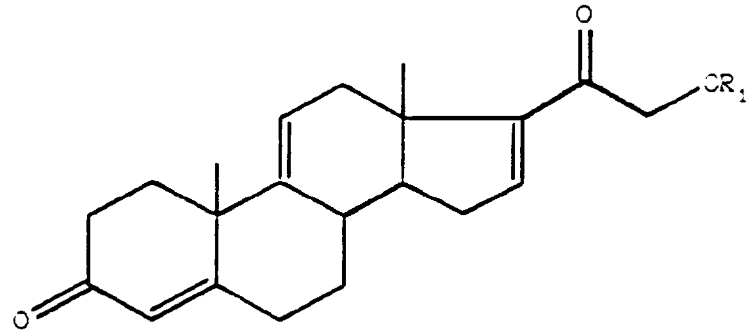

(A)

dans laquelle $R_1$ représente un radical acyle renfermant de 1 à 8 atomes de carbone, caractérisé en ce que l'on traite un composé de formule (I), en milieu basique, par un phénol de formule :

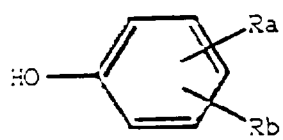

dans laquelle $R_a$ et $R_b$, identiques ou différents, représentent un atome d'hydrogène, un radical alkyle renfermant de 1 à 4 atomes de carbone, un radical alkoxy renfermant de 1 à 4 atomes de carbone ou un radical hydroxy, pour obtenir un composé de formule (V) :

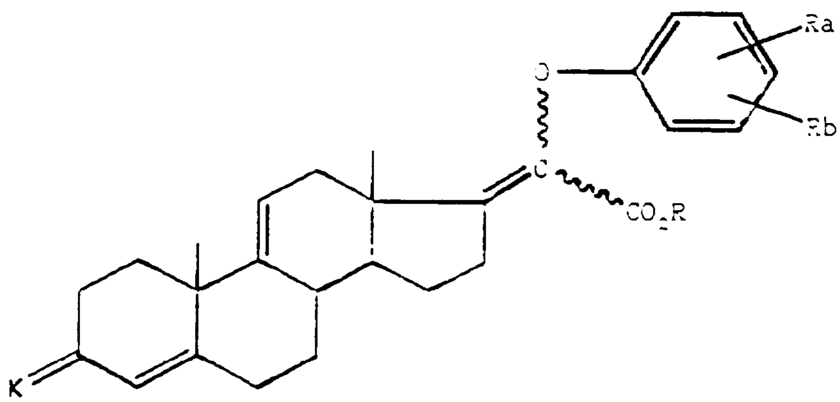

(V)

dans laquelle K, R, $R_a$ et $R_b$ sont définis comme précédemment, que l'on soumet à l'action d'un agent réducteur, pour obtenir un composé de formule (VI) :

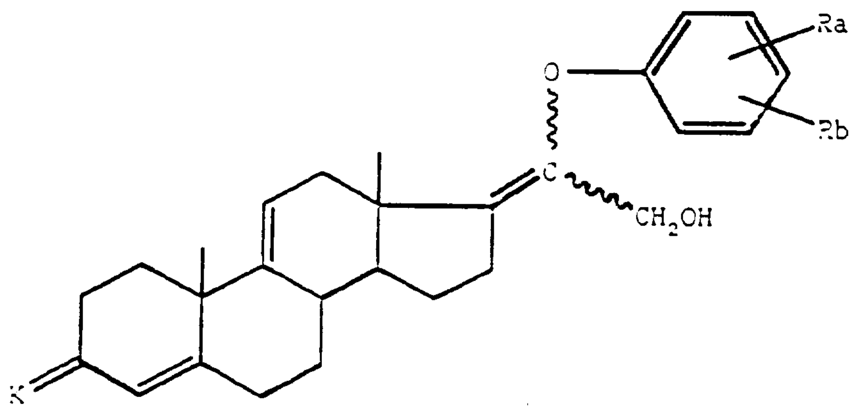

(VI)

dont on déprotège la fonction 3-oxo, puis que l'on traite par un agent d'époxydation, pour obtenir l'époxyde correspondant en position 17,20, que l'on hydrolyse en milieu acide, pour obtenir le composé de formule (VII) :

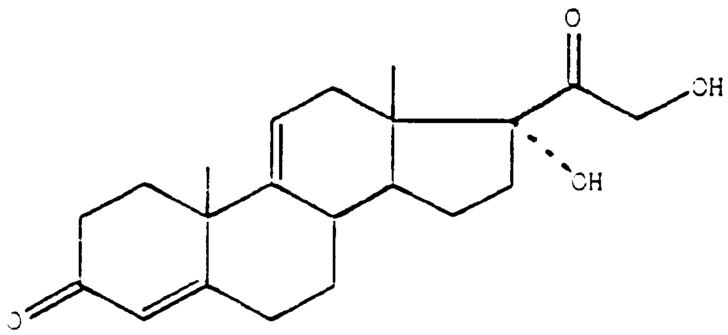

(VII)

dont on acyle les fonctions hydroxy pour obtenir le composé de formule (VIII) :

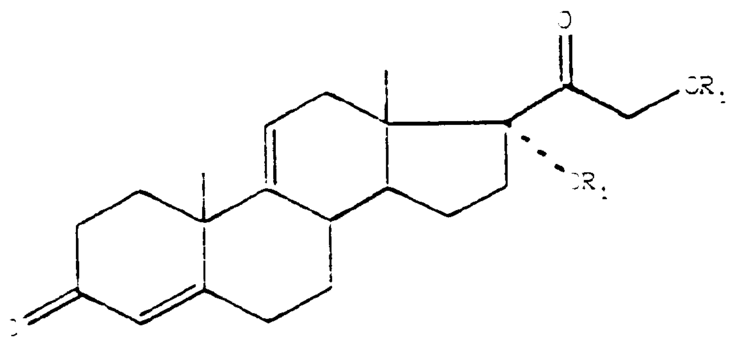

(VIII)

dans laquelle $R_1$ est défini comme précédemment, que l'on soumet à l'action d'un agent d'élimination du radical 17α-$OR_1$, pour obtenir le composé de formule (A) attendu.

L'action du phénol sur le composé de formule (I) est réalisée en présence d'une base qui peut être par exemple un hydroxyde ou un carbonate alcalin ou alcalino terreux, notamment de sodium, potassium, baryum ou calcium, un hydrure, un alcoolate ou un amidure alcalin, notamment de sodium, potassium ou lithium ou encore un alkyllithium, notamment le butyl-lithium.

On opère au sein d'un solvant organique, par exemple une cétone telle que l'acétone ou la méthyléthylcétone, le cas échéant en mélange avec un solvant halogéné tel que le chlorure de méthylène ou avec un éther tel que le dioxanne ou le tétrahydrofuranne.

Lorsque $R_a$ et $R_b$ représentent un radical alkyle, il s'agit d'un radical éthyle, propyle linéaire ou ramifié, butyle linéaire ou ramifié, ou de préférence méthyle.

Lorsque $R_a$ et $R_b$ représentent un radical alkoxy, il s'agit d'un radical éthoxy, propoxy linéaire ou ramifié, butoxy linéaire ou ramifié ou, de préférence méthoxy.

Les valeurs tout particulièrement préférées pour $R_a$ et $R_b$ sont hydrogène, hydroxy et méthyle.

L'agent réducteur peut être notamment un hydrure, par exemple l'hydrure double de lithium et d'aluminium, l'hydrure de diéthylsodiumaluminium, l'hydrure de diisobutylaluminium ou encore le dihydrobis(2-méthoxy éthoxy) aluminate de sodium. On opère par exemple dans le toluène ou le tétrahydrofuranne.

L'agent réducteur peut encore notamment être un borohydrure alcalin, par exemple le borohydrure de sodium, catalysé le cas échéant, par un sel de lithium, ou le borohydrure de lithium.

La déprotection de la fonction 3-oxo bloquée sous forme de cétal est réalisée de préférence par action d'un acide en présence d'eau. Dans le cas d'un dithiocétal, elle est de préférence réalisée par action de l'iode en présence d'une base, par exemple un bicarbonate alcalin, ou par action de l'iode en quantité catalytique, en présence d'un agent oxydant, par exemple l'eau oxygénée, par action de l'iodure de méthyle, de l'acide glyoxylique, ou encore de sels de métaux, tels le mercure ou le cadmium. On peut, en général, opérer dans un solvant tel qu'un alcanol inférieur, par exemple le méthanol ou l'éthanol, en mélange avec un solvant halogéné, par exemple le chlorure de méthylène, en présence d'eau. Dans le cas d'un oxathiolane, la déprotection est effectuée par exemple par un sel mercurique tel que le chlorure mercurique en présence d'un tampon acide acétique/acétate de potassium à 100°C environ, par le Nickel de Raney dans les mêmes conditions que ci-dessus ou par le mélange acide chlorhydrique-acide acétique à chaud.

L'agent d'époxydation peut être un peracide tel que l'acide métachloroperbenzoïque, l'acide perphtalique, l'acide pertungsténique ou encore l'eau oxygénée utilisée seule ou en présence d'hexachloro ou d'hexafluoroacétone.

L'agent d'époxydation peut encore être un hydroperoxyde tel que l'hydroperoxyde de tert-butyle, utilisé en présence d'acétylacétonate de vanadium en quantité catalytique.

On opère de préférence au sein d'un solvant organique tel que le toluène, le chlorure de méthylène, le chloroforme, le tétrahydrofuranne, le dioxanne ou encore l'acétate d'éthyle.

L'hydrolyse de l'époxyde en 17,20 est effectuée par action d'un acide aqueux, l'acide étant notamment un acide minéral tel que l'acide chlorhydrique, l'acide sulfurique ou l'acide nitrique. On peut également opérer en milieu tamponné.

L'agent d'acylation est de préférence un anhydride ou un chlorure d'acide formique, acétique, propionique, butyrique ou benzoïque et on opère en présence d'une base, par exemple une amine telle que la triéthylamine, la pyridine ou la diméthylaminopyridine, un acétate ou un carbonate alcalin.

L'agent d'acylation est préférentiellement l'anhydride acétique ou le chlorure d'acétyle.

L'acylation en 17 et 21 peut s'accompagner d'une acylation partielle sur la forme énol en 3. Le dérivé 3-acylé peut alors être facilement hydrolysé par un acide tel que l'acide chlorhydrique aqueux en opérant par exemple dans le chlorure de méthylène.

L'agent d'élimination du groupement $-OR_1$ est une base qui peut être par exemple un sel alcalin, alcalino terreux ou d'amine, notamment un sel de sodium, de potassium de l'acide correspondant au radical acyle. On opère dans un solvant polaire tel que le diméthylformamide, le diméthylsulfoxyde ou encore l'hexaméthylphosphotriamide, à une température comprise de préférence entre 90 et 140°C.

L'invention a encore pour objet l'application des composés de formule (I) telle que définie précédemment à la préparation des composés de formule (A), telle que définie précédemment , caractérisée en ce que l'on traite un composé de formule (I), en milieu basique par un sulfinate alcalin de formule :

$$R_2\text{-}SO_2\text{-}Q$$

dans laquelle $R_2$ représente un radical méthyle, phényle, tolyle ou xylyle, en présence d'une base, pour obtenir après saponification et décarboxylation, un composé de formule (IX) :

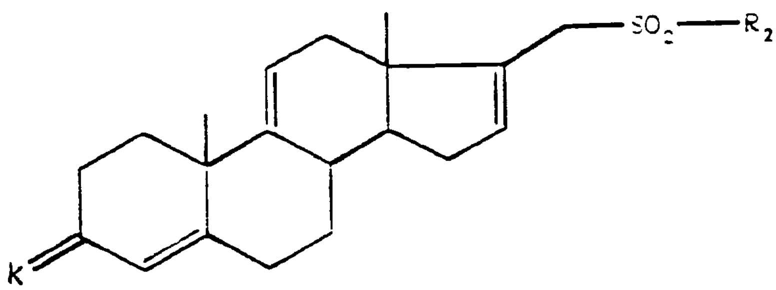

(IX)

dans laquelle K et $R_2$ sont définis comme précédemment, que l'on soumet à l'action du formaldéhyde en présence d'une base, pour obtenir un composé de formule (X) :

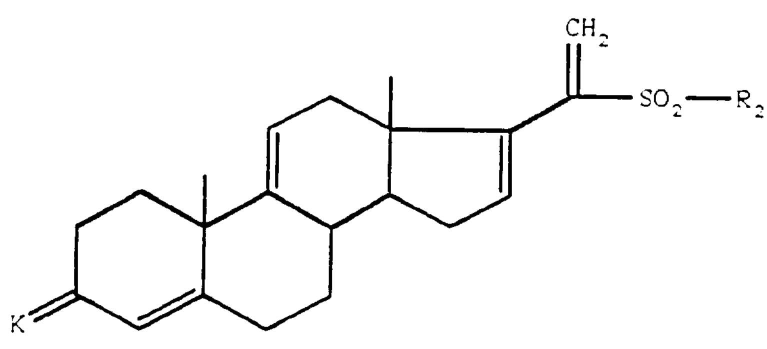

(X)

que l'on soumet à l'action d'un agent d'époxydation en milieu alcalin, pour obtenir un composé de formule (XI) :

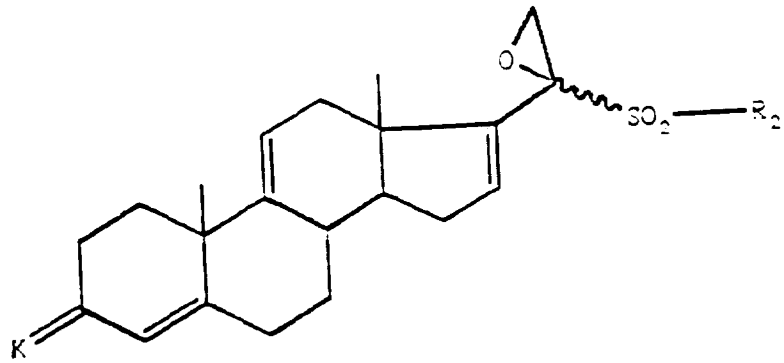

(XI)

dont on ouvre la fonction époxyde en milieu basique et en présence d'ions $R_1O^{\ominus}$, dans lesquels $R_1$ est défini comme précédemment, pour obtenir un composé de formule (XII) :

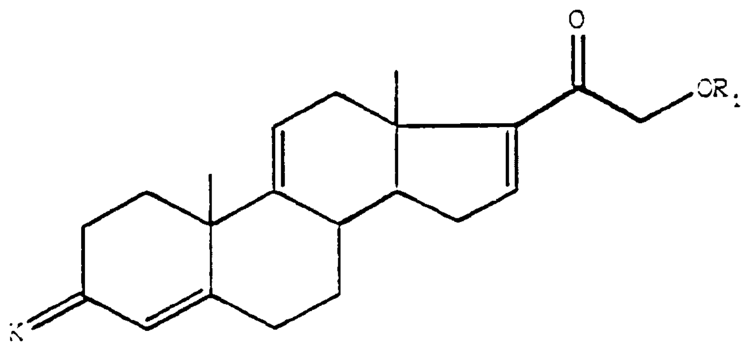

(XII)

dont on déprotège la fonction 3-oxo, pour obtenir le composé de formule (A) attendu.

L'action du sulfinate alcalin est réalisée en présence d'un agent basique assurant dans les conditions de la réaction la saponification de l'ester. Cet agent basique peut être par exemple un carbonate alcalin, tel le carbonate de sodium ou de potassium, ou un hydroxyde alcalin, tel la soude ou la potasse et est utilisé de préférence en excès. On opère au sein .d'un solvant polaire aprotique, par exemple le diméthylformamide ou le diméthylsulfoxyde. La décarboxylation s'opère de préférence à une température voisine de 100°C. On opère également avantageusement en phase homogène.

Le sulfinate alcalin est préférentiellement le toluènesulfinate de sodium.

La base en présence de laquelle on condense le formaldéhyde est une base faible telle qu'un carbonate ou bicarbonate de sodium ou de potassium. On peut encore utiliser un hydroxyde alcalin ou alcalinoterreux. On opère de préférence au sein du diméthylformamide ou du diméthylsufoxyde, à une température voisine de 60°C.

L'agent d'époxydation est de préférence l'eau oxygénée. On opère avantageusement en présence d'une base telle qu'un hydroxyde ou un carbonate alcalin ou alcalinoterreux, par exemple de sodium, de potassium ou de baryum. L'agent d'époxydation peut encore être un peracide en milieu alcalin, l'hydroperoxyde de tert-butyle catalysé ou non par des métaux tels que le vanadium, le tungstène ou le titane, ou encore l'oxone. On opère avantageusement au sein d'un solvant tel que le dioxanne, le tétrahydrofuranne ou encore le mélange méthanol-chlorure de méthylène.

L'ouverture de la fonction époxyde est effectuée à l'aide d'un sel alcalin, d'ammonium ou d'amine de l'acide $R_1OH$.

Dans des conditions préférées de mise en oeuvre de la réaction, on opère à l'aide d'un acétate tel que l'acétate de sodium, de potassium, d'ammonium ou plus généralement, d'alkylamines, ou encore d'un mélange acide acétique-triéthylamine. On peut opérer au sein d'un solvant organique tel que le diméthylformamide, le diméthylsulfoxyde, la méthyléthylsulfone ou encore le polyéthylèneglycol, de préférence en chauffant légèrement le milieu réactionnel.

L'ouverture de la fonction époxyde peut encore être effectuée à l'aide d'une résine à caractère basique et, de préférence, une résine sous forme acétate.

La déprotection de la fonction 3-oxo bloquée est réalisée dans les conditions rappelées plus haut.

L'invention a encore pour objet l'application des composés de formule (I) telle que définie précédemment, à la préparation des composés de formule (A), telle que définie précédemment, caractérisée en ce que l'on traite un composé de formule (I) par un sulfinate alcalin de formule $R_2SO_2Q$, telle que définie précédemment, en présence d'une base, pour obtenir un composé de formule (XIII) :

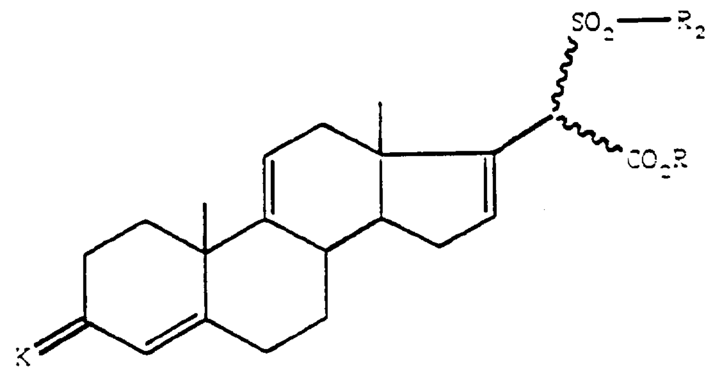

**(XIII)**

dans laquelle R, $R_2$ et K sont définis comme précédemment, que l'on traite par un agent d'halogénation en présence d'une base, puis que l'on saponifie et décarboxyle pour obtenir un composé de formule (XIV) :

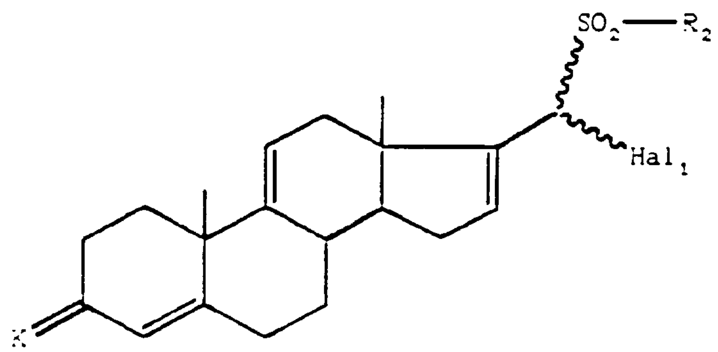

**(XIV)**

dans laquelle $Hal_1$ représente un atome d'halogène, que l'on traite par le formaldéhyde en présence d'une base, pour obtenir le composé de formule (XI) telle que définie précédemment, que l'on traite comme décrit précédemment, pour obtenir le composé de formule (A) désiré.

L'action du sulfinate alcalin, qui est de préférence le tolyl sulfinate de sodium, est effectuée en présence d'un agent basique sauvegardant, dans les conditions de la réaction, la fonction ester. Cet agent basique peut être un carbonate ou un hydroxyde alcalin, notamment de sodium ou de potassium, ou encore l'imidazole, la morpholine, la N-méthyl morpholine, la triéthylamine, la pipéridine, la N-méthyl pipéridine, la pyrrolidine, la N-méthylpyrrolidine, le phosphate tripotassique, l'alumine, la triéthanolamine, la pipérazine, la N,N-diméthyl pipérazine, l'hexaméthyldisilazane, le diazabicyclooctane, la diméthylpropylène urée ou encore l'hexaméthylène tétramine. On peut avantageusement utiliser un défaut de base.

On opère de préférence au sein d'un solvant polaire aprotique tel que le diméthylformamide, le diméthylsulfoxyde, le diméthylacétamide ou la N-méthyl pyrrolidone ou encore dans l'acétonitrile ou la méthylisobutylcétone, à une température pouvant aller de 50 à 120°C.

L'halogénation du composé de formule (XIII) est effectuée notamment par l'intermédiaire d'un hypohalogénite alcalin, d'un N-halogéno succinimide ou d'un halogène. On utilise de préférence l'hypochlorite ou l'hypobromite de sodium, Hall est donc de préférence un atome de chlore ou de brome. La réaction est effectuée en présence d'une base telle qu'un hydroxyde alcalin ou alcalinoterreux, notamment l'hydroxyde de sodium ou de potassium, ou encore un carbonate ou un bicarbonate alcalin, notamment de sodium ou de potassium et l'on opère au sein d'un solvant qui peut être par exemple un éther tel que le dioxanne ou le tétrahydrofuranne ou encore un solvant halogéné, de préférence le chlorure de méthylène ou le dichloréthane, à température ambiante ou inférieure. On peut opérer en présence d'un catalyseur de transfert de phase, par exemple le chlorure de triéthylbenzylammonium ou le bromure de tétrabutylammonium.

La saponification et la décarboxylation de l'ester sont de préférence effectuées par action d'une base forte, par exemple un hydroxyde alcalin ou alcalinoterreux tel que l'hydroxyde de sodium ou de potassium, ou encore de calcium ou de baryum, le cas échéant en présence d'un alcanol tel que le méthanol ou l'éthanol et en chauffant éventuellement légèrement la solution, puis par action d'un acide minéral, par exemple l'acide chlorhydrique, sulfurique, bromhydrique, nitrique ou encore phosphorique.

Il est à noter que la saponification et la décarboxylation peuvent intervenir déjà partiellement lors de l'étape d'halogénation du composé de formule (XIII), celle-ci s'effectuant en milieu basique.

L'action du formaldéhyde sur le composé de formule (XIV) génère in situ une halohydrine qui se transforme en l'époxyde désiré. On opère en présence d'une base forte qui peut être en particulier un hydroxyde ou un carbonate alcalin, par exemple de sodium ou de potassium, ou encore un hydroxyde de tétraméthyl ou tétraéthyl ammonium au sein d'un solvant organique qui est de préférence le chlorure de méthylène, et en présence d'un agent de transfert de phase, par exemple, l'un de ceux évoqués plus haut.

L'invention a aussi pour objet l'une ou l'autre des applications telles que définies précédemment, caractérisée en ce que l'on traite le composé de formule (XI) par un halogénure alcalin pour obtenir le composé de formule (XV) :

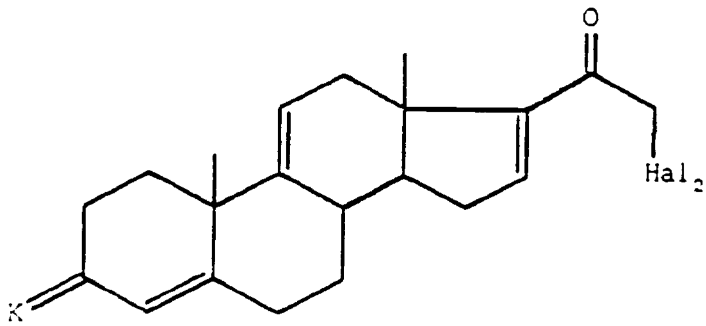

(XV)

dans laquelle $Hal_2$ représente un atome de chlore, de brome ou d'iode et K est défini comme précédemment, que l'on soumet à l'action d'un agent d'acyloxylation en milieu alcalin, pour obtenir le composé de formule (XII) telle que définie précédemment, puis poursuit la synthèse comme décrit précédemment.

L'halogénure alcalin peut être un chlorure, bromure ou iodure de lithium, sodium ou potassium. On utilise de préférence le bromure de lithium.

On opère au sein d'un solvant organique qui peut être un alcanol, par exemple le méthanol, l'éthanol, l'isopropanol, ou un solvant polaire aprotique, par exemple le diméthylformamide ou le diméthylsulfoxyde.

L'agent d'acyloxylation est un sel alcalin, d'ammonium ou d'amine de l'acide $R_1OH$.

On utilise préférentiellement un acétate, par exemple de sodium, de potassium, d'ammonium ou d'alkyl amines ou le mélange acide acétique triéthylamine. On opère au sein d'un solvant pouvant être notamment le diméthylformamide, le diméthylsulfoxyde, l'acétone, la méthyléthylcétone ou le chlorure de méthylène. L'acétate de potassium dans le diméthylformamide est particulièrement préféré. On peut opérer en présence d'acide acétique et d'eau. On peut encore citer l'acétate de potassium dans le chlorure de méthylène en présence d'un catalyseur de transfert de phase tel que l'un de ceux évoqués précédemment et d'eau.

L'invention a encore pour objet l'application des composés de formule (I) telle que définie précédemment à la préparation des composés de formule (A), telle que définie précédemment, caractérisée en ce que l'on traite un composé de formule (I) par un agent réducteur, pour obtenir un composé de formule (XVI) :

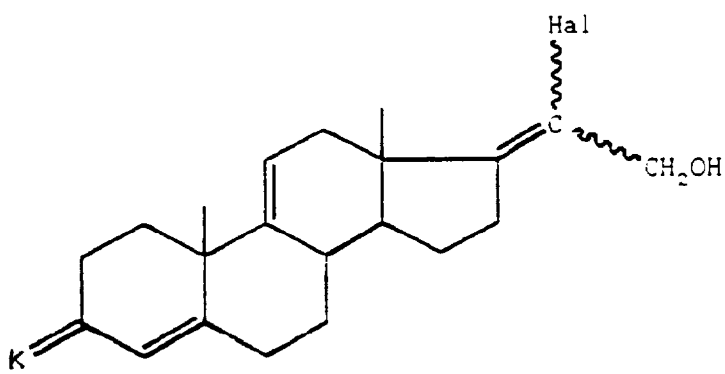

(XVI)

dans laquelle K et Hal sont définis comme précédemment, que l'on soumet à l'action d'un agent oxydant, pour obtenir un composé de formule (XVII) :

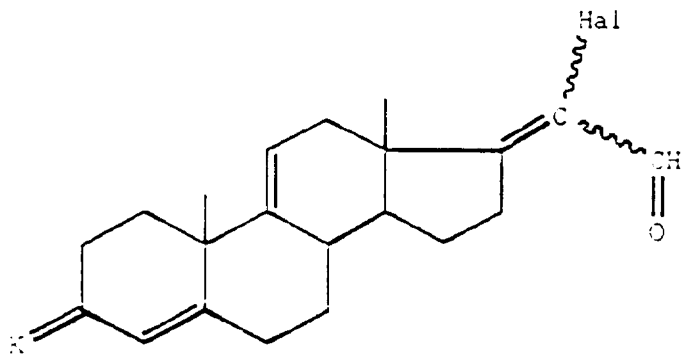

(XVII)

que l'on soumet à l'action d'un agent d'acyloxylation pour obtenir un composé de formule (XII) telle que définie précédemment, puis poursuit la synthèse comme décrit précédemment.

Les conditions de la réduction du composé de formule (I) sont identiques à celles qui ont été précisées plus haut pour la réduction du composé de formule (V).

L'agent oxydant que l'on fait réagir avec le composé de formule (XVI) peut être par exemple le complexe pyridine $-SO_3$ en opérant dans le diméthylsulfoxyde et avantageusement, en présence d'une base faible, par exemple la triéthylamine ou peut être le dicyclohexylcarbodiimide, en opérant dans le diméthylsulfoxyde en présence d'acide phosphorique, ou encore, plus généralement, il peut être tout agent oxydant connu de l'homme de métier pour oxyder une fonction alcool en fonction aldéhyde.

L'agent d'acyloxylation que l'on fait agir sur le composé de formule (XVII) peut être l'un de ceux mentionnés plus haut pour l'acyloxylation des composés de formule (XV).

L'invention a enfin pour objet, à titre de produits industriels nouveaux et notamment à titre de produits intermédiaires nécessaire à l'une ou l'autre des applications des composés de formule (I) telles que définies précédemment ;

- les composé de formule (B) :

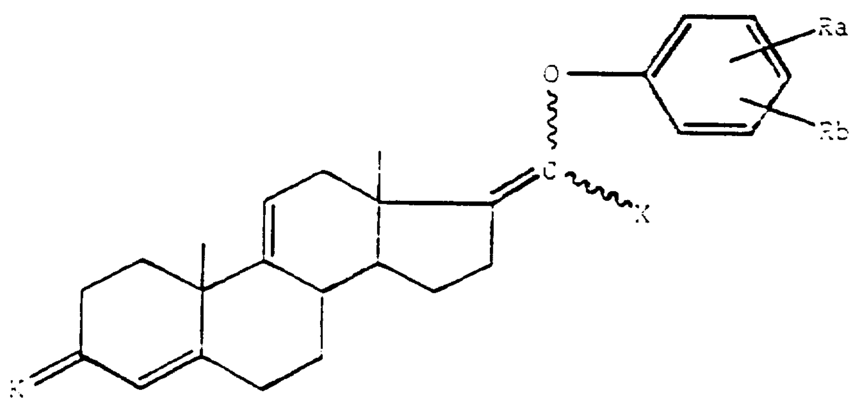

(B)

dans laquelle K, $R_a$ et $R_b$ sont définis comme précédemment et X représente un radical $CH_2OH$ ou $CO_2R$, R étant défini comme précédemment ;

- les composés de formule (C) :

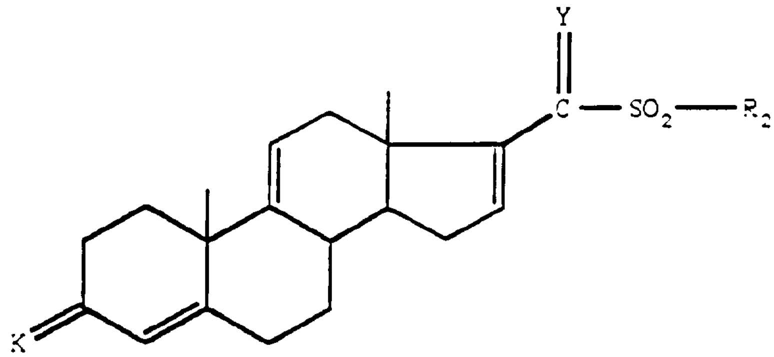

(C)

dans laquelle K et $R_2$ sont définis comme précédemment et le groupement >C=Y représente un groupement $>CH_2$, $>C=CH_2$ ou

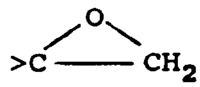 ;

- les composés de formule (XII) telle que définie précédemment ;
- les composés de formule (D) :

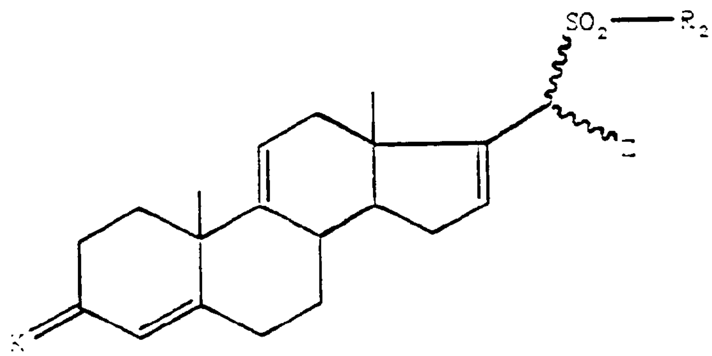

(D)

dans laquelle K et $R_2$ sont définis comme précédemment et Z représente un radical $CO_2R$, R étant défini comme précédemment, ou un atome d'halogène ;

- les composés de formule (XV) telle que définie précédemment ;
- les composés de formule (E) :

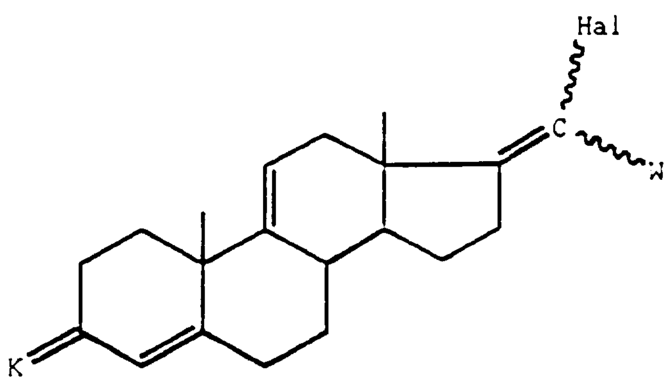

(E)

dans laquelle K et Hal sont définis comme précédemment et W représente un radical $-CH_2OH$.

Le composé de formule (A) est un intermédiaire très utile dans la synthèse de divers composés thérapeutiquement actifs, comme décrit, par exemple, dans le brevet français 1 241 109.

Le composé de formule (II) est décrit, par exemple, dans le brevet US 3 023 229. Le document EP- 336,521 décrit

l'utilisation des dérivés 9-alpha hydroxy, 17-methylène des stéroïdes dans la synthèse des corticostéroïdes.

Les exemples suivants illustrent l'invention sans toutefois la limiter.

**EXEMPLE 1 : 20-chloro 3,3-[(1,2-éthanediyl) bis(thio)] pregna-4,9(11),17(20)-trièn-21-oate de méthyle**

STADE A : Androsta-4,9(11)-diène 3,17-dione

On mélange sous gaz inerte, 500 g de 9alpha-hydroxy androst-4-ène 3,17-dione et 1 1 d'anhydride acétique puis ajoute à température ambiante 50 g d'acide paratoluène sulfonique. On agite pendant 3 h puis ajoute lentement, à température ambiante, 500 $cm^3$ d'acide formique. On agite pendant 16 heures, verse lentement le mélange réactionnel dans 3,5 1 d'eau et agite pendant 2 h à température ambiante. On essore, lave à l'eau, sèche et obtient 463,9 g de produit attendu.

alpha $^{20}_{D}$= + 212° (c = 1 % DMF).

STADE B : 3,3-[(1,2-éthanediyl) bis(thio)] androsta-4,9(11)-dièn-17-one.

On mélange sous gaz inerte, 3 g de produit obtenu au stade A, 30 $cm^3$ de méthanol et 1 $cm^3$ d'éthane dithiol. On ajoute lentement à 20/25°C, 0,1 $cm^3$ d'acide chlorhydrique 22°Bé. On refroidit à 0/+ 5°C, agite 1 h, essore les cristaux, les lave au méthanol, puis à l'eau et les sèche. On obtient

3,65 g de produit attendu. F = 179°C.
Spectre IR ($CHCl_3$)
Absorption à 1733 et 1405 $cm^{-1}$ (17 céto)
Absorption à 1645 $cm^{-1}$ (C=C delta4).

STADE C : 20-chloro 3,3-[(1,2-éthanediyl) bis(thio)] pregna-4,9(11),17(20)-trièn-21-oate de méthyle

On introduit sous agitation et gaz inerte, 21,8 g de poudre de zinc dans 150 $cm^3$ de tétrahydrofuranne, puis ajoute lentement à -15/-20°C, 13,7 $cm^3$ de tétrachlorure de titane. On maintient l'agitation à -15°C pendant 15 mn, puis ajoute lentement à -20°C, une solution de 30 g de 3,3-[(1,2-éthanediyl) bis(thio)] androsta-4,9-dièn-17-one et 15 $cm^3$ de trichloroacétate de méthyle dans 150 $cm^3$ de tétrahydrofuranne. On maintient l'agitation pendant 10 mn à -20°C, puis laisse revenir à 20°C et, après 1 h 30 mn, ajoute à +10/+15°C, 150 $cm^3$ de mélange eau-pyridine (4-1). On agite pendant 1 h, puis acidifie par le mélange acide chlorhydrique concentré (50 $cm^3$)-eau glacée (150 $cm^3$) à +10/+15°C. On extrait au chlorure de méthylène et après lavage à l'eau, séchage et évaporation de solvant, obtient 40 g de produit attendu brut. On dissout ce produit dans du chlorure de méthylène et le traite par un mélange silice-alumine. On filtre, évapore en partie le solvant, ajoute de l'éther isopropylique, évapore à nouveau partiellement le solvant, glace et essore les cristaux. On obtient en 2 jets 35 g de produit attendu cristallisé, sous forme d'un mélange de 2 isomères.

Spectre IR ($CHCl_3$)
Absorption à 1649 $cm^{-1}$ C=C (4,5)
1719-1436 $cm^{-1}$ $CO_2CH_3$
1637-1605 $cm^{-1}$ 2 bandes C=C.
Spectre RMN ($CDCl_3$ - 400 MHz ppm)
Isomère 1 : 18 $CH_3$ = 0,95 - 19 $CH_3$ = 1,19 - $CO_2CH_3$ = 3,79 - $H_{11}$= 5,42.
Isomère 2 : 18 $CH_3$ = 1,00 - 19 $CH_3$ = 1,18 - $CO_2CH_3$ = 3,81 - $H_{11}$ = 5,37.
$H_4$ = 5,49 et $-CH_2S-$ = 3,30.

**EXEMPLE 2 : 21-acétoxy pregna-4,9 (11),16-triène-3,20-dione**

STADE A : 3,3-[(1,2-éthanediyl) bis(thio)] 20-phénoxy pregna-4,9(11),17(20)-trièn-21-oate de méthyle

On mélange sous gaz inerte, 130 $cm^3$ de méthyléthylcétone, 13 g de 20-chloro 3,3[(1,2-éthanediyl) bis(thio)] pregna-4,9(11),17(20)-trièn-21-oate de méthyle, 8,12 g de phénol et 11,95 g de carbonate de potassium. On porte au reflux sous agitation pendant 26 heures, chasse la majeure partie du solvant, reprend par une solution aqueuse de bicarbonate de sodium, extrait à l'acétate d'éthyle, sèche et évapore le solvant. On cristallise le produit dans le méthanol et obtient 6,85 g de produit attendu. F = 184°C. On chromatographie les liqueurs mères sur silice en éluant avec un mélange chlorure de méthylène-cyclohexane (7/3) et obtient à nouveau 3,21 g de produit attendu, lequel est constitué d'un mélange de 2 isomères.

Spectre RMN ($CDCl_3$ - 400 MHz ppm)
Isomère 1 : 19 $CH_3$ = 1,13 (s) - 18 $CH_3$ = 0,87 (s) - $CH_2$-S = 3,2 à 3,4 - $CO_2CH_3$ = 3,62 (s) - $H_{11}$ = 5,32 (d).
Isomère 2 : 19 $CH_3$ = 1,20 - 18 $CH_3$ = 1,10 - $CO_2CH_3$ = 3,66 - $H_{11}$ = 5,44 (d) - $H_4$ = 5,29 (s) - H aromatiques = 6,88.

STADE B : 3,3-[(1,2-éthanediyl) bis(thio)] 21-hydroxy 20-phénoxy pregna-4,9(11),17(20)-triène

On mélange sous gaz inerte 45 $cm^3$ de toluène et 8,3 g du produit obtenu au stade A, refroidit à 0/+5°C et ajoute lentement à +5/+7°C, 29 $cm^3$ d'une solution 1,2 M d'hydrure de diisobutyl aluminium dans le toluène. On maintient à +5°C sous agitation pendant 1 h 30 mn puis ajoute lentement à +10/+15°C, 1 $cm^3$ de méthanol puis 5 $cm^3$ de soude à 36°Bé et 20 $cm^3$ d'eau. Après 1 heure à +10°C, on filtre, lave à l'eau, puis au chlorure de méthylène à 10 % de méthanol. On décante la phase organique, la lave à l'eau, sèche et évapore le solvant. On reprend le produit brut par 30 $cm^3$ de méthanol, glace et essore. On obtient 6,67 g de produit attendu. F = 228°C.

spectre RMN ($CDCl_3$ - 300 MHz ppm)
18 $CH_3$ = 0,85 (s) - 19 $CH_3$ = 1,13 (s) - $CH_2$-S = 3,1 à 3,4 - $CH_2$OM = 4,16 - $H_{11}$ = 5,33 - $H_4$ = 5,47 - H aromatiques = 6,92 (d), 6,98 (t) et 7,26 (t).

STADE C : 21-hydroxy 20-phénoxy pregna-4,9(11),17(20)-trièn-3-one

On mélange à température ambiante 1,349 g de produit obtenu au stade précédent et 13,5 $cm^3$ de mélange méthanol-chlorure de méthylène-eau (5/1/1), agite une demi-heure, puis ajoute 54 mg d'iode. On ajoute ensuite en 3 heures, 0,4 $cm^3$ d'eau oxygénée à 110 vol. puis poursuit l'agitation pendant 1 heure environ. On neutralise ensuite le mélange par addition de thiosulfate de sodium 0,2 N, extrait au chlorure de méthylène, lave la phase organique à l'eau, la sèche et évapore le solvant. On chromatographie le produit brut sur silice en éluant au mélange cyclohexane-acétate d'éthyle (6-4) et obtient 1,005 g de produit attendu. F = 149°C.

Spectre IR ($CHCl_3$)
Absorption à 1662, 1613 et 866 $cm^{-1}$ (delta4-3-one) ;
1596-1491 $cm^{-1}$ (Φ-O) ; 3609 $cm^{-1}$ (OH libre) ;
1596 $cm^{-1}$ + épaulement à 1591 $cm^{-1}$ (Φ-O-C=C).

STADE D : pregna-4,9(11)diène-17alpha,21-diol-3,20-dione

On mélange sous gaz inerte 1,5 g de produit obtenu au stade précédent et 15 $cm^3$ de toluène, refroidit à +5°C et ajoute 13,8 mg d'acétylacétonate de vanadium. On ajoute à la solution en 5 mn à +5°C, 0,5 $cm^3$ d'hydropéroxyde de tert-butyle à 70 %, agite pendant 15 mn à +5°C puis laisse remonter à température ambiante et agite encore 1 h. On rajoute alors 0,1 $cm^3$ d'hydropéroxyde de tert-butyle, agite 2 h 15 mn puis ajoute 2 $cm^3$ d'une solution 0,5 M de thiosulfate de sodium. On agite 15 mn, ajoute 6 $cm^3$ d'acide chlorhydrique 2N et agite pendant 3 h. On extrait au chlorure de méthylène à 10 % d'éthanol, lave la phase organique à l'eau saturée de chlorure de sodium, sèche et évapore à sec. On reprend le résidu par un mélange éthanol-chlorure de méthylène au reflux, chasse le chlorure de méthylène, glace, essore et obtient 1,14 g de produit que l'on traite comme ci-dessus, cette fois par un mélange chlorure de méthylène-méthanol. On obtient 1,015 g de produit attendu (F > 264°C).

Spectre RMN ($CDCl_3$ + pyridine deutérée - 300 MHz ppm)
18 $CH_3$= 0,62 (s) - 19 $CH_3$ = 1,33 (s) - CO-$CH_2$-OH = 4,35 (d) et 4,79 (d) - $H_{11}$ = 5,55 (d) - $H_4$ = 5,74 (s) - Autre proton = 5,71.

STADE E : 17alpha,21-diacétoxy pregna-4,9(11)diène-3,20-dione

On mélange sous gaz inerte 4 g de produit obtenu selon le procédé décrit au stade précédent, 0,15 g de diméthylaminopyridine, 16 $cm^3$ de toluène et 5,9 g d'anhydride acétique. On porte au reflux pendant 20 heures puis après refroidissement, ajoute 1 $cm^3$ d'eau et évapore à sec. On reprend au chlorure de méthylène et ajoute 1,2 $cm^3$ d'acide chlorhydrique 22°Bé. On agite à température ambiante pendant 1 heure, lave à l'eau, sèche et évapore à sec. On obtient 4,9 g de produit attendu brut que l'on solubilise dans 40 $cm^3$ de chlorure de méthylène en présence d'alumine. Après 10 mn d'agitation on filtre, ajoute environ 1 volume de méthanol et concentre à chaud la solution jusqu'à cristallisation. On refroidit, essore les cristaux, les lave au méthanol et les sèche. On obtient 2,9 g de produit attendu.

Spectre IR ($CHCl_3$)

Absence d'OH.
Absorption à 1735 $cm^{-1}$ (-OAC), 1665 et 1614 $cm^{-1}$
(delta4-3-one)

STADE F : 21-acétoxy pregna-4,9(11),16triène-3,20-dione

On mélange sous gaz inerte 180 $cm^3$ de diméthylformamide et 3 g d'acétate de potassium, porte à ébullition, distille lentement 60 $cm^3$ de diméthylformamide, refroidit à 115°C et introduit 30 g de produit obtenu au stade précédent. On maintient ensuite à 115°C pendant 3 h., distille une partie du solvant, refroidit à 40°C environ et, en maintenant l'agitation, on ajoute 200 $cm^3$ d'eau. On agite 1 h. à température ambiante, essore et sèche les cristaux formés. On les reprend par 50 $cm^3$ de méthanol, chauffe à 45°C puis refroidit à 0°C, essore les cristaux et les sèche. On obtient 16,2 g de produit attendu.
$alpha_D^{20}$ = + 166° ± 2°5 (c = 1 % DMF).

**EXEMPLE 3 : 21-acétoxy pregna-4,9(11),16-triène-3,20-dione**

STADE A : 3,3-[(1,2-éthanediyl) bis(thio)] 17-[[(4-méthylphényl) sulfonyl] méthyl] androsta-4,9(11)16-triène

On mélange sous gaz inerte, à température ambiante, 12 $cm^3$ de diméthylformamide, 200 mg de phénol, 300 mg de carbonate de potassium, 2 g de tolylsufinate de sodium et 2 g de produit obtenu à l'exemple 1. On chauffe à 105°C pendant 22 heures puis ajoute 0,7 g de carbonate de potassium et 1,5 $cm^3$ d'eau. On porte à 100°C pendant 1 h puis ajoute 10 $cm^3$ de soude 2N et maintient à 100°C pendant 4 heures. On refroidit à 10°C, verse lentement dans 60 $cm^3$ d'acide chlorhydrique 2N, agite 30 mn et essore le produit. On le reprend par du chlorure de méthylène, lave à l'eau, sèche et évapore le solvant. On chromatographie le résidu sur silice en éluant au mélange cyclohexane-acétate d'éthyle (8-2) et obtient, après cristallilsation dans le méthanol, 1,65 g de produit attendu. F = 198°C.

Spectre IR ($CHCl_3$)
Absorptions à 1643 $cm^{-1}$(delta4), 1598, 1494, 1317, 1303 et 1150 $cm^{-1}$ (Tosyle).
Spectre RMN ($CDCl_3$ - 400 MHz ppm)
18 $CH_3$ = 0,63 (s) - 19 $CH_3$ = 1,16 (s) - $H_{11}$ = 5,37 - $H_4$ = 5,49 - $H_{16}$ = 5,77 - H éthanediyl = 3,20 à 3,40 (m) - H aromatiques = 7,33 (d), 7,75 (d) (J = 8 Hz) - $CH_3$ tosyle = 2,45 (s) - $H_{20}$ = 1,01, 1,44 et 1,77 à 2,42 (m), 3,76 (d) et 3,82 (d)
(J = 15 Hz).

STADE B : 3,3-[(1,2-éthanediyl) bis-(thio)] 20-[(4-méthylphényl) sulfonyl] pregna-4,9(11)16,20-tétraène

On mélange à température ambiante, sous gaz inerte, 1,5 $cm^3$ de diméthylformamide, 0,3 g de produit obtenu au stade A, 0,1 g de carbonate de potassium et 0,1 g de paraformaldéhyde, agite pendant 20 heures, puis porte à 60°C environ pendant 3 heures. On refroidit à + 10°C, ajoute 10 $cm^3$ d'eau, agite 30 mn et essore. On reprend les cristaux par du chlorure de méthylène, sèche et évapore le solvant. On chromatographie le résidu sur silice en éluant au mélange cyclohexane-acétate d'éthyle (7-3) et obtient 0,28 g de produit attendu.

Spectre IR ($CHCl_3$)
Absence d'OH.
Spectre RMN ($CDCl_3$ - 300 MHz ppm)
18 $CH_3$ = 0,67 (s) - 19 $CH_3$ = 1,15 (s) - $H_{11}$ = 5,34 (m) - $H_4$ = 5,48 - H éthanediyl = 3,20 à 3,40 - $H_{16}$ = 6,40 (m) -$H_{21}$ = 5,94 et 6,48 - H aromatiques = 7,28 (d) et 7,68 (d) - $CH_3$ tosyle = 2,42 (s).

STADE C : 20-21-époxy 3,3-[(1,2-éthanediyl) bis-(thio)] 20-[(4-méthylphényl) sulfonyl] pregna-4,9(11)16-triène

On mélange à température ambiante, sous gaz inerte, 2 g de produit obtenu comme décrit au stade B, 12 $cm^3$ de dioxanne, 2 $cm^3$ d'eau et ajoute simultanément en 1 h à pH ~ 11, 3,23 $cm^3$ de soude 2N et 0,39 $cm^3$ d'eau oxxygénée à 50 %. On agite à température ambiante et, après 2 h., ajoute 0,3 $cm^3$ de soude 2N et 0,06 $cm^3$ d'eau oxygénée à 50 %, puis, après 7 h., 0,03 $cm^3$ d'eau oxygénée à 50 %. Après 16 h. à température ambiante, on verse dans l'eau saturée de chlorure de sodium et extrait au chlorure de méthylène. On lave l'extrait à l'aide d'une solution 0,5 M de thiosulfate de sodium puis à l'eau, sèche et évapore le solvant. On chromatographie le résidu sur silice en éluant au mélange chlorure de méthylène-hexane (8-2) et obtient 1,55 g de produit attendu.

Spectre RMN ($CDCl_3$ - 300 MHz ppm)
18 $CH_3$ = 0,31 (s) et 0,66 (s) - 19 $CH_3$ = 1,12 (s) et 1,13 (s) - $CH_3$-Φ = 2,44 (s) et 2,47 (s) - $CH_2$-O époxyde = 2,85 (d), 2,99 (d) et 3,68 (d) - thiocétal = 3,2 à 3,4 - $H_{11}$ = 5,19 et 5,32 - $H_4$ = 5,47 - $H_{16}$ = 6,29 (m) et 6,39 (m) - Φ$SO_2$ = 7,33 (m) et 7,73 (m).

STADE D : 21-acétoxy 3,3-[(1,2-éthanediyl) bis(thio)] pregna-4,9(11)16-trièn-20-one.

On mélange sous gaz inerte, 7,5 $cm^3$ de polyéthylène glycol, 1 g de produit obtenu au stade C et 0,8 g d'acétate de sodium. On chauffe par un bain à 48°C pendant 2 h 45 mn, refroidit à 0/+5°C, verse dans l'eau glacée, agite pendant 30 mn, essore, lave les cristaux à l'eau et les dissout dans le chlorure de méthylène. On sèche la solution et amène à sec. On reprend au chlorure de méthylène, ajoute de l'éther isopropylique et chasse le chlorure de méthylène. On refroidit et essore les cristaux formés.

On évapore le filtrat à sec et chromatographie le résidu sur silice en éluant au chlorure de méthylène. On obtient 0,62 g de produit attendu. F = 172°C.

Spectre IR ($CHCl_3$)
Acétate 1748 $cm^{-1}$
Cétone conjuguée -C=C 1589 $cm^{-1}$
Delta4 à 1644 $cm^{-1}$
Spectre RMN ($CDCl_3$ - 300 MHz ppm)
18 Me = 0,85 (s) - 19 Me = 1,19 (s) - OAC = 2,19 (s) - C-$CH_2$O = 4,91, 5,05 (d,J = 16)- $H_{11}$ = 5,45 (m) = $H_4$ = 5,50 - O $H_{16}$ = 6,76 (m).

STADE E : 21-acétoxy pregna-4,9(11)16-triène-3,20-dione

On mélange à température ambiante 1 g de produit obtenu au stade D et 12 $cm^3$ d'un mélange méthanol-eau-chlorure de méthylène (5-1-2). On ajoute 0,042 g d'iode, agite pendant 15 mn puis ajoute en 3 h. 0,32 $cm^3$ d'eau oxygénée à 50 %. On maintient sous agitation pendant 16 heures puis ajoute 6 $cm^3$ d'une solution 0,2 N de thiosulfate de sodium. On extrait au chlorure de méthylène, lave à l'eau, sèche et évapore à sec. On chromatographie le résidu sur silice en éluant au mélange cyclohexane-acétate d'éthyle (1-1) et obtient 0,66 g de produit attendu que l'on cristallise dans l'éther isopropylique. F = 128°C.

Spectre IR ($CHCl_3$)
Absorptions à 1748 $cm^{-1}$ (acétate) et 1684, 1664, 1614 et 1590 $cm^{-1}$ (cétones conjuguées).
Spectre RMN ($CDCl_3$ - 300 MHz ppm)
18 $CH_3$ = 0,89 (s) - 19 $CH_3$ = 1,36 (s) - $H_{11}$ = 5,55 (m) - $H_4$ = 5,76 (m) - $H_{21}$ = 4,91 et 5,06 (d, J = 16 Hz).

**EXEMPLE 4 : 21-acétoxy pregna-4,9(11),16-triène-3,20-dione**

STADE A : 3,3-[(1,2-éthanediyl) bis(thio)] 20-[(4-méthylphényl) sulfonyl] pregna-4,9(11)16-trièn-21-oate de méthyle

On mélange sous gaz inerte à température ambiante 60 $cm^3$ de diméthylformamide, 25 $cm^3$ de toluène, 10 g de tolylsulfinate de sodium, 1 g de phénol, 2 g de carbonate de sodium et 0,56 $cm^3$ de tris[2-(2-méthoxyéthoxy) éthyl] amine. On porte au reflux du toluène et distille pour éliminer l'eau. On refroidit à 90/100°C et ajoute 10 g de produit obtenu à l'exemple 1. On maintient à 100°C environ pendant 10 h., ajoute 2 g de tolylsulfinate de sodium et poursuit le chauffage pendant 6 heures. On refroidit à 5/10°C et verse lentement dans 500 $cm^3$ d'eau et de glace renfermant 7 g de phosphate monosodique. On extrait au chlorure de méthylène, lave la phase organique à l'eau, sèche et évapore à sec. On chromatographie le résidu sur silice en éluant au mélange cyclohexane-acétate d'éthyle (90-10) et obtient 9,9 g de produit attendu.

Spectre IR ($CHCl_3$)
Absorptions à 1743 $cm^{-1}$ (C=O), 1330, 1148 $cm^{-1}$ ($SO_2$), 1598, 1493 $cm^{-1}$ (aromatiques), 1645 $cm^{-1}$ (C=C-delta4).
Spectre RMN ($CDCl_3$ - 400 MHz ppm)
18 $CH_3$ = 0,65 (s) - 19 $CH_3$ = 1,17 (s) - $H_{14}$ = 1,04 et 1,45 à 2,45 (m) - Φ-$CH_3$ = 2,45 (s) - S-$CH_2$-$CH_2$-S = 3,24 à 3,40 (m) -$COOCH_3$ = 3,66 et 3,72 (2s) - $H_{20}$ = 4,53 et 4,55 (2s) - $H_{11}$ = 5,38 (m) - $H_4$ = 5,50 (s) - $H_{16}$ = 6,00 et 6,15 (2s) - $H_3$ et $H_5$Φ = 7,33 (d, J = 8 Hz) - $H_2$ et $H_6$ = 7,72 et 7,77 (2d, J = 8 Hz).

STADE B : 17-[chloro [(4-méthylphényl) sulfonyl] méthyl] 3,3[(1,2-éthanediyl) bis(thio)] androsta-4,9(11),16(17)-triène

On mélange sous gaz inerte 14 $cm^3$ de dioxanne et 6,4 g de produit obtenu au stade A. On ajoute lentement à 20/25°C, le mélange de 13 $cm^3$ d'hypochlorite de sodium et 3,4 g de potasse en pastilles, agite pendant 1 h 30, puis refroidit à +10°C et ajoute lentement une solution de 1,4 g de thiosulfate de sodium à $5H_2O$ dans 1,4 $cm^3$ d'eau puis 1,4 $cm^3$ de lessive de soude à 32°Bé. On laisse la température remonter à 20/25°C puis porte pendant 3 h à 40°C. On refroidit à +10°C et ajoute lentement 60 $cm^3$ d'acide chlorhydrique 2N. On agite dans un bain de glace pendant 1 h., filtre les cristaux formés, les lave à l'eau et les reprend au chlorure de méthylène. On sèche la solution et évapore le solvant. On chromatographie le résidu sur silice en éluant au chlorure de méthylène. On obtient 3,73 g de produit attendu. F ~ 250°C.

Spectre RMN ($CDCl_3$ - 300 MHz ppm)
18 $CH_3$= 0,75 et 0,81 - 19 $CH_3$ = 1,19 (s) - $CH_3$-Φ = 2,47 -S-$CH_2$-$CH_2$-S = 3,2 à 3,4 - $SO_2$-CH- = 5,01 et 5,04 - $H_{11}$ = 5,41 - $H_4$ = 5,50 - $H_{16}$ = 6,20 et 6,41 - -Φ-$SO_2$ = 7,36 (d) 7,83 (d, dédoublé).

STADE C : 20,21-époxy 3,3-[(1,2-éthanediyl) bis(thio)] 20-[(4-méthylphényl) sulfonyl] pregna-4,9(11),16-triène

On mélange à +10°C, sous gaz inerte, 2,7 $cm^3$ de chlorure de méthylène, 0,55 g de produit obtenu au stade B, 0,2 g de paraformaldéhyde, 0,03 g de chlorure de benzyl triéthylammonium et 2 $cm^3$ de lessive de soude à 32°Bé. On agite en laissant remonter la température à 20/25°C. Après 2 h 30, on rajoute 0,075 g de paraformaldéhyde et poursuit l'agitation pendant 1 heure. On verse dans une solution aqueuse saturée de chlorure de sodium, extrait au chlorure de méthylène, sèche et évapore à sec. On chromatographie le résidu sur silice en éluant au chlorure de méthylène et obtient 0,462 g de produit attendu.

Spectre RMN ($CDCl_3$ - ppm)
18 $CH_3$ = 0,31 - 0,66 ; 19 $CH_3$ = 1,12 - 1,13 ; $H_{11}$ = 5,19 (d) - 5,32 ; S-$CH_2$-$CH_2$-S = 3,24 (m - 1H) - 3,36 ; $H_4$ = 5,47 ; $H_{16}$ = 6,30 et 6,39 ; $CH_2$ époxyde = 2,86 (d) - 2,99 (d) - 3,68 ; Φ-$CH_3$ = 2,44 (d) - 2,47 et 7,33 (d) - 7,73 (d).

STADE D : 21-acétoxy pregna-4,9(11),16-triène-3,20-dione

On opère comme indiqué aux stades D et E de l'exemple 3 et obtient le produit attendu.

**EXEMPLE 5: 21-acétoxy pregna-4,9(11),16-triène-3,20-dione**

STADE A : 21-bromo 3,3-[(1,2-éthanediyl) bis(thio)] pregna-4,9(11)16-trièn-20-one

On mélange sous gaz inerte 1,55 g de produit obtenu au stade C de l'exemple 4 et 8 $cm^3$ de chlorure de méthylène. On y ajoute à -1/+1°C, 0,95 g de bromure de lithium puis 0,18 $cm^3$ de méthanol. On maintient sous agitation à -1/+1°C pendant 7 heures, puis ajoute à 22°C maxium, 6 $cm^3$ d'eau. On agite pendant 10 mn, décante la phase organique et la lave à l'eau puis la décolore au charbon actif et la sèche. On obtient ainsi une solution chlorométhylénique du produit attendu, utilisé sous cette forme pour le stade suivant.

Un échantillon de produit obtenu par évaporation du solvant a été isolé pour analyse.

Spectre RMN ($CDCl_3$ - 300 MHz ppm)
18 $CH_3$ = 0,85 (s) - 19 $CH_3$ = 1,20 (s) - thiocétal = 3,2 à 3,4 - CO-$CH_2$-X = 4,17 - $H_{11}$ = 5,46 - $H_4$ = 5,50 - $H_{16}$ = 6,83 (m).

STADE B : 21-acétoxy 3,3-[(1,2-éthanediyl) bis(thio)] pregna-4,9(11)16-trièn-20-one

On évapore à demi volume environ 9,5 $cm^3$ de solution dans le chlorure de méthylène du produit 21-bromo obtenue comme décrit au stade A, puis ajoute 4 $cm^3$ de diméthylformamide et poursuit la distillation du chlorure de méthylène. On ajoute ensuite, sous gaz inerte à la suspension obtenue, à 25°C environ, 0,8 g d'acétate de potassium, 0,08 $cm^3$ d'acide acétique et 0,04 $cm^3$ d'eau, puis maintient sous agitation à 25°C pendant 1 heure. On chauffe ensuite à 60°C environ pendant 1 heure puis introduit lentement 1,4 $cm^3$ d'eau. On refroidit à 20°C, essore les cristaux, les lave par un mélange eau-diméthylformamide et les sèche. On obtient 1,54 g de produit attendu identique à celui obtenu au stade D de l'exemple 3 et pouvant être purifié comme décrit dans cet exemple .

STADE C : 21-acétoxy pregna-4,9(11)16-triène-3,20-dione

On opère comme indiqué au stade E de l'exemple 3 et obtient le produit attendu.

**EXEMPLE 6 : 21-acétoxy pregna-4,9(11),16-triène-3,20-dione**

STADE A : 20-chloro 3,3-[(1,2-éthanediyl) bis(thio)] 21-hydroxy pregna-4,9(11),17(20)-triène

On mélange sous gaz inerte 100 $cm^3$ de toluène et 6 g de produit obtenu à l'exemple 1, refroidit à -20°C et introduit lentement 38 $cm^3$ d'hydrure de diisobutylaluminium. On poursuit l'agitation pendant 1 heure puis ajoute lentement 3 $cm^3$ de méthanol, puis 50 $cm^3$ d'eau. On poursuit l'agitation pendant 30 mn, puis ajoute de l'acide chlorhydrique 2N, extrait à l'acétate d'éthyle, lave la phase organique à l'eau, sèche et évapore à sec. On empâte le résidu dans 20 $cm^3$ d'éther isopropylique, glace, filtre et sèche le produit. On obtient 5,113 g de produit attendu. F = 198°C.

Spectre RMN ($CDCl_3$ - 300 MHz ppm)
18 $CH_3$ = 0,88 et 0,90 - 19 $CH_3$ = 1,18 (s) - $H_{11}$ = 5,40 (m) et 5,49 (m) - -S-$CH_2$-$CH_2$-S = 3,2 à 3,4 - $H_4$ = 5,5 - $CH_2OH$ - 4,19 (m) - 4,27 (dd) et 4,40 (dd).

STADE B : 20-chloro 3,3-[(1,2-éthanediyl) bis(thio)] pregna-4,9(11),17(20)-trièn-21-al

On mélange à température ambiante, sous gaz inerte, 1 g de produit obtenu au stade A, 6 $cm^3$ de diméthylsulfoxyde et 3,3 $cm^3$ de triéthylamine. Après 15 mn, on ajoute lentement 1,47 g de complexe pyridine-$SO_3$ en maintenant la température à +20/+22°C. On poursuit l'agitation à cette température pendant 16 h, verse dans un mélange de 15 $cm^3$ d'acide chlorhydrique 2N et 20 $cm^3$ d'eau glacée et agite pendant 30 mn. On essore l'insoluble, le lave à l'eau et le reprend par du chlorure de méthylène. On sèche la solution organique et évapore le solvant. On chromatographie le résidu sur silice en éluant au mélange cyclohexane-acétate d'éthyle (8-2) et après recristallisation dans 5 $cm^3$ d'éther isopropylique on obtient 0,622 g de produit attendu. F = 230°C.

Spectre RMN ($CDCl_3$ - 300 MHz ppm)
18 $CH_3$ = 0,99 (s) - 1,08 (s) ; 19 $CH_3$ = 1,20 (s) - $H_{11}$ = 5,44 (m) ; -S-$CH_2$-$CH_2$-S = 3,2 à 3,4 (m) ; $H_4$ = 5,52 (s) ; H de CHO = 9,73 (s) - 9,91 (s) ; $H_{16}$ et autres = 1,40 à 2,95 (m).

STADE C : 21-acétoxy 3,3-[(1,2-éthanediyl) bis(thio)] pregna-4,9(11),16-trièn-20-one.

On mélange sous gaz inerte 10 $cm^3$ de diméthylsulfoxyde et 0,7 g d'acétate de sodium, porte à 60°C et introduit lentement 1,2 g de produit obtenu au stade B. On maintient sous agitation pendant 3 h à 70°C, refroidit, ajoute 20 $cm^3$ d'eau saturée de chlorure de sodium, agite 30 mn et essore. On lave à l'eau et reprend au chlorure de méthylène, sèche et évapore le solvant. On chromatographie le résidu sur silice en éluant au mélange cyclohexane-acétate d'éthyle (8-2) et obtient 0,89 g de produit attendu, que l'on recristallise dans l'éther isopropylique. F = 183°C.

Spectre RMN ($CDCl_3$ - 300 MHz ppm)
18 $CH_3$ = 0,85 (s) ; 19 $CH_3$ = 1,19 (s) - $H_{11}$ = 5,45 (d) ; -S-$CH_2$-$CH_2$-S = 3,2 à 3,4 ; $H_4$ = 5,50 (s) ; $CH_3$ acétyl = 2,19 (s) ; $CH_2$21 = 4,91 (d;J=16) - 5,06 (d;J=16).

STADE D : 21-acétoxy pregna-4,9(11),16-triène-3,20-dione

On opère comme indiqué au stade E de l'exemple 3 en utilisant au départ le produit obtenu au stade C ci-dessus. On obtient le produit attendu.

## Revendications

**Revendications pour les Etats contractants suivants : AT, BE, CH, DE, DK, FR, GB, IT, LI, LU, NL, PT, SE**

**1.** Les composés de formule (I) :

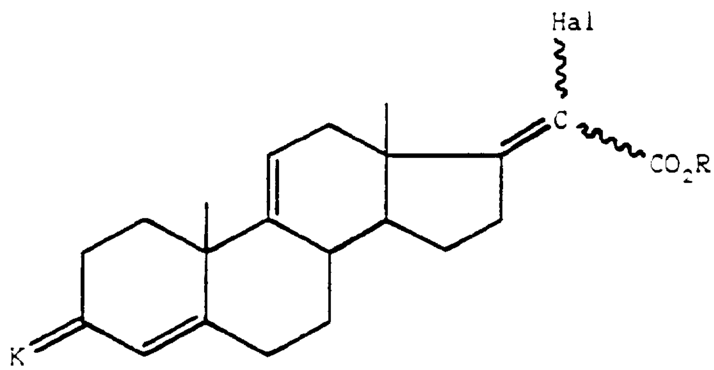

(I)

dans laquelle Hal représente un atome de chlore ou de brome, R représente un radical alkyle renfermant de 1 à 6 atomes de carbone, un radical aralkyle renfermant de 7 à 15 atomes de carbone ou un reste trialkylsilyle, triphénylsilyle ou diphényl tert-butyle silyle, K représente un groupement protecteur du radical oxo de formule :

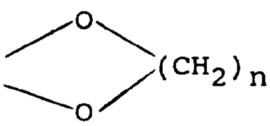

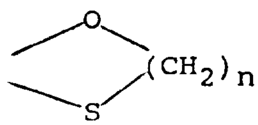

ou

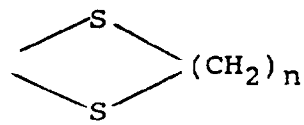

dans laquelle n est égal à 2 ou 3 et les traits ondulés symbolisent l'une quelconque des formes isomères ou leurs mélanges.

**2.** Les composés de formule (I), telle que définie à la revendication 1, dans laquelle Hal représente un atome de chlore, R représente un radical alkyle renfermant de 1 à 3 atomes de carbone et K représente un groupement de formule

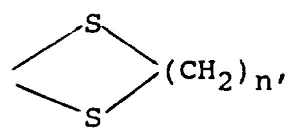

dans laquelle n est égal à 2 ou 3.

**3.** Un composé selon la revendication 1, dont le nom suit :-
20-chloro 3,3-[(1,2-éthanediyl) bis(thio)] pregna4,9(11),17(20)-trièn-21-oate -21-oate de méthyle.

**4.** Le procédé de préparation des composés de formule (I) telle que définie à la revendication 1, caractérisé en ce que l'on traite le composé de formule (II) :

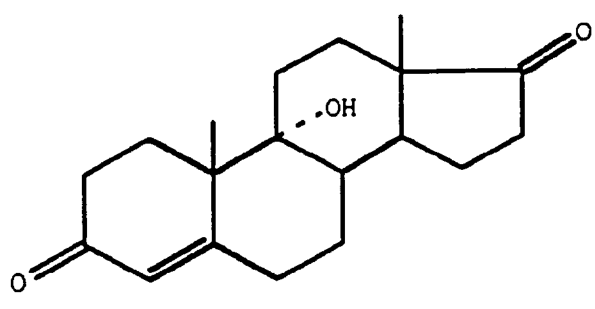

(II)

par l'anhydride acétique en présence d'un acide fort puis par un agent d'hydrolyse acide, pour obtenir le composé de formule (III) :

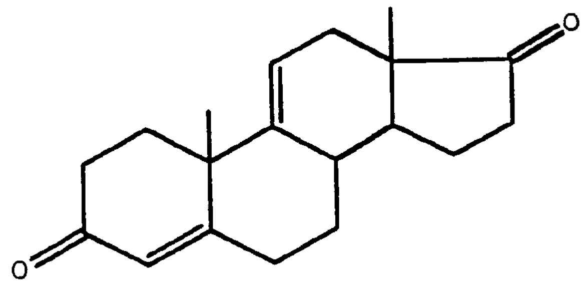

(III)

dont on bloque sélectivement la fonction 3-oxo par action d'un diol, d'un thiol ou d'un dithiol de formule $HO-(CH_2)_n-OH$, $HO-(CH_2)_n-SH$ ou $HS-(CH_2)_n-SH$ dans laquelle n est défini comme à la revendication 1 pour obtenir un composé de formule (IV) :

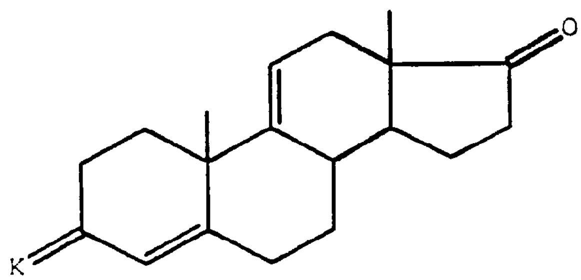

(IV)

dans laquelle K est défini comme à la revendication 1 sur lequel l'on fait réagir un composé de formule :

$$Hal_3C-CO_2R$$

dans laquelle Hal et R sont définis comme à la revendication 1, en présence du zinc et d'un acide de Lewis pour obtenir le composé de formule (I) attendu.

**5.** Procédé selon la revendication 4, caractérisé en ce que :

- l'acide fort en présence duquel on fait réagir l'anhydride acétique sur le composé de formule (II) est l'acide paratoluène sulfonique,
- l'agent d'hydrolyse acide est l'acide formique,
- l'agent de blocage de la fonction 3-oxo est l'éthane dithiol, utilisé en présence d'une quantité catalytique d'acide chlorhydrique ou bromhydrique concentré,
- l'acide de Lewis est le tétrachlorure de titane.

**6.** Application des composés de formule (I) telle que définie à la revendication 1, à la préparation des composés de formule (A) :

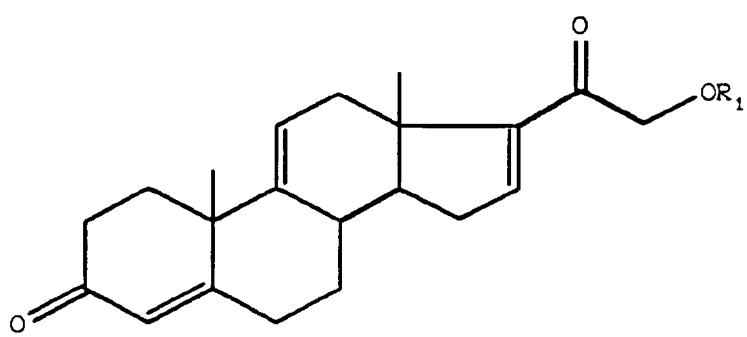

(A)

dans laquelle $R_1$ représente un radical acyle renfermant de 1 à 8 atomes de carbone, caractérisé en ce que l'on traite un composé de formule (I), en milieu basique, par un phénol de formule :

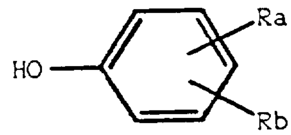

dans laquelle $R_a$ et $R_b$, identiques ou différents, représentent un atome d'hydrogène, un radical alkyle renfermant de 1 à 4 atomes de carbone, un radical alkoxy renfermant de 1 à 4 atomes de carbone, ou un radical hydroxy, pour obtenir un composé de formule (V) :

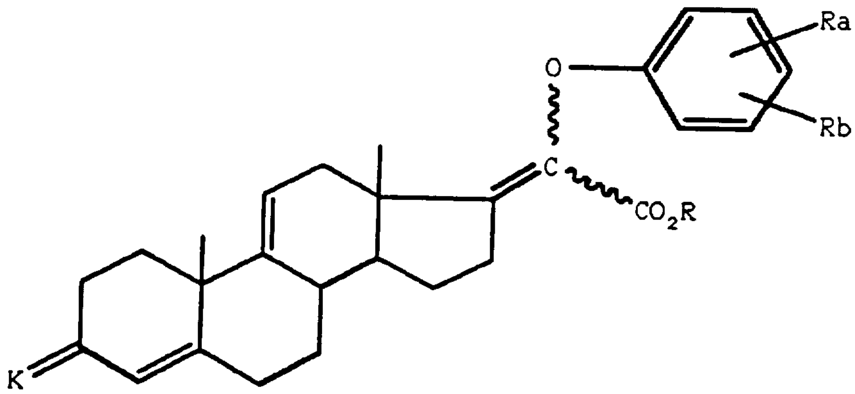

(V)

dans laquelle K et R sont définis comme à la revendication 1 et $R_a$ et $R_b$ sont définis comme précédemment, que l'on soumet à l'action d'un agent réducteur, pour obtenir un composé de formule (VI) :

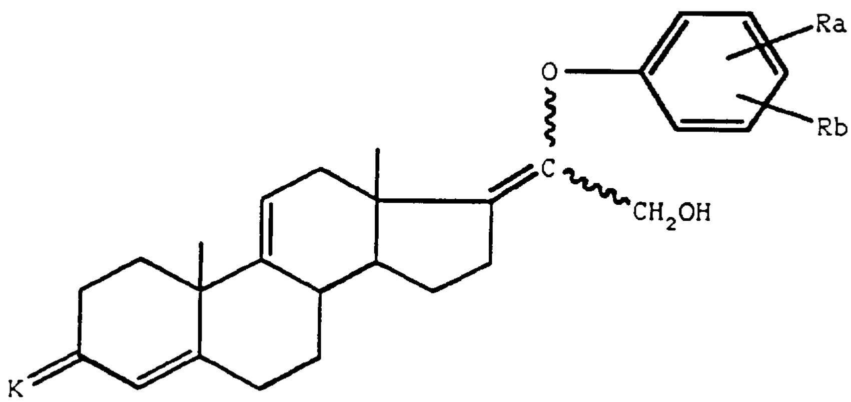

(VI)

dont on déprotège la fonction 3-oxo, puis que l'on traite par un agent d'époxydation, pour obtenir l'époxyde correspondant en position 17,20, que l'on hydrolyse en milieu acide, pour obtenir le composé de formule (VII) :

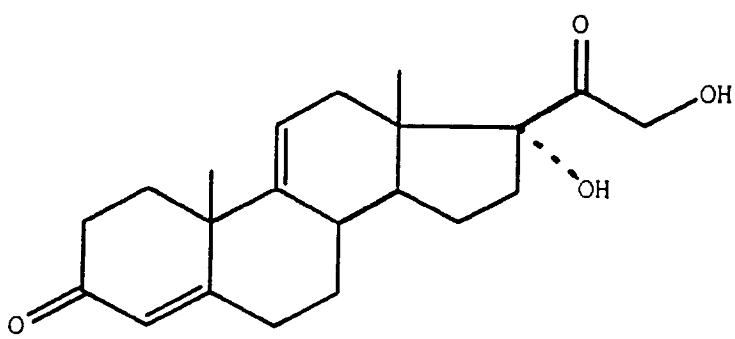

(VII)

dont on acyle les fonctions hydroxy pour obtenir le composé de formule (VIII) :

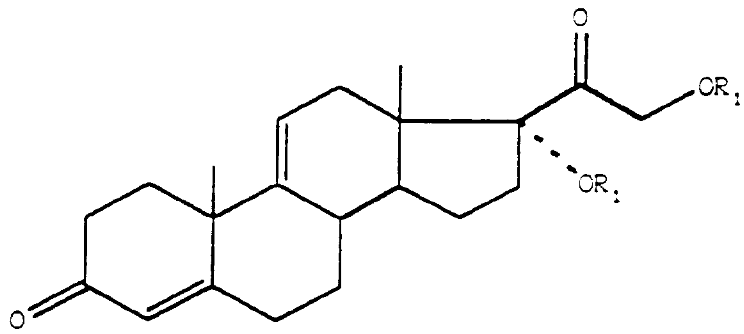

(VIII)

dans laquelle $R_1$ est défini comme précédemment, que l'on soumet à l'action d'un agent d'élimination du radical 17α-$OR_1$, pour obtenir le composé de formule (A) attendu.

**7.** Application selon la revendication 6, caractérisé en ce que :

- $R_a$ et $R_b$ représentent un atome d'hydrogène, un radical hydroxy ou un radical méthyle,
- l'agent d'acylation est l'anhydride acétique ou le chlorure d'acétyle.

**8.** Application des composés de formule (I) telle que définie à la revendication 1, à la préparation des composés de formule (A), telle que définie à la revendication 6, caractérisée en ce que l'on traite un composé de formule (I), en milieu basique par un sulfinate alcalin de formule :

$$R_2\text{-}SO_2\text{-}Q$$

dans laquelle $R_2$ représente un radical méthyle, phényle, tolyle ou xylyle et Q représente un métal alcalin, en présence d'une base, pour obtenir après saponification et décarboxylation, un composé de formule (IX) :

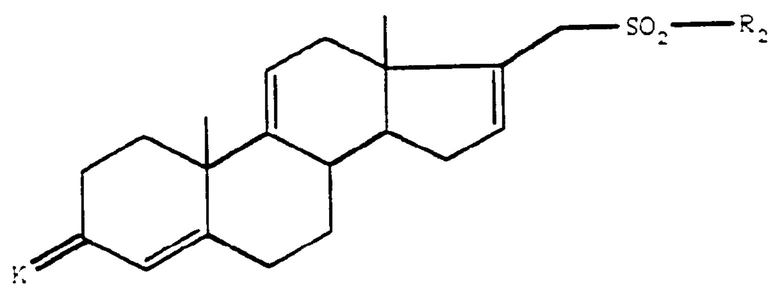

(IX)

dans laquelle K et $R_2$ sont définis comme précédemment, que l'on soumet à l'action du formaldéhyde en présence d'une base, pour obtenir un composé de formule (X) :

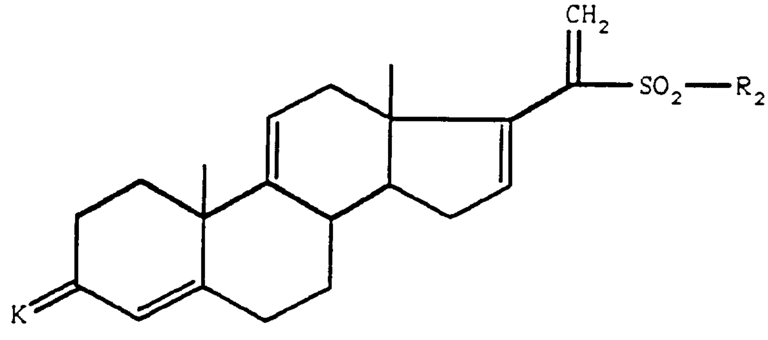

(X)

que l'on soumet à l'action d'un agent d'époxydation en milieu alcalin, pour obtenir un composé de formule (XI) :

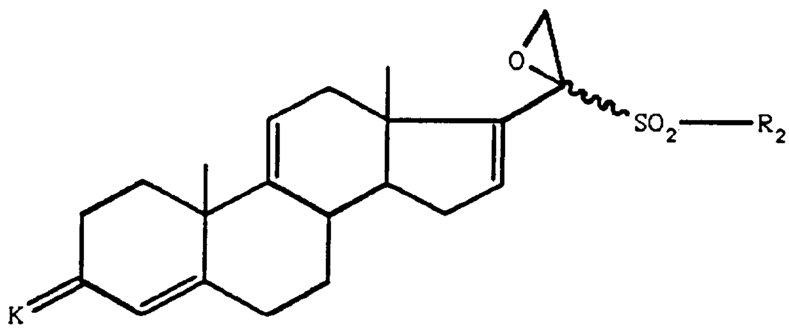

(XI)

dont on ouvre la fonction époxyde en milieu basique et en présence d'ions $R_1O^{\ominus}$, dans lesquels $R_1$ est défini comme à la revendication 6, pour obtenir un composé de formule (XII) :

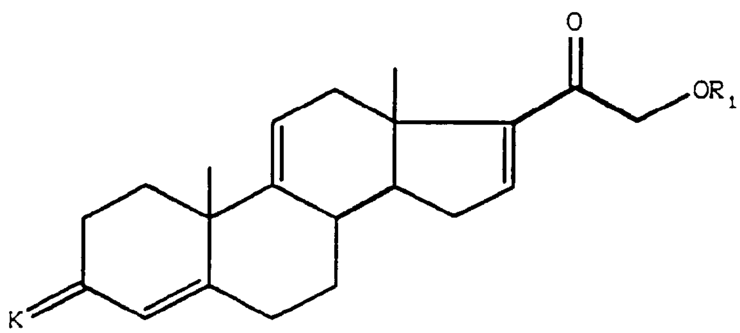

(XII)

dont on déprotège la fonction 3-oxo, pour obtenir le composé de formule (A) attendu.

**9.** Application selon la revendication 8, caractérisée en ce que :

- le sulfinate alcalin est le toluènesulfinate de sodium,
- l'ouverture de l'époxyde est effectuée en présence d'ions acétate.

**10.** Application des composés de formule (I) telle que définie à la revendication 1, à la préparation des composés de formule (A), telle que définie à la revendication 6, caractérisée en ce que l'on traite un composé de formule (I) par un sulfinate alcalin de formule $R_2SO_2Q$, telle que définie précédemment, en présence d'une base, pour obtenir un composé de formule (XIII) :

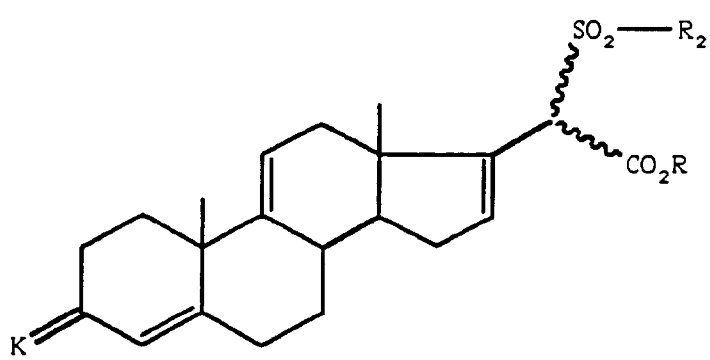

(XIII)

dans laquelle R, $R_2$ et K sont définis comme précédemment, que l'on traite par un agent d'halogénation en présence d'une base, puis que l'on saponifie et décarboxyle pour obtenir un composé de formule (XIV) :

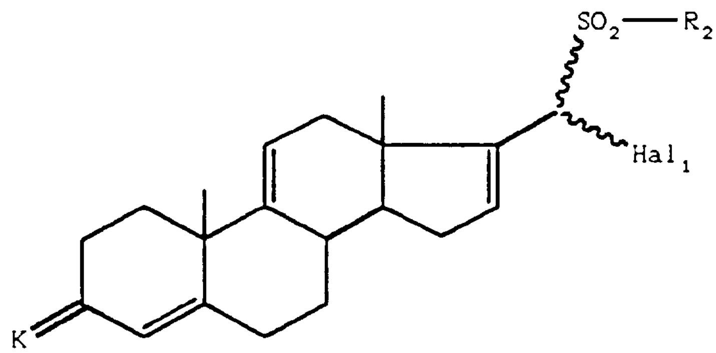

(XIV)

dans laquelle $Hal_1$ représente un atome d'halogène, que l'on traite par le formaldéhyde en présence d'une base, pour obtenir le composé de formule (XI) telle que défini à la revendication 8, que l'on traite comme décrit à la revendication 8, pour obtenir le composé de formule (A) désiré.

**11.** Application selon la revendication 10, caractérisée en ce que :

- le sulfinate alcalin utilisé est le toluènesulfinate de sodium,
- l'agent d'halogénation utilisé est l'hypochlorite ou l'hypobromite de sodium,
- l'ouverture de l'époxyde est effectuée en présence d'ions acétate.

**12.** Application des composés de formule (I) telle que définie à la revendication 1, à la production des composés de formule (A) caractérisée en ce qu'on traite le composé de formule (XI) obtenu à partir des composés de formule (I) selon les méthodes décrites dans les revendications 8 ou 10, par un halogénure alcalin pour obtenir le composé de formule (XV) :

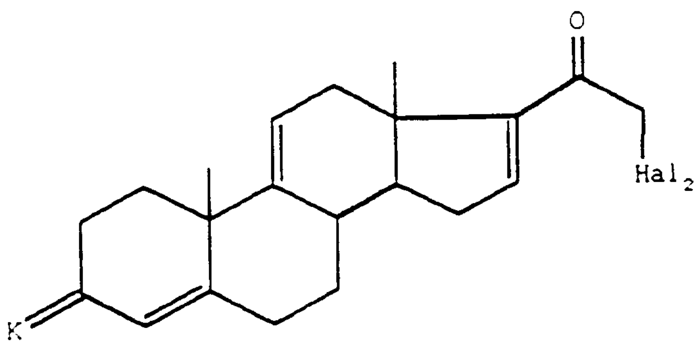

(XV)

dans laquelle $Hal_2$ représente un atome de chlore, de brome ou d'iode et K est défini comme précédemment, que l'on soumet à l'action d'un agent d'acyloxylation en milieu alcalin, pour obtenir le composé de formule (XII) telle que définie à la revendication 8, puis poursuit la synthèse comme décrit à la revendication 8.

**13.** Application selon la revendication 12, caractérisée en ce que :

- l'halogénure alcalin est le bromure de lithium,
- l'agent d'acyloxylation est l'acétate de potassium.

**14.** Application des composés de formule (I) telle que définie à la revendication 1, à la préparation des composés de formule (A), telle que définie à la revendication 6, caractérisée en ce que l'on traite un composé de formule (I) par un agent réducteur, pour obtenir un composé de formule (XVI) :

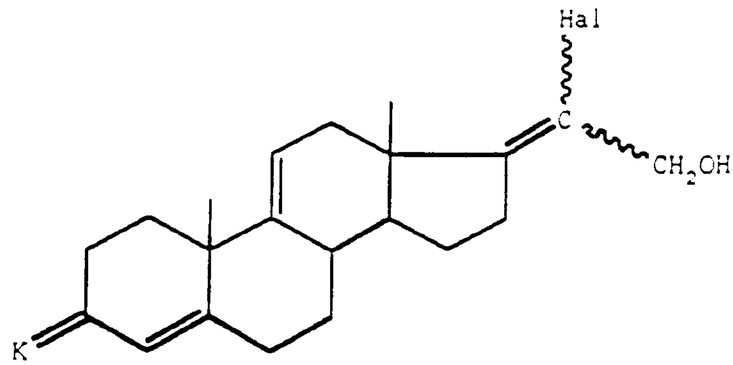

(XVI)

dans laquelle K et Hal sont définis comme à la revendication 1, que l'on soumet à l'action d'un agent oxydant, pour obtenir un composé de formule (XVII) :

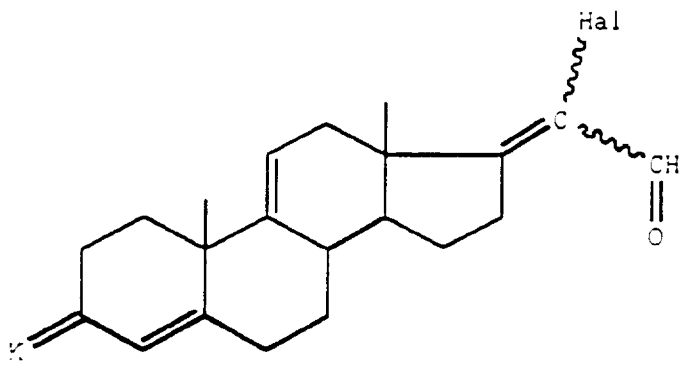

(XVII)

que l'on soumet à l'action d'un agent d'acyloxylation pour obtenir un composé de formule (XII) telle que définie à la revendication 8, puis poursuit la synthèse comme décrit à la revendication 8.

**15.** A titre de produits industriels :

- les composés de formule (F) :

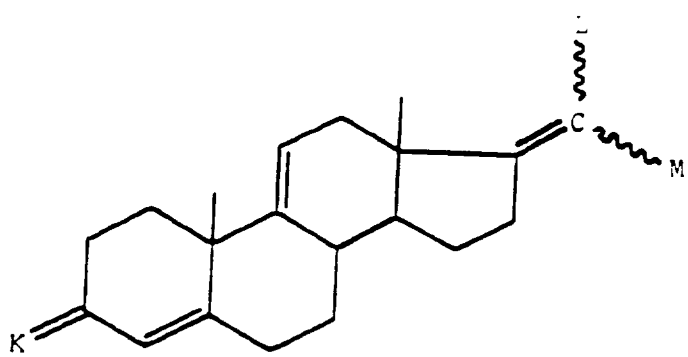

(F)

dans laquelle K est défini comme à la revendication 1, et

<u>soit</u> L représente un groupement

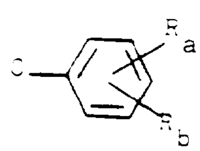

$R_a$ et $R_b$ identiques ou différents, représentant un atome d'hydrogène, un radical alkyle renfermant de 1 à 4 atomes de carbone, un radical alkoxy renfermant de 1 à 4 atomes de carbone ou un radical hydroxy, M représente un radical $CH_2OH$ ou $CO_2R$, R représentant un radical alkyle renfermant de 1 à 6 atomes de carbone, un radical aralkyle renfermant de 7 à 15 atomes de carbone ou un reste trialkylsilyle, triphénylsilyle ou diphény tert-butyle silyle,
soit L représente un atome de chlore ou de brome et M représente un radical $CH_2OH$,
et les traits ondulés symbolisent l'une quelconque des formes isomères ou leurs mélanges.

**16.** A titre de produits industriels, les produits de formule (G) :

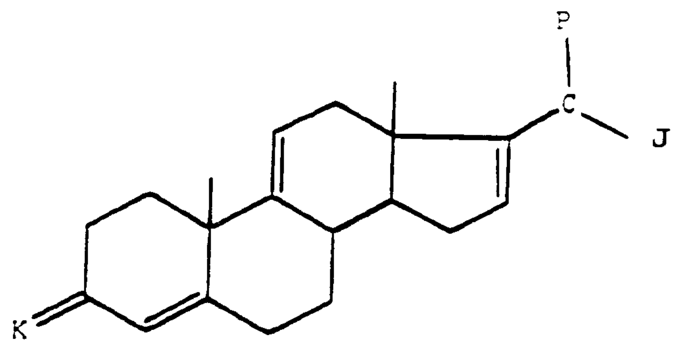

(G)

dans laquelle K est défini comme à la revendication 1, le groupement C-P représente un groupement $CH_2$, $C{=}CH_2$ ou

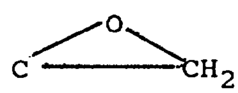

et le groupement C-J représente un groupement $C\text{-}SO_2R_2$, ou bien le groupement C-P représente un groupement -

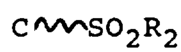

et le groupement C-J représente un groupement

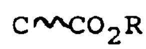

ou C-$Hal_1$, $R_2$ représentant un radical méthyle, phényle, tolyle ou xylyle, R représentant un radical alkyle renfermant de 1 à 6 atomes de carbone, un radical aralkyle renfermant de 7 à 15 atomes de carbone ou un reste trialkylsilyle, triphénylsilyle ou diphényl tert-butyle silyle, $Ha1_1$ représente un atome d'halogène, notamment un atome de chlore ou de brome, les traits ondulés symbolisant l'une quelconque des formes isomères ou leurs mélanges.

**Revendications pour l'Etat contractant suivant : ES**

**1.** Procédé pour préparer les composés de formule (I) :

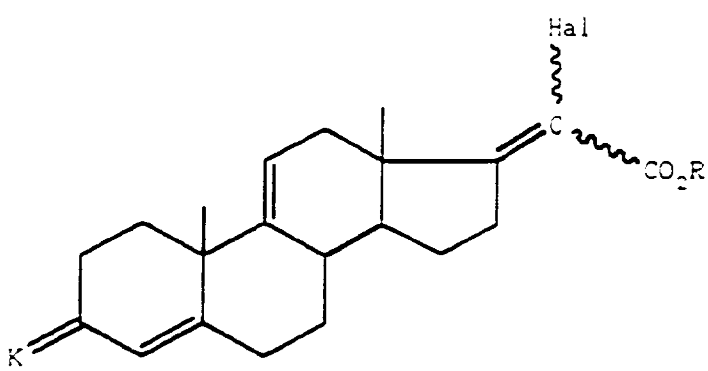

(I)

dans laquelle Hal représente un atome de chlore ou de brome, R représente un radical alkyle renfermant de 1 à 6 atomes de carbone, un radical aralkyle renfermant de 7 à 15 atomes de carbone ou un reste trialkylsilyle, triphénylsilyle ou diphényl tert-butyle silyle, K représente un groupement protecteur du radical oxo de formule :

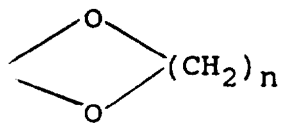 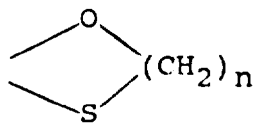 ou 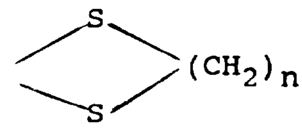

dans laquelle n est égal à 2 ou 3 et les traits ondulés symbolisent l'une quelconque des formes isomères ou leurs mélanges, caractérisé en ce que l'on traite le composé de formule (II) :

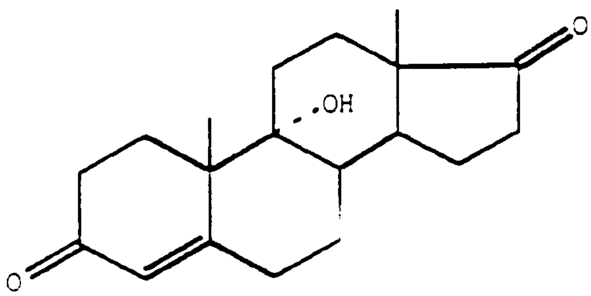

(II)

par l'anhydride acétique en présence d'un acide fort puis par un agent d'hydrolyse acide, pour obtenir le composé de formule (III) :

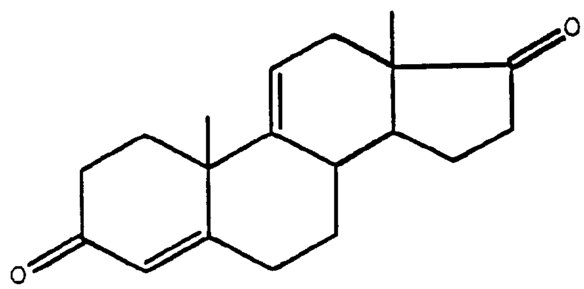

(III)

dont on bloque sélectivement la fonction 3-oxo par action d'un diol, d'un thiol ou d'un dithiol de formule HO-$(CH_2)_n$-OH, HO-$(CH_2)_n$-SH ou HS-$(CH_2)_n$-SH dans laquelle n est défini comme ci-dessus pour obtenir un composé de formule (IV) :

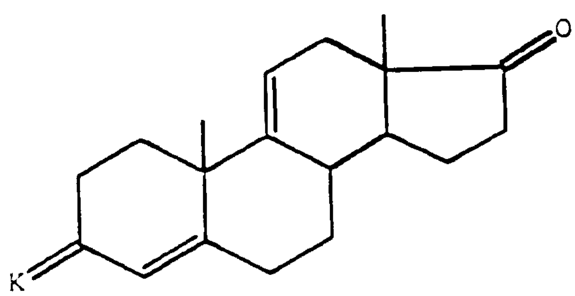

(IV)

dans laquelle K est défini comme ci-dessus sur lequel l'on fait réagir un composé de formule :

$$Hal_3C\text{-}CO_2R$$

dans laquelle Hal et R sont définis comme ci-dessus en présence du zinc et d'un acide de Lewis pour obtenir le composé de formule (I) attendu.

**2.** Procédé selon la revendication 1, caractérisé en ce que l'on utilise comme agent de blocage de la fonction 3-oxo un dithiol de formule HS-$(CH_2)_n$-SH dans laquelle n est égal à 2 ou 3 et en ce que l'on fait réagir sur le composé de formule (IV) le composé de formule $Cl_3C\text{-}CO_2R$ dans laquelle R représente un radical alkyle renfermant de 1

à 3 atomes de carbone.

3. Procédé selon la revendication 1 ou 2, caractérisé en ce que :

- l'acide fort en présence duquel on fait réagir l'anhydride acétique sur le composé de formule (II) est l'acide paratoluène sulfonique,
- l'agent d'hydrolyse acide est l'acide formique,
- l'agent de blocage de la fonction 3-oxo est l'éthane dithiol, utilisé en présence d'une quantité catalytique d'acide chlorhydrique ou bromhydrique concentré,
- l'acide de Lewis est le tétrachlorure de titane.

4. Procédé selon l'une quelconque des revendications 1 à 3, caractérisé en ce que l'on utilise comme agent de blocage de la fonction oxo l'éthanethiol puis fait réagir sur le composé de formule (IV) le trichloroacétate de méthyle.

5. Application des composés de formule (I) telle que définie à la revendication 1, à la préparation des composés de formule (A) :

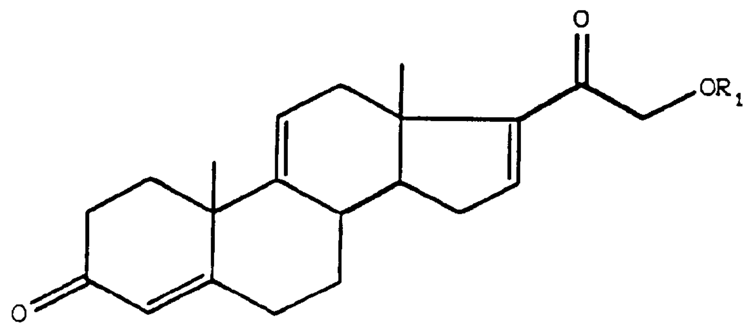

(A)

dans laquelle $R_1$ représente un radical acyle renfermant de 1 à 8 atomes de carbone, caractérisé en ce que l'on traite ledit composé de formule (I)

- soit en milieu basique, par un phénol de formule :

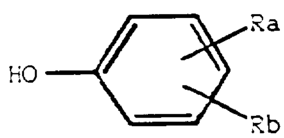

dans laquelle $R_a$ et $R_b$, identiques ou différents, représentent un atome d'hydrogène, un radical alkyle renfermant de 1 à 4 atomes de carbone, un radical alkoxy renfermant de 1 à 4 atomes de carbone, ou un radical hydroxy, pour obtenir un composé de formule (V) :

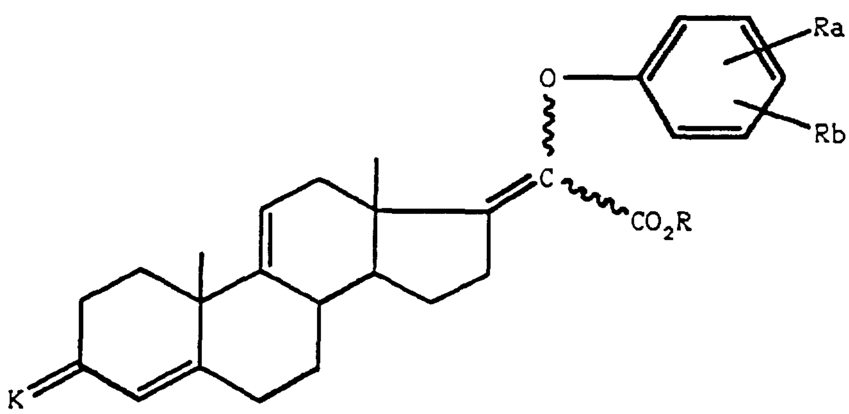

(V)

dans laquelle K et R sont définis comme à la revendication 1 et $R_a$ et $R_b$ sont définis comme précédemment, que l'on soumet à l'action d'un agent réducteur, pour obtenir un composé de formule (VI) :

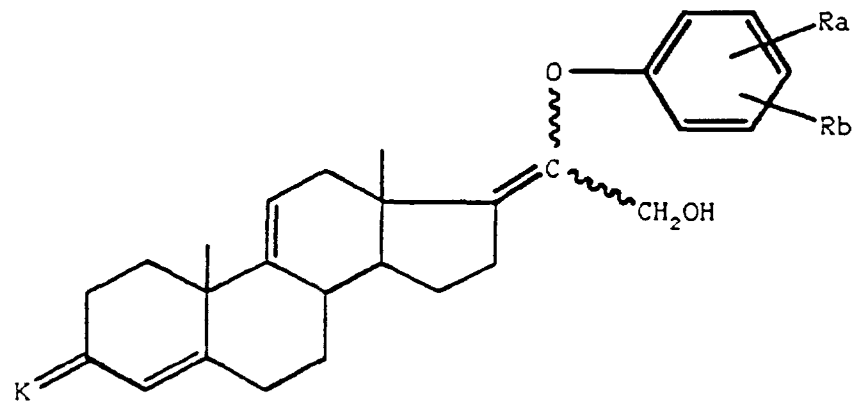

(VI)

dont on déprotège la fonction 3-oxo, puis que l'on traite par un agent d'époxydation, pour obtenir l'époxyde correspondant en position 17,20, que l'on hydrolyse en milieu acide, pour obtenir le composé de formule (VII) :

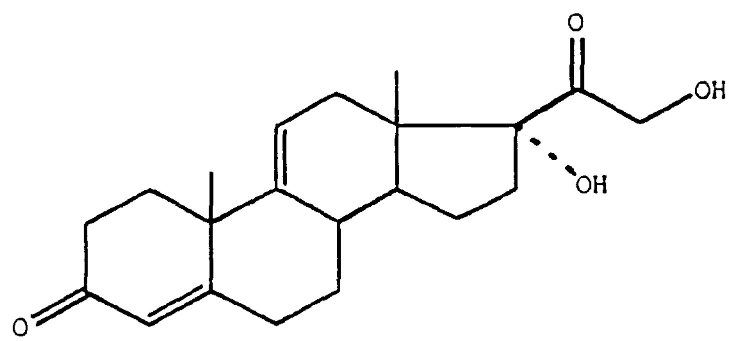

(VII)

dont on acyle les fonctions hydroxy pour obtenir le composé de formule (VIII) :

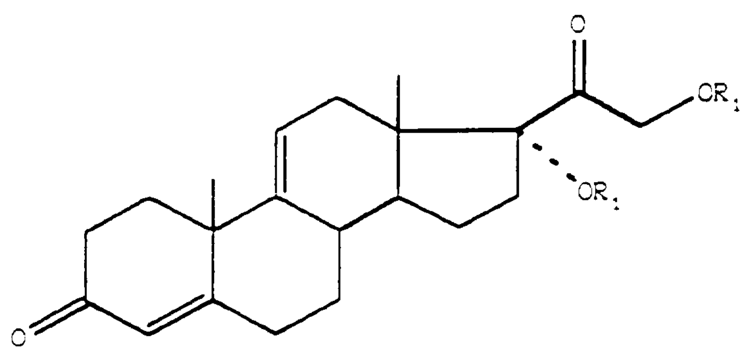

(VIII)

dans laquelle $R_1$ est défini comme précédemment, que l'on soumet à l'action d'un agent d'élimination du radical 17α-$OR_1$, pour obtenir le composé de formule (A) attendu,

- soit en milieu basique par un sulfinate alcalin de formule :

$$R_2\text{-}SO_2\text{-}Q$$

dans laquelle $R_2$ représente un radical méthyle, phényle, tolyle ou xylyle et Q représente un métal alcalin, en présence d'une base, pour obtenir après saponification et décarboxylation, un composé de formule (IX) :

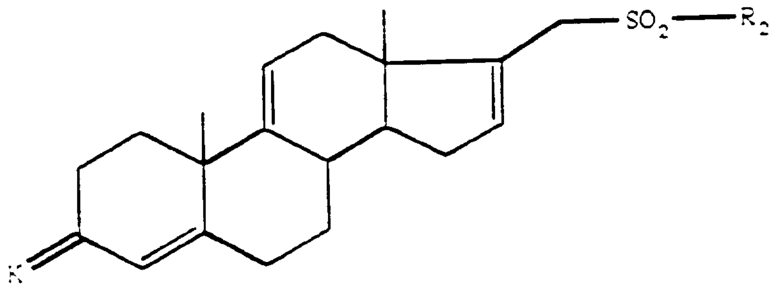

(IX)

dans laquelle K et $R_2$ sont définis comme précédemment, que l'on soumet à l'action du formaldéhyde en présence d'une base, pour obtenir un composé de formule (X) :

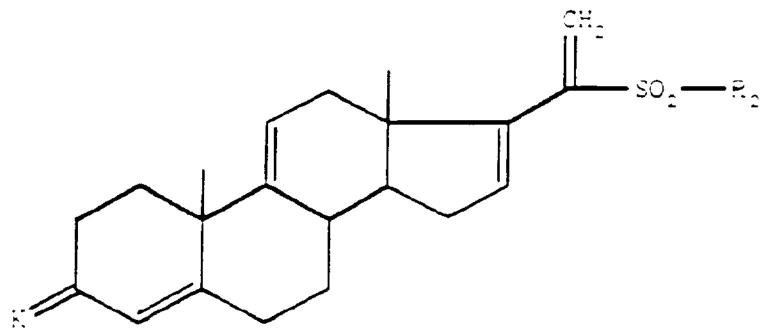

(X)

que l'on soumet à l'action d'un agent d'époxydation en milieu alcalin, pour obtenir un composé de formule (XI) :

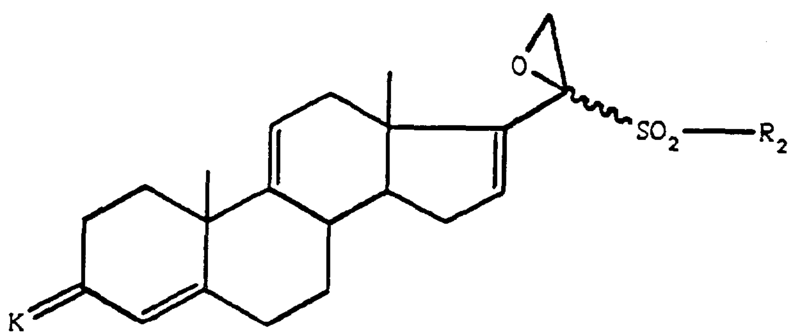

(XI)

dont on ouvre la fonction époxyde en milieu basique et en présence d'ions $R_1O^{\theta}$ , dans lesquels $R_1$ est défini comme ci-dessus, pour obtenir un composé de formule (XII) :

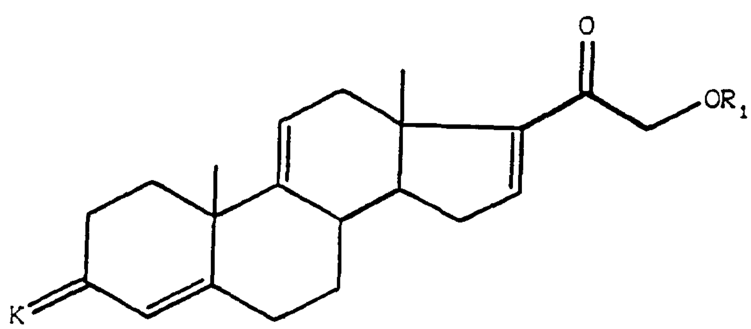

(XII)

dont on déprotège la fonction 3-oxo, pour obtenir le composé de formule (A) attendu ou bien composé de formule (XI) que l'on traite par un halogénure alcalin pour obtenir le composé de formule (XV) :

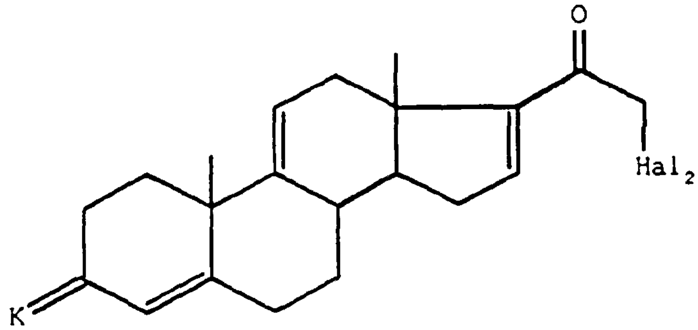

(XV)

dans laquelle $Hal_2$ représente un atome de chlore, de brome ou d'iode et K est défini comme précédemment, que l'on soumet à l'action d'un agent d'acyloxylation en milieu alcalin, pour obtenir le composé de formule (XII) telle que définie ci-dessus puis poursuit la synthèse comme décrit ci-dessus,

- soit par un sulfinate alcalin de formule $R_2SO_2Q$, telle que définie précédemment, en présence d'une base, pour obtenir un composé de formule (XIII) :

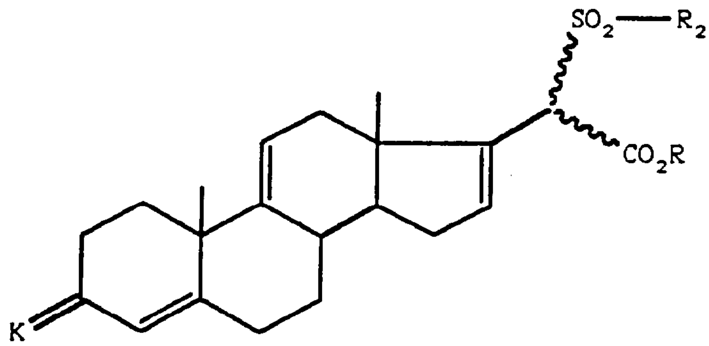

(XIII)

dans laquelle R, $R_2$ et K sont définis comme précédemment, que l'on traite par un agent d'halogénation en présence d'une base, puis que l'on saponifie et décarboxyle pour obtenir un composé de formule (XIV) :

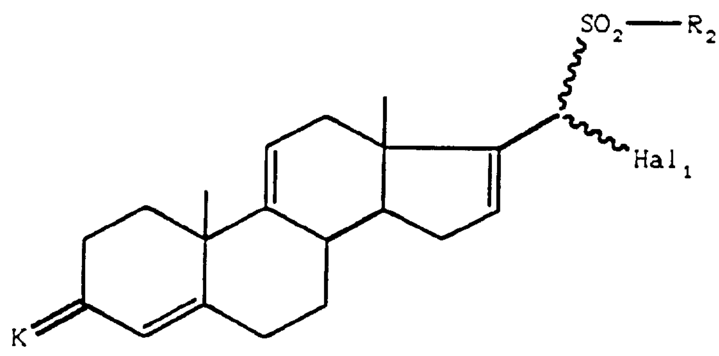

(XIV)

dans laquelle $Hal_1$ représente un atome d'halogène, que l'on traite par le formaldéhyde en présence d'une base, pour obtenir le composé de formule (XI) telle que définie ci-dessus, que l'on traite comme décrit ci-dessus, pour obtenir le composé de formule (A) désiré,

- soit par un agent réducteur, pour obtenir un composé de formule (XVI) :

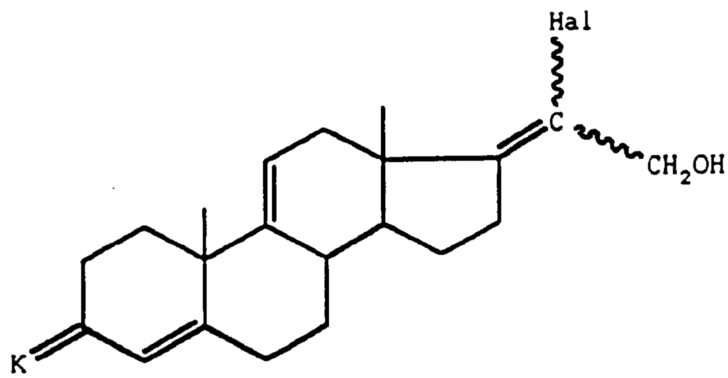

(XVI)

dans laquelle K et Hal sont définis comme à la revendication 1, que l'on soumet à l'action d'un agent oxydant, pour obtenir un composé de formule (XVII) :

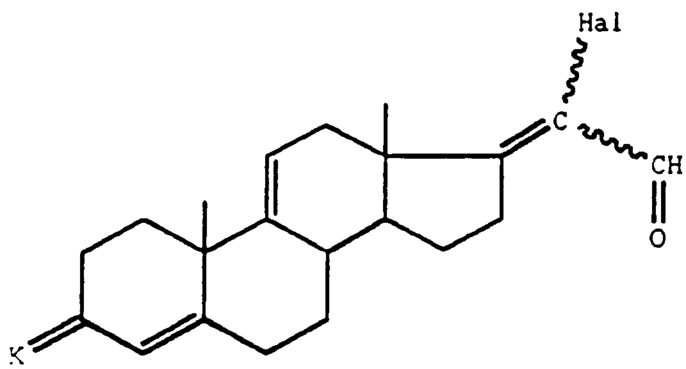

(XVII)

que l'on soumet à l'action d'un agent d'acyloxylation pour obtenir un composé de formule (XII) telle que définie ci-dessus, puis poursuit la synthèse comme décrit ci-dessus pour obtenir le composé de formule (A) attendu.

**6.** Application selon la revendication 5, caractérisé en ce que :

- $R_a$ et $R_b$ représentent un atome d'hydrogène, un radical hydroxy ou un radical méthyle,
- l'agent d'acylation est l'anhydride acétique ou le chlorure d'acétyle,
- le sulfinate alcalin est le toluènesulfinate de sodium,
- l'ouverture de l'époxyde est effectuée en présence d'ions acétate,
- l'halogénure alcalin est le bromure de lithium,
- l'agent d'acyloxylation est l'acétate de potassium,
- l'agent d'halogénation utilisé est l'hypochlorite ou l'hypobromite de sodium.

**Revendications pour l'Etat contractant suivant : GR**

**1.** Procédé pour préparer les composés de formule (I) :

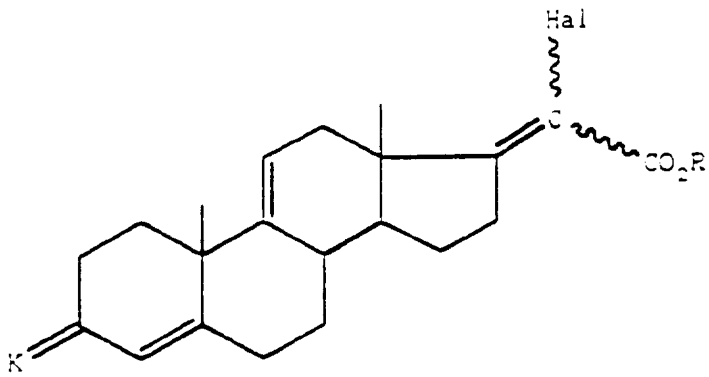

(I)

dans laquelle Hal représente un atome de chlore ou de brome, R représente un radical alkyle renfermant de 1 à 6 atomes de carbone, un radical aralkyle renfermant de 7 à 15 atomes de carbone ou un reste trialkylsilyle, triphénylsilyle ou diphényl tert-butyle silyle, K représente un groupement protecteur du radical oxo de formule :

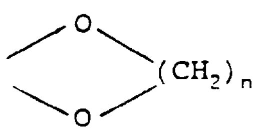 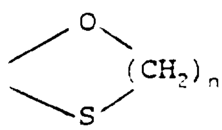 ou 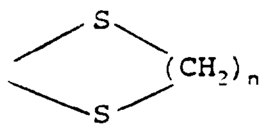

dans laquelle n est égal à 2 ou 3 et les traits ondulés symbolisent l'une quelconque des formes isomères ou leurs mélanges, caractérisé en ce que l'on traite le composé de formule (II) :

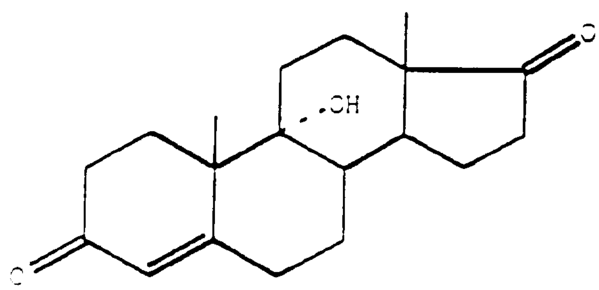

(II)

par l'anhydride acétique en présence d'un acide fort puis par un agent d'hydrolyse acide, pour obtenir le composé de formule (III) :

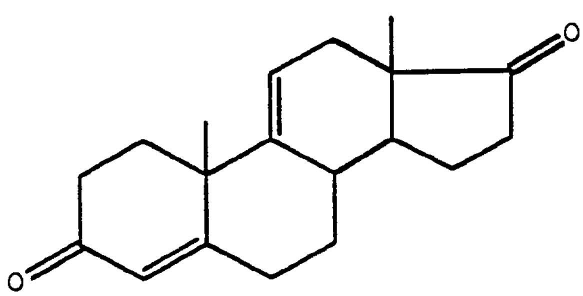

(III)

dont on bloque sélectivement la fonction 3-oxo par action d'un diol, d'un thiol ou d'un dithiol de formule $HO-(CH_2)_n-OH$, $HO-(CH_2)_n-SH$ ou $HS-(CH_2)_n-SH$ dans laquelle n est défini comme ci-dessus pour obtenir un composé de formule (IV) :

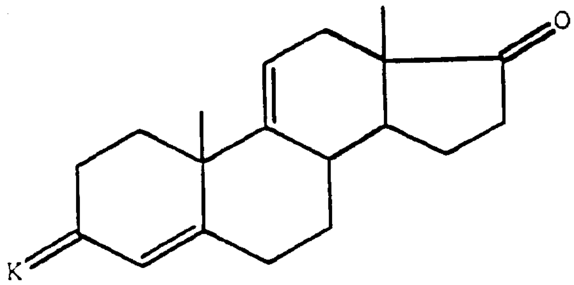

(IV)

dans laquelle K est défini comme ci-dessus sur lequel l'on fait réagir un composé de formule :

$$Hal_3C\text{-}CO_2R$$

dans laquelle Hal et R sont définis comme ci-dessus en présence du zinc et d'un acide de Lewis pour obtenir le composé de formule (I) attendu.

**2.** Procédé selon la revendication 1, caractérisé en ce que l'on utilise comme agent de blocage de la fonction 3-oxo un dithiol de formule $HS\text{-}(CH_2)_n\text{-}SH$ dans laquelle n est égal à 2 ou 3 et en ce que l'on fait réagir sur le composé de formule (IV) le composé de formule $Cl_3C\text{-}CO_2R$ dans laquelle R représente un radical alkyle renfermant de 1 à 3 atomes de carbone.

**3.** Procédé selon la revendication 1 ou 2, caractérisé en ce que :

- l'acide fort en présence duquel on fait réagir l'anhydride acétique sur le composé de formule (II) est l'acide paratoluène sulfonique,
- l'agent d'hydrolyse acide est l'acide formique,
- l'agent de blocage de la fonction 3-oxo est l'éthane dithiol, utilisé en présence d'une quantité catalytique d'acide chlorhydrique ou bromhydrique concentré,
- l'acide de Lewis est le tétrachlorure de titane.

**4.** Procédé selon l'une quelconque des revendications 1 à 3, caractérisé en ce que l'on utilise comme agent de blocage de la fonction oxo l'éthanethiol puis fait réagir sur le composé de formule (IV) le trichloroacétate de méthyle.

**5.** Application des composés de formule (I) telle que définie à la revendication 1, à la préparation des composés de formule (A) :

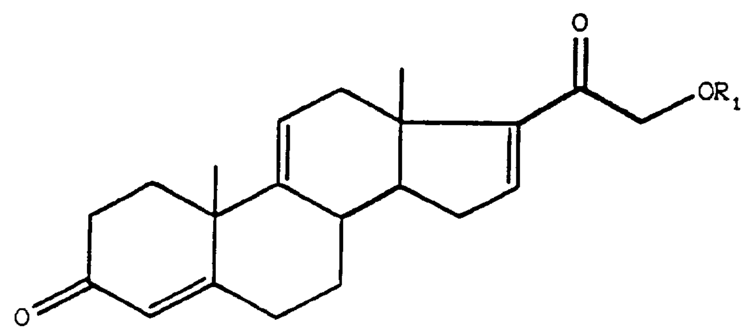

(A)

dans laquelle $R_1$ représente un radical acyle renfermant de 1 à 8 atomes de carbone, caractérisé en ce que l'on traite ledit composé de formule (I)

- soit en milieu basique, par un phénol de formule :

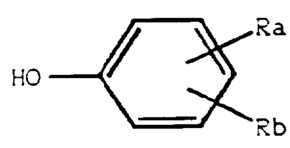

dans laquelle $R_a$ et $R_b$, identiques ou différents, représentent un atome d'hydrogène, un radical alkyle renfermant de 1 à 4 atomes de carbone, un radical alkoxy renfermant de 1 à 4 atomes de carbone, ou un radical hydroxy, pour obtenir un composé de formule (V) :

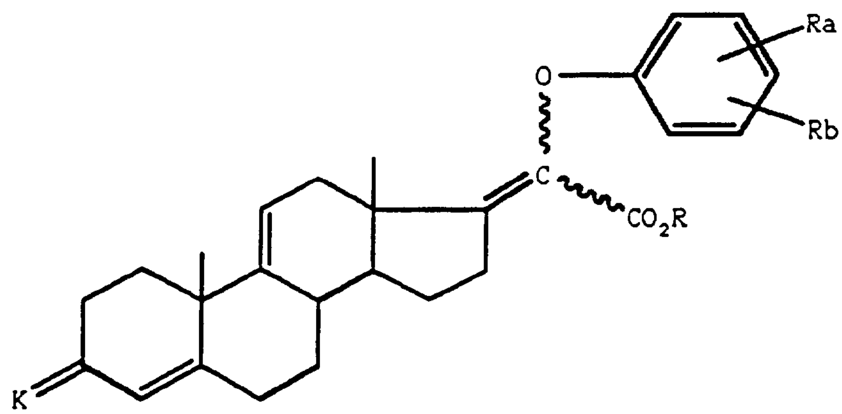

(V)

dans laquelle K et R sont définis comme à la revendication 1 et $R_a$ et $R_b$ sont définis comme précédemment, que l'on soumet à l'action d'un agent réducteur, pour obtenir un composé de formule (VI) :

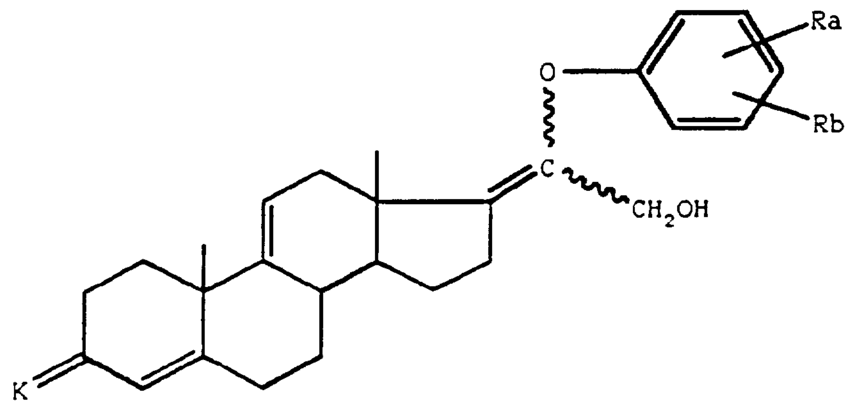

(VI)

dont on déprotège la fonction 3-oxo, puis que l'on traite par un agent d'époxydation, pour obtenir l'époxyde correspondant en position 17,20, que l'on hydrolyse en milieu acide, pour obtenir le composé de formule (VII) :

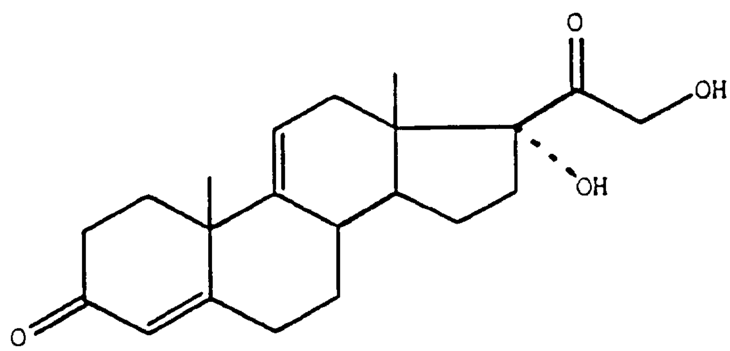

(VII)

dont on acyle les fonctions hydroxy pour obtenir le composé de formule (VIII) :

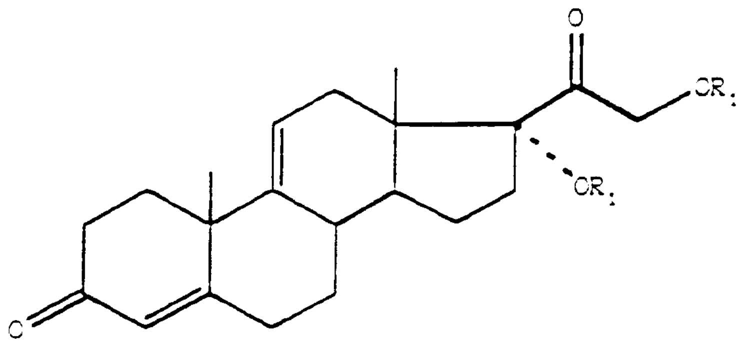

(VIII)

dans laquelle $R_1$ est défini comme précédemment, que l'on soumet à l'action d'un agent d'élimination du radical 17α-$OR_1$, pour obtenir le composé de formule (A) attendu,

- soit en milieu basique par un sulfinate alcalin de formule :

$$R_2\text{-}SO_2\text{-}Q$$

dans laquelle $R_2$ représente un radical méthyle, phényle, tolyle ou xylyle et Q représente un métal alcalin, en présence d'une base, pour obtenir après saponification et décarboxylation, un composé de formule (IX) :

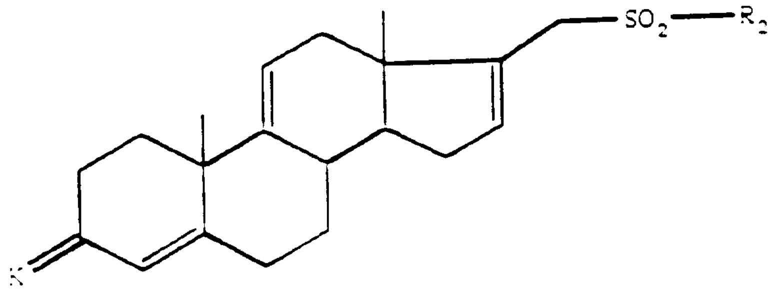

(IX)

dans laquelle K et $R_2$ sont définis comme précédemment, que l'on soumet à l'action du formaldéhyde en présence d'une base, pour obtenir un composé de formule (X) :

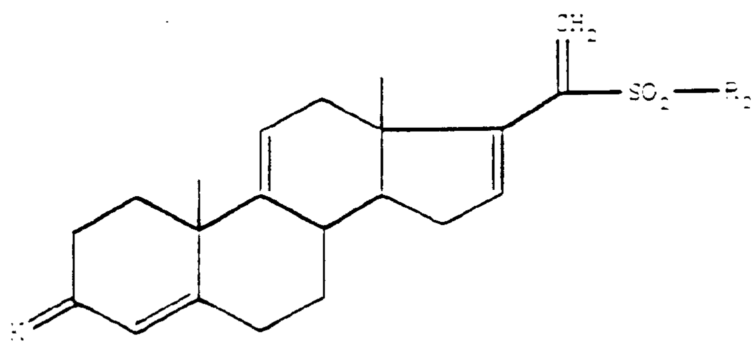

(X)

que l'on soumet à l'action d'un agent d'époxydation en milieu alcalin, pour obtenir un composé de formule (XI) :

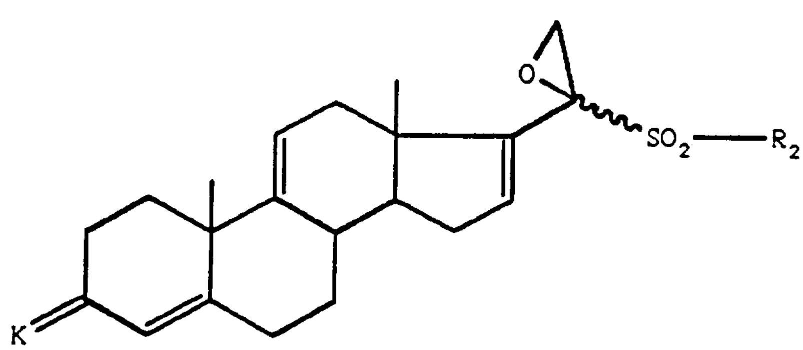

(XI)

dont on ouvre la fonction époxyde en milieu basique et en présence d'ions $R_1O^{\theta}$, dans lesquels $R_1$ est défini comme ci-dessus, pour obtenir un composé de formule (XII) :

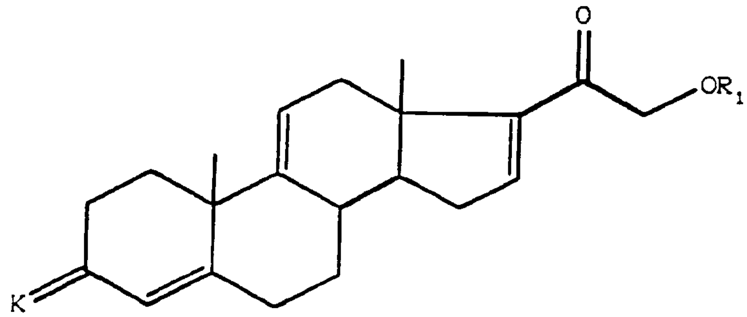

(XII)

dont on déprotège la fonction 3-oxo, pour obtenir le composé de formule (A) attendu ou bien composé de formule (XI) que l'on traite par un halogénure alcalin pour obtenir le composé de formule (XV) :

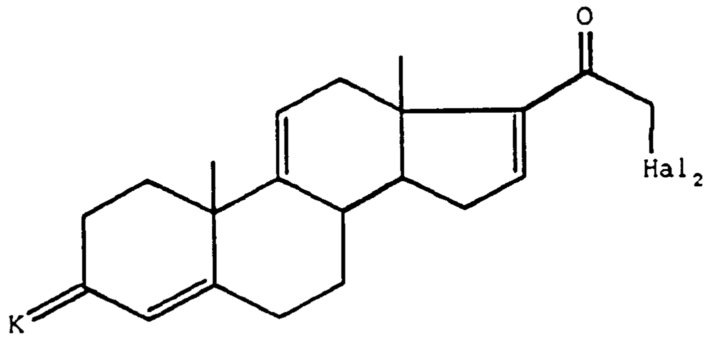

(XV)

dans laquelle $Hal_2$ représente un atome de chlore, de brome ou d'iode et K est défini comme précédemment, que l'on soumet à l'action d'un agent d'acyloxylation en milieu alcalin, pour obtenir le composé de formule (XII) telle que définie ci-dessus puis poursuit la synthèse comme décrit ci-dessus,

- soit par un sulfinate alcalin de formule $R_2SO_2Q$, telle que définie précédemment, en présence d'une base, pour obtenir un composé de formule (XIII) :

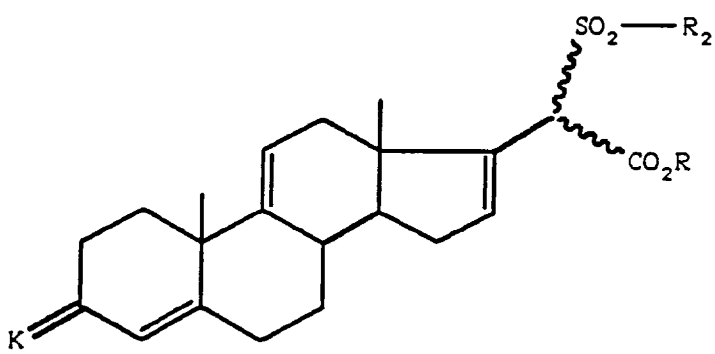

(XIII)

dans laquelle R, $R_2$ et K sont définis comme précédemment, que l'on traite par un agent d'halogénation en présence d'une base, puis que l'on saponifie et décarboxyle pour obtenir un composé de formule (XIV) :

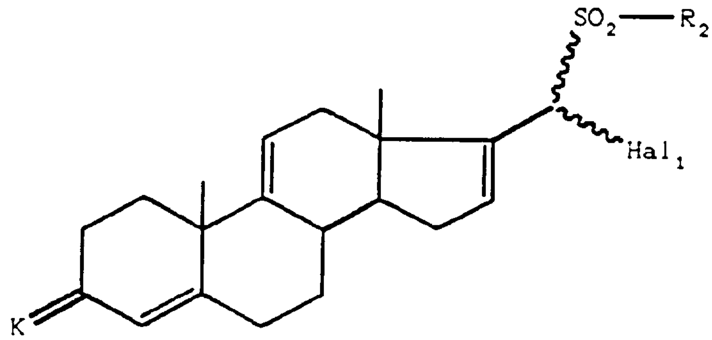

(XIV)

dans laquelle $Hal_1$ représente un atome d'halogène, que l'on traite par le formaldéhyde en présence d'une base, pour obtenir le composé de formule (XI) telle que définie ci-dessus, que l'on traite comme décrit ci-dessus, pour obtenir le composé de formule (A) désiré,

- soit par un agent réducteur, pour obtenir un composé de formule (XVI) :

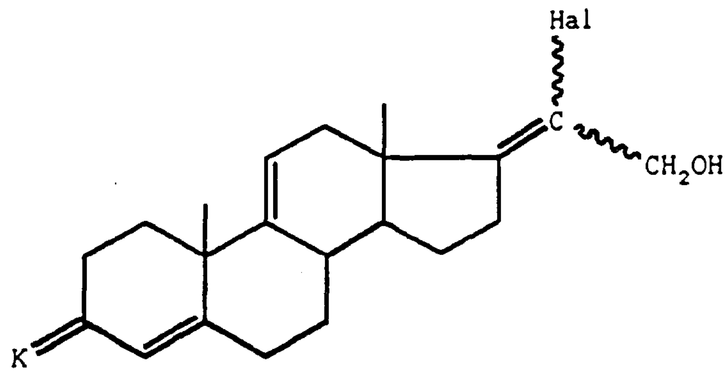

(XVI)

dans laquelle K et Hal sont définis comme à la revendication 1, que l'on soumet à l'action d'un agent oxydant, pour obtenir un composé de formule (XVII) :

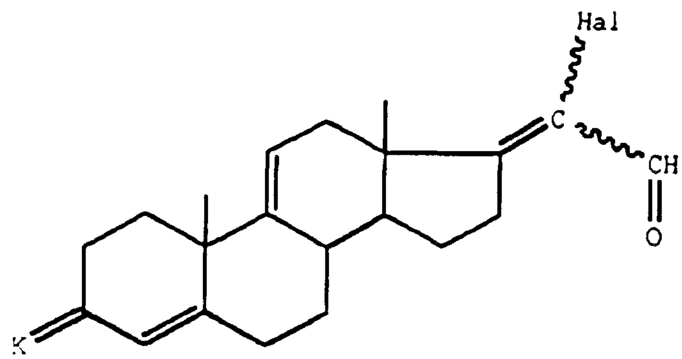

(XVII)

que l'on soumet à l'action d'un agent d'acyloxylation pour obtenir un composé de formule (XII) telle que définie ci-dessus, puis poursuit la synthèse comme décrit ci-dessus pour obtenir le composé de formule (A) attendu.

**6.** Application selon la revendication 5, caractérisé en ce que :

- $R_a$ et $R_b$ représentent un atome d'hydrogène, un radical hydroxy ou un radical méthyle,
- l'agent d'acylation est l'anhydride acétique ou le chlorure d'acétyle,
- le sulfinate alcalin est le toluènesulfinate de sodium,
- l'ouverture de l'époxyde est effectuée en présence d'ions acétate,
- l'halogénure alcalin est le bromure de lithium,
- l'agent d'acyloxylation est l'acétate de potassium,
- l'agent d'halogénation utilisé est l'hypochlorite ou l'hypobromite de sodium.

**7.** A titre de produits industriels :

- les composés de formule (F) :

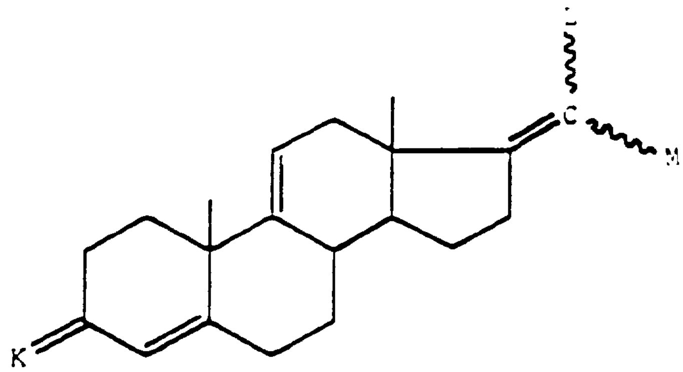

(F)

dans laquelle K est défini comme à la revendication 1, et

<u>soit</u> L représente un groupement

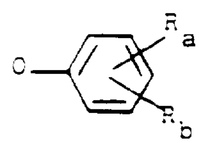

,

$R_a$ et $R_b$ identiques ou différents, représentant un atome d'hydrogène, un radical alkyle renfermant de 1 à 4 atomes de carbone, un radical alkoxy renfermant de 1 à 4 atomes de carbone ou un radical hydroxy, M représente un radical $CH_2OH$ ou $CO_2R$, R représentant un radical alkyle renfermant de 1 à 6 atomes de carbone, un radical aralkyle renfermant de 7 à 15 atomes de carbone ou un reste trialkylsilyle, triphénylsilyle ou diphényl tert-butyle silyle,
<u>soit</u> L représente un atome de chlore ou de brome et M représente un radical $CH_2OH$,
et les traits ondulés symbolisent l'une quelconque des formes isomères ou leurs mélanges.

**8.** A titre de produits industriels, les produits de formule (G) :

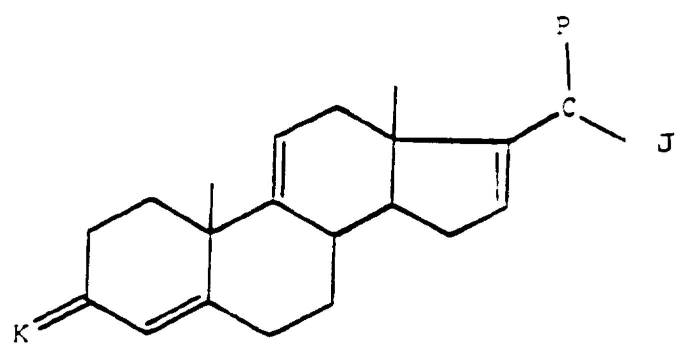

(G)

dans laquelle K est défini comme à la revendication 1, le groupement C-P représente un groupement $CH_2$, $C{=}CH_2$ ou

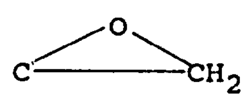

et le groupement C-J représente un groupement $C\text{-}SO_2R_2$, <u>ou bien</u> le groupement C-P représente un groupement

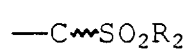

et le groupement C-J représente un groupement

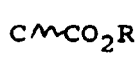

ou C-$Hal_1$, $R_2$ représentant un radical méthyle, phényle, tolyle ou xylyle, R représentant un radical alkyle renfermant de 1 à 6 atomes de carbone, un radical aralkyle renfermant de 7 à 15 atomes de carbone ou un reste trialkylsilyle, triphénylsilyle ou diphényl tert-butyle silyle, $Hal_1$ représente un atome d'halogène, notamment un atome de chlore ou de brome, les traits ondulés symbolisant l'une quelconque des formes isomères ou leurs mélanges.

## Patentansprüche

### Patentansprüche für folgende Vertragsstaaten : AT, BE, CH, DE, DK, FR, GB, IT, LI, LU, NL, PT, SE

**1.** Verbindungen der Formel (I)

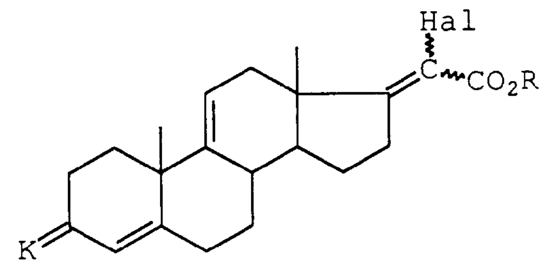

(I)

in der Hal ein Chloratom oder Bromatom darstellt, R einen Alkylrest mit 1 bis 6 Kohlenstoffatomen, einen Aralkylrest mit 7 bis 15 Kohlenstoffatomen oder einen Rest Trialkylsilyl, Triphenylsilyl oder Diphenyl-tert.-butylsilyl bedeutet und K eine Schutzgruppe der Formel

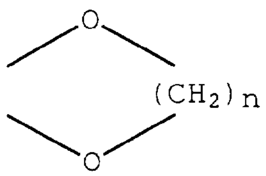 , 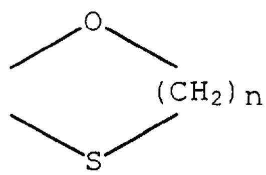 oder 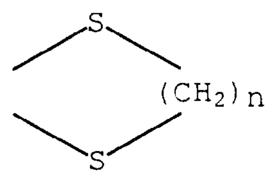

für den Oxorest darstellt, worin n gleich 2 oder 3 ist und die Wellenlinien irgendeine der isomeren Formen oder ihre Mischungen symbolisieren.

**2.** Verbindungen der Formel (I) wie in Anspruch 1 definiert, worin Hal ein Chloratom darstellt, R einen Alkylrest mit 1 bis 3 Kohlenstoffatomen bedeutet und K eine Gruppe der Formel

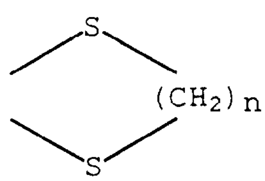

darstellt, in der n gleich 2 oder 3 ist.

**3.** Verbindung nach Anspruch 1 mit dem folgenden Namen: 20-Chlor-3,3-[(1,2-ethandiyl)-bis(thio)]-pregna-4,9(11), 17(20) - trien-21-oat von Methyl.

**4.** Verfahren zur Herstellung der Verbindungen der Formel (I) wie in Anspruch 1 definiert, dadurch gekennzeichnet, daß man die Verbindung der Formel (II)

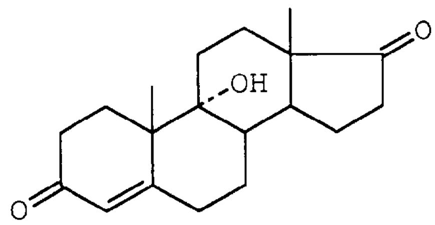

(II)

mit Essigsäureanhydrid in Anwesenheit einer starken Säure und anschließend mit einem Mittel zur sauren Hydrolyse behandelt, um die Verbindung der Formel (III)

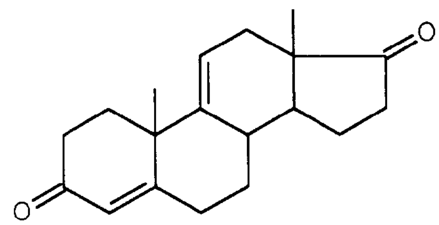

(III)

zu erhalten, bei der man selektiv die 3-Oxo-Funktion durch Einwirkung eines Diols, Thiols oder Dithiols der Formel $HO-(CH_2)_n-OH$, $HO-(CH_2)_n-SH$ oder $HS-(CH_2)_n-SH$ blockiert, worin n wie in Anspruch 1 definiert ist, um eine Verbindung der Formel (IV)

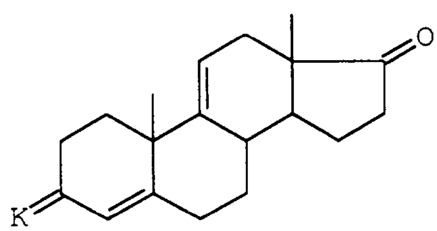

(IV)

zu erhalten, in der K wie in Anspruch 1 definiert ist, die man dann mit einer Verbindung der Formel

$$Hal_3\text{-}C\text{-}CO_2R$$

in der Hal und R wie in Anspruch 1 definiert sind, in Anwesenheit von Zink und einer Lewis-Säure zur Reaktion bringt, um die erwartete Verbindung der Formel (I) zu erhalten.

**5.** Verfahren nach Anspruch 4, dadurch gekennzeichnet, daß

- die starke Säure, in deren Anwesenheit man das Essigsäureanhydrid mit der Verbindung der Formel (II) zur Reaktion bringt, die para-Toluolsulfonsäure ist,
- das Mittel zur sauren Hydrolyse die Ameisensäure ist,
- das Mittel zur Blockierung der 3-Oxo-Funktion das Ethandithiol ist, verwendet in Anwesenheit einer katalytischen Menge von konzentrierter Chlorwasserstoffsäure oder Bromwasserstoffsäure,
- die Lewis-Säure das Titantetrachlorid ist.

**6.** Verwendung der Verbindungen der Formel (I) wie in Anspruch 1 definiert zur Herstellung von Verbindungen der Formel (A)

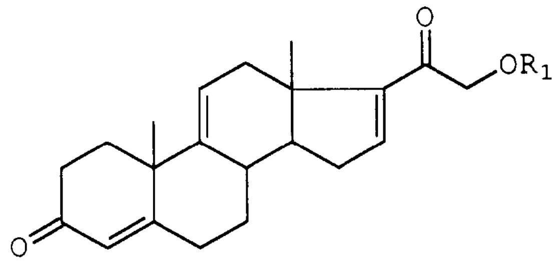

(A)

in der $R_1$ einen Acylrest mit 1 bis 8 Kohlenstoffatomen darstellt, dadurch gekennzeichnet, daß man eine Verbindung der Formel (I) im basischen Medium mit einem Phenol der Formel

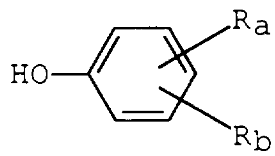

behandelt, in der $R_a$ und $R_b$, gleich oder verschieden, ein Wasserstoffatom, einen Alkylrest mit 1 bis 4 Kohlenstoffatomen, einen Alkoxyrest mit 1 bis 4 Kohlenstoffatomen oder einen Hydroxyrest darstellen, um eine Verbindung der Formel (V)

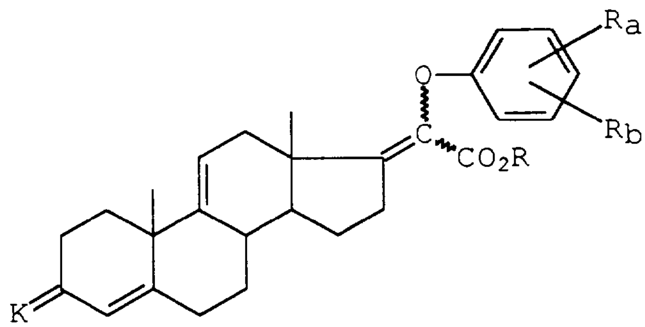

(V)

zu erhalten, in der K und R wie in Anspruch 1 und $R_a$ und $R_b$ wie vorstehend definiert sind, die man dann der Einwirkung eines Reduktionsmittels unterzieht, um eine Verbindung der Formel (VI)

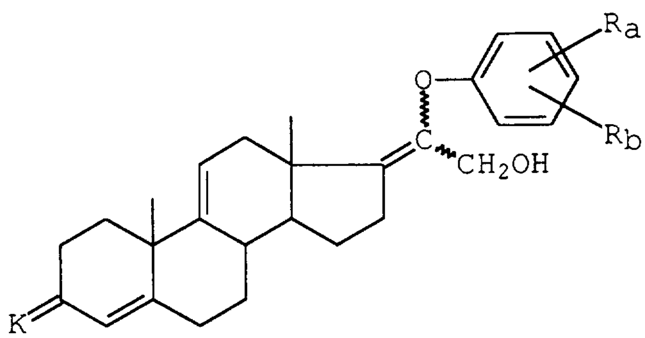

(VI)

zu erhalten, bei der man die Schutzgruppe von der 3-Oxo-Funktion entfernt, und die man an schließend mit einem Epoxidierungsmittel behandelt, um das entsprechende Epoxid in Position 17,20 zu erhalten, das man danach im sauren Medium hydrolysiert, um die Verbindung der Formel (VII)

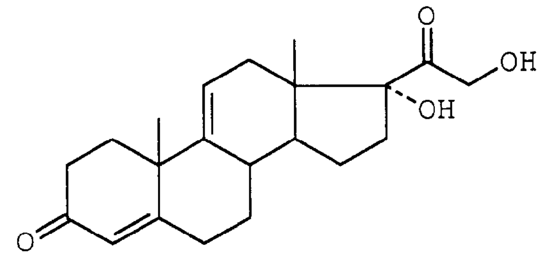

(VII)

zu erhalten, bei der man die Hydroxyfunktionen acyliert, um die Verbindung der Formel (VIII)

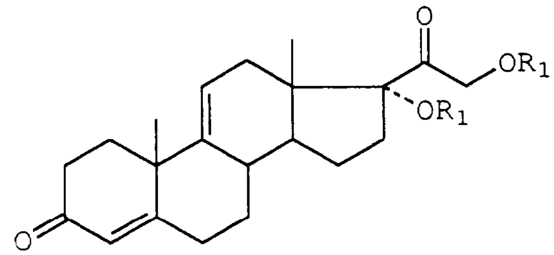

(VIII)

zu erhalten, in der $R_1$ wie oben definiert ist, die man anschließend der Einwirkung eines Mittels zur Eliminierung des Restes 17α-$OR_1$ unterwirft, um die erwartete Verbindung der Formel (A) zu erhalten.

**7.** Verwendung nach Anspruch 6, dadurch gekennzeichnet, daß

- $R_a$ und $R_b$ ein Wasserstoffatom, einen Hydroxyrest oder einen Methylrest darstellen,
- das Acylierungsmittel Essigsäureanhydrid oder Acetylchlorid ist.

**8.** Verwendung der Verbindungen der Formel (I) wie in Anspruch 1 definiert zur Herstellung von Verbindungen der Formel (A) wie in Anspruch 6 definiert, dadurch gekennzeichnet, daß man eine Verbindung der Formel (I) im basischen Medium mit einem Alkalisulfinat der Formel

$$R_2\text{-}SO_2\text{-}Q$$

in der $R_2$ einen Rest Methyl, Phenyl, Tolyl oder Xylyl darstellt und Q ein Alkalimetall bedeutet, in Anwesenheit einer Base behandelt, um nach Verseifung und Decarboxylierung eine Verbindung der Formel (IX)

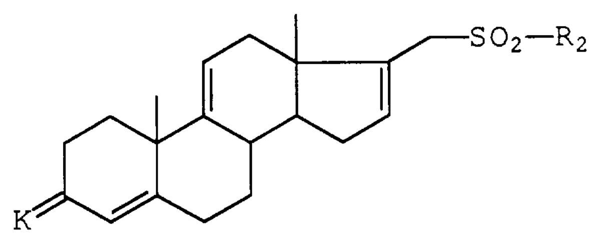

(IX)

zu erhalten, in der K und $R_2$ wie vorstehend definiert sind, die man dann der Einwirkung von Formaldehyd in Anwesenheit einer Base unterzieht, um eine Verbindung der Formel (X)

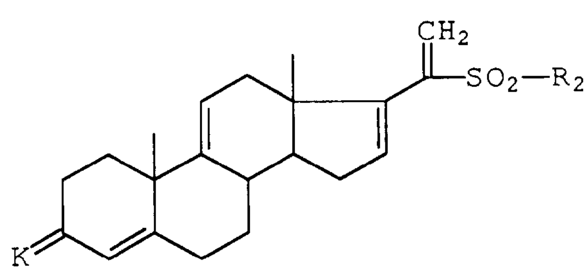

(X)

zu erhalten, die man anschließend der Einwirkung eines Mittels zur Epoxidierung im alkalischen Medium unterwirft,

um eine Verbindung der Formel (XI)

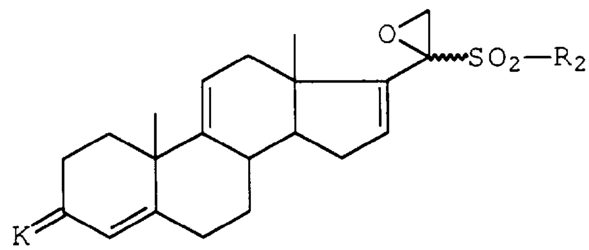

(XI)

zu erhalten, bei der man die Epoxid-Funktion im basischen Medium und in Anwesenheit von Ionen $R_1O^-$, worin $R_1$ wie in Anspruch 6 definiert ist, öffnet, um eine Verbindung der Formel (XII)

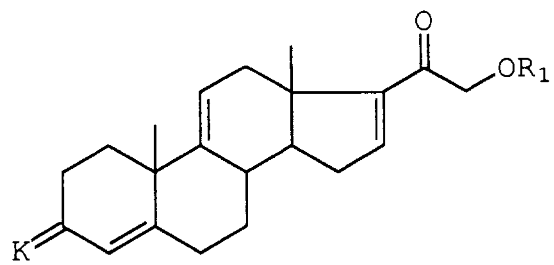

(XII)

zu erhalten, bei der man die Schutzgruppe von der 3-Oxo-Funktion entfernt, um die erwartete Verbindung der Formel (A) zu erhalten.

**9.** Verwendung nach Anspruch 8, dadurch gekennzeichnet, daß

- das Alkalisulfinat das Natrium-toluolsulfinat ist,
- die Öffnung des Epoxides in Anwesenheit von Acetationen durchgeführt wird.

**10.** Verwendung der Verbindungen der Formel (I) wie in Anspruch 1 definiert zur Herstellung von Verbindungen der Formel (A) wie in Anspruch 6 definiert, dadurch gekennzeichnet, daß man eine Verbindung der Formel (I) mit einem wie vorstehend definierten Alkalisulfinat der Formel $R_2$-$SO_2$-Q in Anwesenheit einer Base behandelt, um eine Verbindung der Formel (XIII)

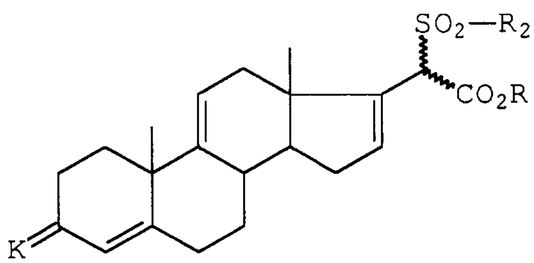

(XIII)

zu erhalten, in der R, $R_2$ und K wie vorstehend definiert sind, die man mit einem Halogenierungsmittel in Anwesenheit einer Base behandelt, und die man anschließend verseift und decarboxyliert, um eine Verbindung der Formel (XIV)

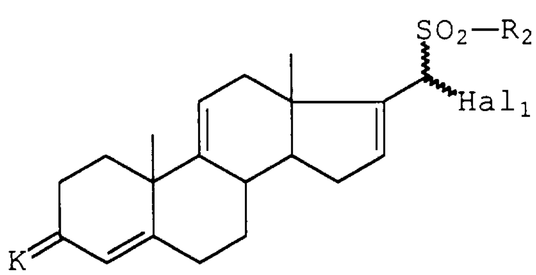

(XIV)

zu erhalten, in der $Hal_1$ ein Halogenatom darstellt, die man danach mit Formaldehyd in Anwesenheit einer Base

behandelt, um die wie in Anspruch 8 definierte Verbindung der Formel (XI) zu erhalten, die man weiter wie in Anspruch 8 beschrieben behandelt, um die gewünschte Verbindung der Formel (A) zu erhalten.

**11.** Verwendung nach Anspruch 10, dadurch gekennzeichnet, daß

- das verwendete Alkalisulfinat das Natrium-toluolsulfinat ist,
- das verwendete Halogenierungsmittel das Hypochlorit oder das Hypobromit von Natrium ist,
- die Öffnung des Epoxides in Anwesenheit von Acetationen durchgeführt wird.

**12.** Verwendung der Verbindungen der Formel (I) wie in Anspruch 1 definiert zur Herstellung von Verbindungen der Formel (A), dadurch gekennzeichnet, daß man die Verbindung der Formel (XI), die ausgehend von den Verbindungen der Formel (I) gemäß den in den Ansprüchen 8 oder 10 beschriebenen Methoden erhalten wurde, mit einem Alkalihalogenid behandelt, um die Verbindung der Formel (XV)

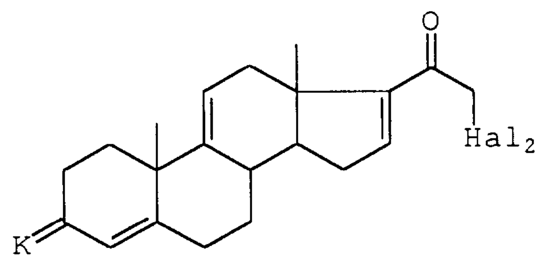

(XV)

zu erhalten, in der $Hal_2$ ein Atom von Chlor, Brom oder Iod darstellt und K wie vorstehend definiert ist, die man dann der Einwirkung eines Mittels zur Acyloxylierung im alkalischen Medium unterwirft, um die wie in Anspruch 8 definierte Verbindung der Formel (XII) zu erhalten, und man anschließend die Synthese wie in Anspruch 8 beschrieben fortsetzt.

**13.** Verwendung nach Anspruch 12, dadurch gekennzeichnet, daß

- das Alkalihalogenid Lithiumbromid ist,
- das Mittel zur Acyloxylierung Kaliumacetat ist.

**14.** Verwendung der Verbindungen der Formel (I) wie in Anspruch 1 definiert zur Herstellung von Verbindungen der Formel (A) wie in Anspruch 6 definiert, dadurch gekennzeichnet, daß man eine Verbindung der Formel (I) mit einem Reduktionsmittel behandelt, um eine Verbindung der Formel (XVI)

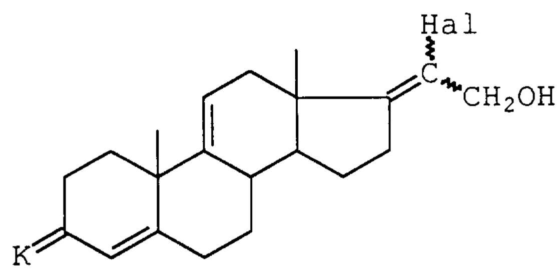

(XVI)

zu erhalten, in der K und Hal wie in Anspruch 1 definiert sind, die man der Einwirkung eines Oxidationsmittels unterzieht, um eine Verbindung der Formel (XVII)

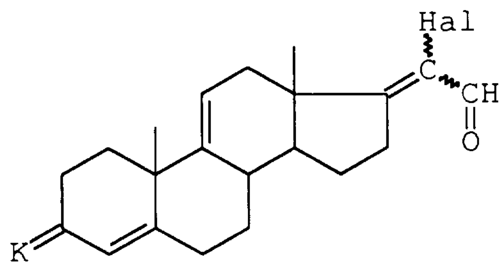

(XVII)

zu erhalten, die man anschließend der Einwirkung eines Mittels zur Acyloxylierung unterwirft, um eine wie in Anspruch 8 definierte Verbindung der Formel (XII) zu erhalten, und man anschließend die Synthese wie in Anspruch 8 beschrieben fortsetzt.

**15.** Als industrielle Produkte

- die Verbindungen der Formel (F)

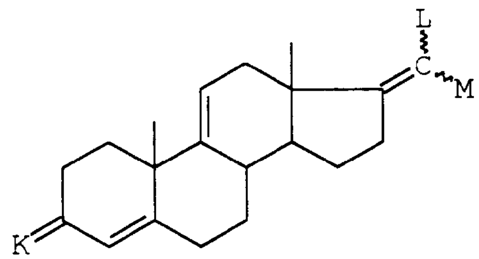

(F)

in der K wie in Anspruch 1 definiert ist, und
entweder L eine Gruppe

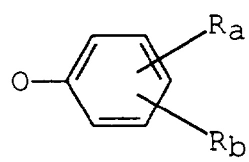

darstellt, worin $R_a$ und $R_b$, gleich oder verschieden, ein Wasserstoffatom, einen Alkylrest mit 1 bis 4 Kohlenstoffatomen, einen Alkoxyrest mit 1 bis 4 Kohlenstoffatomen oder einen Hydroxyrest bedeuten, M einen Rest $CH_2OH$ oder $CO_2R$ darstellt und R einen Alkylrest mit 1 bis 6 Kohlenstoffatomen, einen Aralkylrest mit 7 bis 15 Kohlenstoffatomen oder einen Rest Trialkylsilyl, Triphenylsilyl oder Diphenyl-tert.-butylsilyl bedeutet,
oder L ein Chloratom oder Bromatom darstellt und M einen Rest $CH_2OH$ bedeutet,
und die Wellenlinien irgendeine der isomeren Formen oder ihre Mischungen symbolisieren.

**16.** Als industrielle Produkte die Produkte der Formel (G)

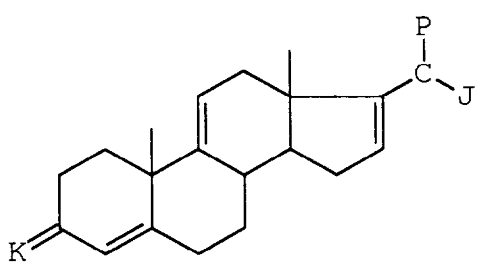

(G)

in der K wie in Anspruch 1 definiert ist, die Gruppe C-P eine Gruppe $CH_2$, $C{=}CH_2$ oder

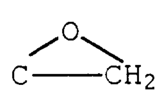

darstellt und die Gruppe C-J eine Gruppe $C\text{-}SO_2R_2$ bedeutet, oder die Gruppe C-P eine Gruppe

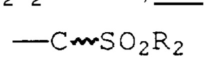

darstellt und die Gruppe C-J eine Gruppe

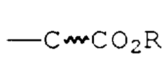

oder C-$Hal_1$ bedeutet,

$R_2$ einen Rest Methyl, Phenyl, Tolyl oder Xylyl darstellt,
R einen Alkylrest mit 1 bis 6 Kohlenstoffatomen, einen Aralkylrest mit 7 bis 15 Kohlenstoffatomen oder einen Rest Trialkylsilyl, Triphenylsilyl oder Diphenyl-tert.-butylsilyl bedeutet, Hall ein Halogenatom darstellt, insbesondere ein Chloratom oder Bromatom,
und die Wellenlinien irgendeine der isomeren Formen oder ihre Mischungen symbolisieren.

**Patentansprüche für folgenden Vertragsstaat : ES**

1. Verfahren zur Herstellung von Verbindungen der Formel (I)

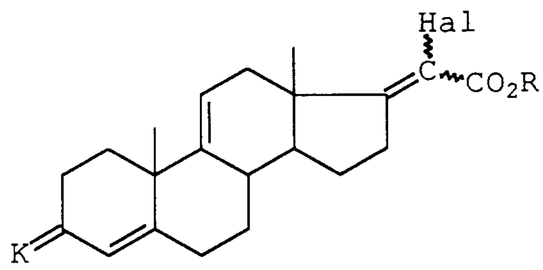

(I)

in der Hal ein Chloratom oder Bromatom darstellt, R einen Alkylrest mit 1 bis 6 Kohlenstoffatomen, einen Aralkylrest mit 7 bis 15 Kohlenstoffatomen oder einen Rest Trialkylsilyl, Triphenylsilyl oder Diphenyl-tert.-butylsilyl bedeutet und K eine Schutzgruppe der Formel

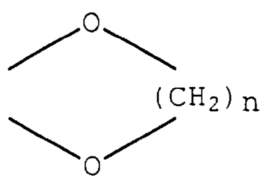 , 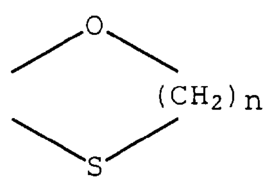 oder 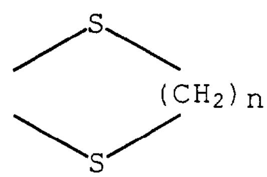

für den Oxorest darstellt, worin n gleich 2 oder 3 ist und die Wellenlinien irgendeine der isomeren Formen oder ihre Mischungen symbolisieren, dadurch gekennzeichnet, daß man die Verbindung der Formel (II)

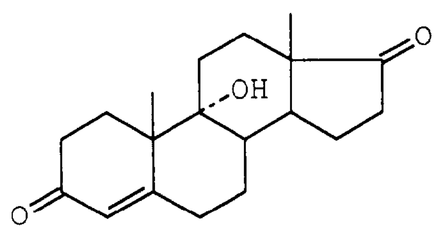

(II)

mit Essigsäureanhydrid in Anwesenheit einer starken Säure und anschließend mit einem Mittel zur sauren Hydrolyse behandelt, um die Verbindung der Formel (III)

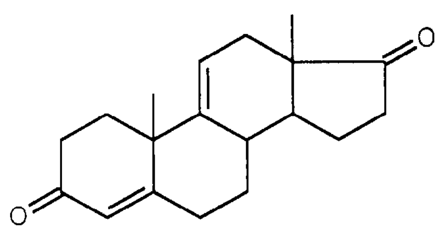

(III)

zu erhalten, bei der man selektiv die 3-Oxo-Funktion durch Einwirkung eines Diols, Thiols oder Dithiols der Formel $HO-(CH_2)_n-OH$, $HO-(CH_2)_n-SH$ oder $HS-(CH_2)_n-SH$ blockiert, worin n wie vorstehend definiert ist, um eine Verbindung der Formel (IV)

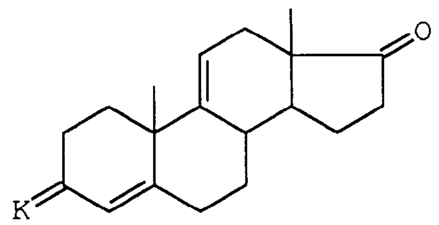

(IV)

zu erhalten, in der K wie oben definiert ist, die man dann mit einer Verbindung der Formel

$$Hal_3\text{-}C\text{-}CO_2R$$

in der Hal und R wie oben definiert sind, in Anwesenheit von Zink und einer Lewis-Säure zur Reaktion bringt, um die erwartete Verbindung der Formel (I) zu erhalten.

2. Verfahren nach Anspruch 1, dadurch gekennzeichnet, daß man als Mittel für die Blockierung der 3-Oxo-Funktion ein Dithiol der Formel $HS\text{-}(CH_2)_n\text{-}SH$ verwendet, in der n gleich 2 oder 3 ist, und dadurch, daß man die Verbindung der Formel (IV) mit der Verbindung der Formel $Cl_3C\text{-}CO_2R$ zur Reaktion bringt, in der R einen Alkylrest mit 1 bis 3 Kohlenstoffatomen darstellt.

3. Verfahren nach Anspruch 1 oder 2, dadurch gekennzeichnet, daß

- die starke Säure, in deren Anwesenheit man das Essigsäureanhydrid mit der Verbindung der Formel (II) zur Reaktion bringt, die para-Toluolsulfonsäure ist,
- das Mittel zur sauren Hydrolyse die Ameisensäure ist,
- das Mittel zur Blockierung der 3-Oxo-Funktion das Ethandithiol ist, verwendet in Anwesenheit einer katalytischen Menge von konzentrierter Chlorwasserstoffsäure oder Bromwasserstoffsäure,
- die Lewis-Säure das Titantetrachlorid ist.

4. Verfahren nach irgendeinem der Ansprüche 1 bis 3, dadurch gekennzeichnet, daß man als Mittel zur Blockierung der Oxo-Funktion das Ethanthiol verwendet und man anschließend die Verbindung der Formel (IV) mit Trichloressigsäure-methylester zur Reaktion bringt.

5. Verwendung der Verbindungen der Formel (I) wie in Anspruch 1 definiert zur Herstellung von Verbindungen der Formel (A)

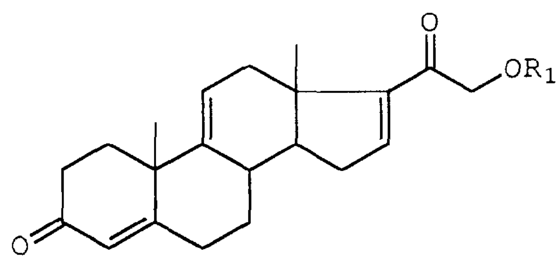

(A)

in der $R_1$ einen Acylrest mit 1 bis 8 Kohlenstoffatomen darstellt, dadurch gekennzeichnet, daß man die genannte Verbindung der Formel (I)

- entweder im basischen Medium mit einem Phenol der Formel

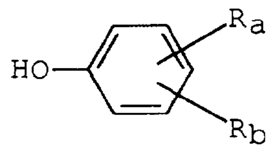

behandelt, in der $R_a$ und $R_b$, gleich oder verschieden, ein Wasserstoffatom, einen Alkylrest mit 1 bis 4 Kohlenstoffatomen, einen Alkoxyrest mit 1 bis 4 Kohlenstoffatomen oder einen Hydroxyrest darstellen, um eine

Verbindung der Formel (V)

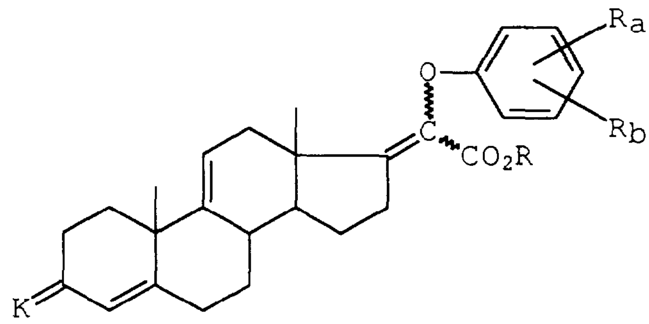

(V)

zu erhalten, in der K und R wie in Anspruch 1 und $R_a$ und $R_b$ wie vorstehend definiert sind, die man dann der Einwirkung eines Reduktionsmittels unterzieht, um eine Verbindung der Formel (VI)

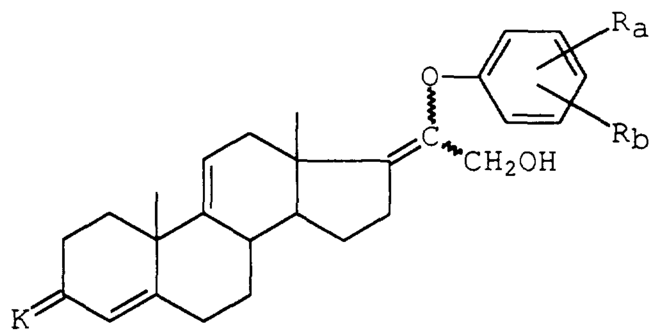

(VI)

zu erhalten, bei der man die Schutzgruppe von der 3-Oxo-Funktion entfernt, und die man anschließend mit einem Epoxidierungsmittel behandelt, um das entsprechende Epoxid in Position 17,20 zu erhalten, das man danach im sauren Medium hydrolysiert, um die Verbindung der Formel (VII)

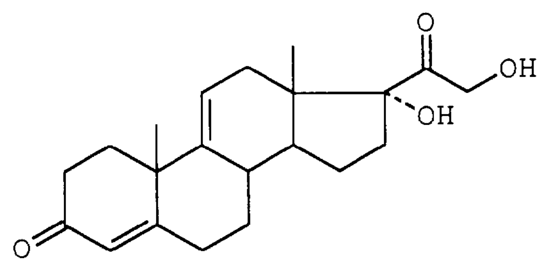

(VII)

zu erhalten, bei der man die Hydroxyfunktionen acyliert, um die Verbindung der Formel (VIII)

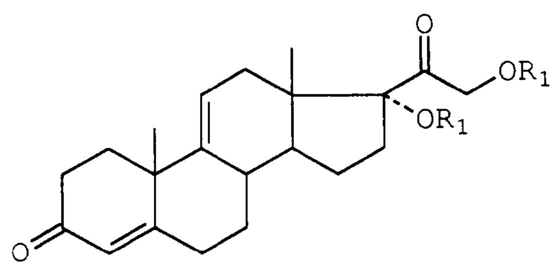

(VIII)

zu erhalten, in der $R_1$ wie oben definiert ist, die man anschließend der Einwirkung eines Mittels zur Eliminierung des Restes 17α-$OR_1$ unterwirft, um die erwartete Verbindung der Formel (A) zu erhalten,

- oder im basischen Medium mit einem Alkalisulfinat der Formel

$$R_2\text{-}SO_2\text{-}Q$$

in der $R_2$ einen Rest Methyl, Phenyl, Tolyl oder Xylyl darstellt und Q ein Alkalimetall bedeutet, in Anwesenheit einer Base behandelt, um nach Verseifung und Decarboxylierung eine Verbindung der Formel (IX)

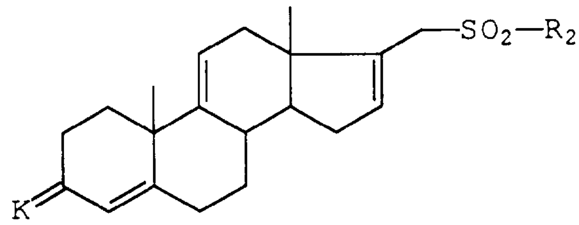

(IX)

zu erhalten, in der K und $R_2$ wie vorstehend definiert sind, die man dann der Einwirkung von Formaldehyd in Anwesenheit einer Base unterzieht, um eine Verbindung der Formel (X)

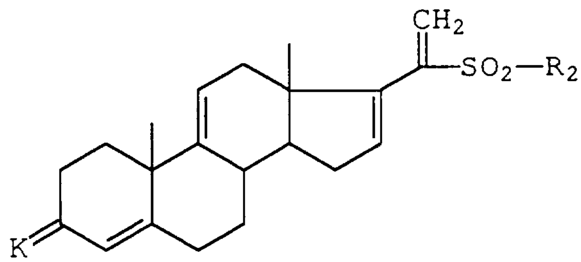

(X)

zu erhalten, die man anschließend der Einwirkung eines Mittels zur Epoxidierung im alkalischen Medium unterwirft, um eine Verbindung der Formel (XI)

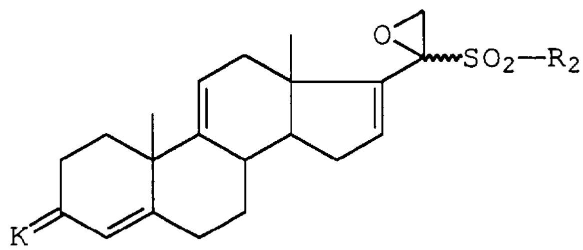

(XI)

zu erhalten, bei der man die Epoxid-Funktion im basischen Medium und in Anwesenheit von Ionen $R_1O^-$, worin $R_1$ wie oben definiert ist, öffnet, um eine Verbindung der Formel (XII)

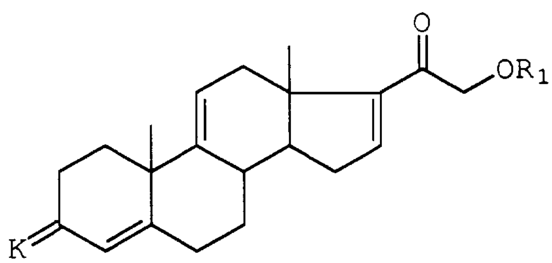

(XII)

zu erhalten, bei der man die Schutzgruppe von der 3-Oxo-Funktion entfernt, um die erwartete Verbindung der Formel (A) oder auch die Verbindung der Formel (XI) zu erhalten, die man dann mit einem Alkalihalogenid behandelt, um die Verbindung der Formel (XV)

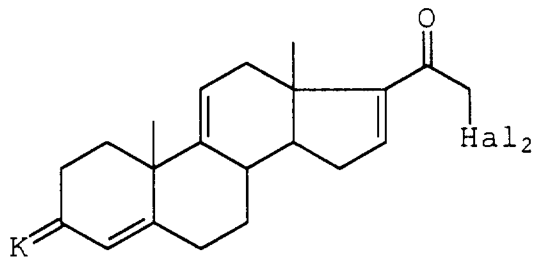

(XV)

zu erhalten, in der $Hal_2$ ein Atom von Chlor, Brom oder Iod darstellt und K wie vorstehend definiert ist, die man danach der Einwirkung eines Mittels zur Acyloxylierung im alkalischen Medium unterwirft, um die wie oben definierte Verbindung der Formel (XII) zu erhalten, und man anschließend die Synthese wie oben beschrieben fortsetzt,

- oder mit einem wie vorstehend definierten Alkalisulfinat der Formel $R_2SO_2Q$ in Anwesenheit einer Base behandelt, um eine Verbindung der Formel (XIII)

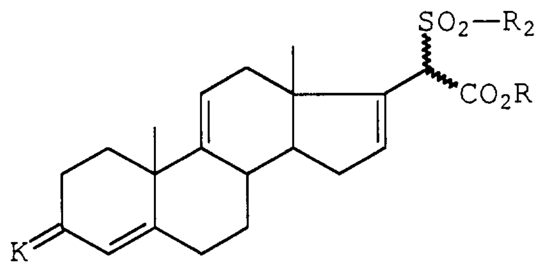

(XIII)

zu erhalten, in der R, $R_2$ und K wie vorstehend definiert sind, die man mit einem Halogenierungsmittel in Anwesenheit einer Base behandelt, und die man anschließend verseift und decarboxyliert, um eine Verbindung der Formel (XIV)

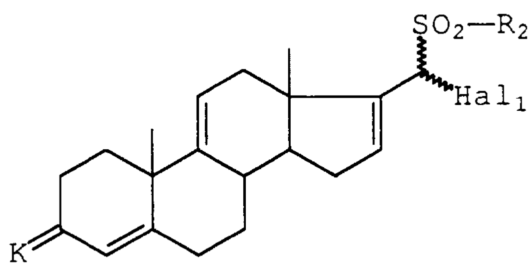

(XIV)

zu erhalten, in der $Hal_1$ ein Halogenatom darstellt, die man danach mit Formaldehyd in Anwesenheit einer Base behandelt, um die wie oben definierte Verbindung der Formel (XI) zu erhalten, die man weiter wie oben beschrieben behandelt, um die gewünschte Verbindung der Formel (A) zu erhalten,

- oder mit einem Reduktionsmittel behandelt, um eine Verbindung der Formel (XVI)

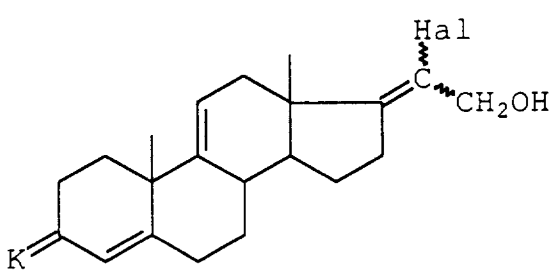

(XVI)

zu erhalten, in der K und Hal wie in Anspruch 1 definiert sind, die man der Einwirkung eines Oxidationsmittels unterzieht, um eine Verbindung der Formel (XVII)

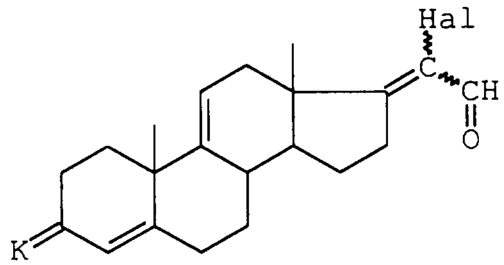

(XVII)

zu erhalten, die man anschließend der Einwirkung eines Mittels zur Acyloxylierung unterwirft, um eine wie oben definierte Verbindung der Formel (XII) zu erhalten, und man anschließend die Synthese wie oben beschrieben fortsetzt, um die erwartete Verbindung der Formel (A) zu erhalten.

**6.** Verwendung nach Anspruch 5, dadurch gekennzeichnet, daß

- $R_a$ und $R_b$ ein Wasserstoffatom, einen Hydroxyrest oder einen Methylrest darstellen,
- das Acylierungsmittel Essigsäureanhydrid oder Acetylchlorid ist,
- das Alkalisulfinat das Natrium-toluolsulfinat ist,
- die Öffnung des Epoxides in Anwesenheit von Acetationen durchgeführt wird,
- das Alkalihalogenid Lithiumbromid ist,
- das Mittel zur Acyloxylierung Kaliumacetat ist,
- das verwendete Halogenierungsmittel das Hypochlorit oder das Hypobromit von Natrium ist.

**Patentansprüche für folgenden Vertragsstaat : GR**

**1.** Verfahren zur Herstellung von Verbindungen der Formel (I)

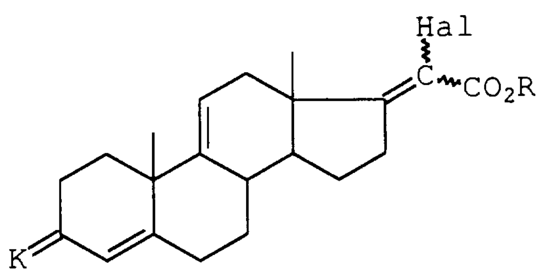

(I)

in der Hal ein Chloratom oder Bromatom darstellt, R einen Alkylrest mit 1 bis 6 Kohlenstoffatomen, einen Aralkylrest mit 7 bis 15 Kohlenstoffatomen oder einen Rest Trialkylsilyl, Triphenylsilyl oder Diphenyl-tert.-butylsilyl bedeutet und K eine Schutzgruppe der Formel

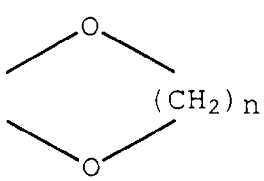 , 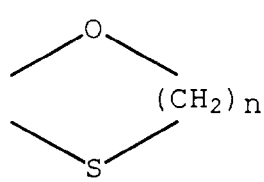 oder 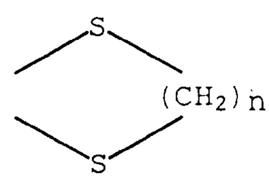

für den Oxorest darstellt, worin n gleich 2 oder 3 ist und die Wellenlinien irgendeine der isomeren Formen oder ihre Mischungen symbolisieren, dadurch gekennzeichnet, daß man die Verbindung der Formel (II)

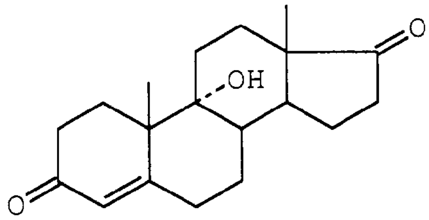

(II)

mit Essigsäureanhydrid in Anwesenheit einer starken Säure und anschließend mit einem Mittel zur sauren Hydrolyse behandelt, um die Verbindung der Formel (III)

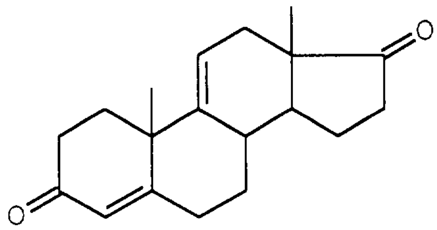

(III)

zu erhalten, bei der man selektiv die 3-Oxo-Funktion durch Einwirkung eines Diols, Thiols oder Dithiols der Formel HO-$(CH_2)_n$-OH, HO-$(CH_2)_n$-SH oder HS-$(CH_2)_n$-SH blockiert, worin n wie vorstehend definiert ist, um eine Verbindung der Formel (IV)

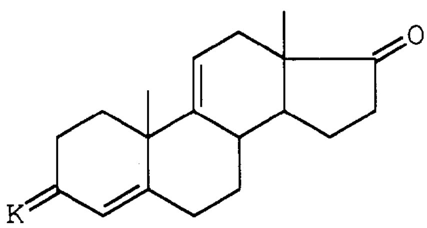

(IV)

zu erhalten, in der K wie oben definiert ist, die man dann mit einer Verbindung der Formel

$$Hal_3\text{-}C\text{-}CO_2R$$

in der Hal und R wie oben definiert sind, in Anwesenheit von Zink und einer Lewis-Säure zur Reaktion bringt, um die erwartete Verbindung der Formel (I) zu erhalten.

**2.** Verfahren nach Anspruch 1, dadurch gekennzeichnet, daß man als Mittel für die Blockierung der 3-Oxo-Funktion ein Dithiol der Formel HS-$(CH_2)_n$-SH verwendet, in der n gleich 2 oder 3 ist, und dadurch, daß man die Verbindung der Formel (IV) mit der Verbindung der Formel $Cl_3C$-$CO_2R$ zur Reaktion bringt, in der R einen Alkylrest mit 1 bis 3 Kohlenstoffatomen darstellt.

**3.** Verfahren nach Anspruch 1 oder 2, dadurch gekennzeichnet, daß

- die starke Säure, in deren Anwesenheit man das Essigsäureanhydrid mit der Verbindung der Formel (II) zur Reaktion bringt, die para-Toluolsulfonsäure ist,
- das Mittel zur sauren Hydrolyse die Ameisensäure ist,
- das Mittel zur Blockierung der 3-Oxo-Funktion das Ethandithiol ist, verwendet in Anwesenheit einer katalytischen Menge von konzentrierter Chlorwasserstoffsäure oder Bromwasserstoffsäure,
- die Lewis-Säure das Titantetrachlorid ist.

**4.** Verfahren nach irgendeinem der Ansprüche 1 bis 3, dadurch gekennzeichnet, daß man als Mittel zur Blockierung der Oxo-Funktion das Ethanthiol verwendet und man anschließend die Verbindung der Formel (IV) mit Trichloressigsäure-methylester zur Reaktion bringt.

**5.** Verwendung der Verbindungen der Formel (I) wie in Anspruch 1 definiert zur Herstellung von Verbindungen der Formel (A)

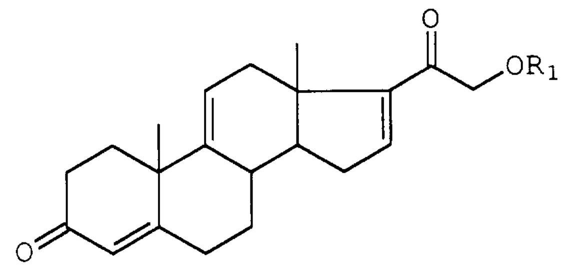

(A)

in der $R_1$ einen Acylrest mit 1 bis 8 Kohlenstoffatomen darstellt, dadurch gekennzeichnet, daß man die genannte Verbindung der Formel (I)

- entweder im basischen Medium mit einem Phenol der Formel

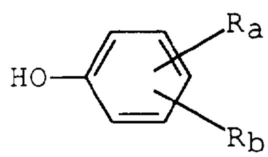

behandelt, in der $R_a$ und $R_b$, gleich oder verschieden, ein Wasserstoffatom, einen Alkylrest mit 1 bis 4 Kohlenstoffatomen, einen Alkoxyrest mit 1 bis 4 Kohlenstoffatomen oder einen Hydroxyrest darstellen, um eine Verbindung der Formel (V)

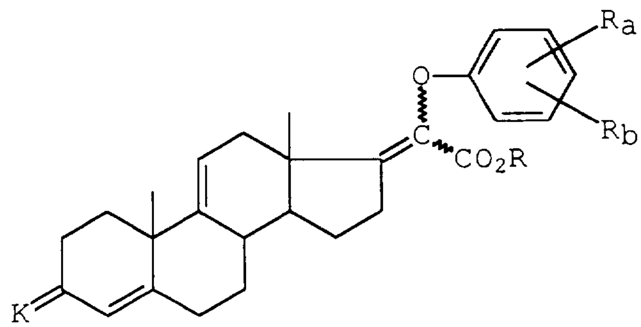

(V)

zu erhalten, in der K und R wie in Anspruch 1 und $R_a$ und $R_b$ wie vorstehend definiert sind, die man dann der Einwirkung eines Reduktionsmittels unterzieht, um eine Verbindung der Formel (VI)

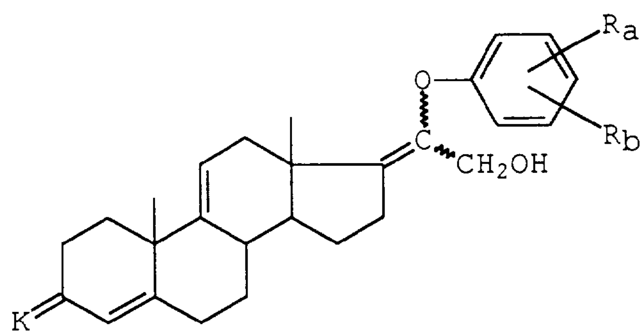

(VI)

zu erhalten, bei der man die Schutzgruppe von der 3-Oxo-Funktion entfernt, und die man anschließend mit einem Epoxidierungsmittel behandelt, um das entsprechende Epoxid in Position 17,20 zu erhalten, das man danach im sauren Medium hydrolysiert, um die Verbindung der Formel (VII)

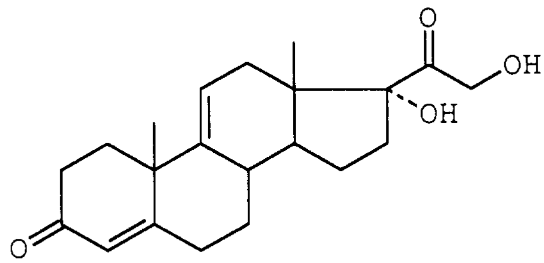

(VII)

zu erhalten, bei der man die Hydroxyfunktionen acyliert, um die Verbindung der Formel (VIII)

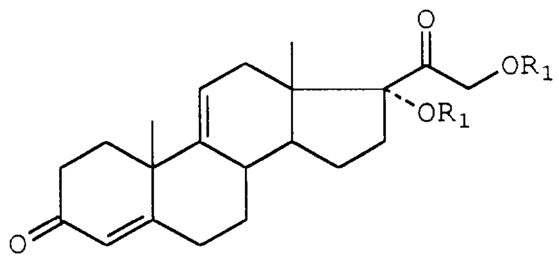

(VIII)

zu erhalten, in der $R_1$ wie oben definiert ist, die man anschließend der Einwirkung eines Mittels zur Eliminierung des Restes 17α-$OR_1$ unterwirft, um die erwartete Verbindung der Formel (A) zu erhalten,

- oder im basischen Medium mit einem Alkalisulfinat der Formel

$$R_2\text{-}SO_2\text{-}Q$$

in der $R_2$ einen Rest Methyl, Phenyl, Tolyl oder Xylyl darstellt und Q ein Alkalimetall bedeutet, in Anwesenheit einer Base behandelt, um nach Verseifung und Decarboxylierung eine Verbindung der Formel (IX)

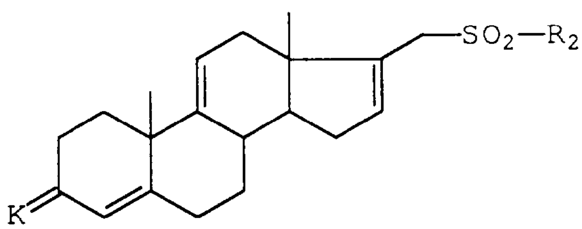

(IX)

zu erhalten, in der K und $R_2$ wie vorstehend definiert sind, die man dann der Einwirkung von Formaldehyd in Anwesenheit einer Base unterzieht, um eine Verbindung der Formel (X)

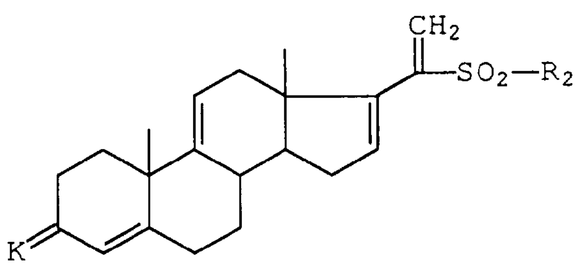

(X)

zu erhalten, die man anschließend der Einwirkung eines Mittels zur Epoxidierung im alkalischen Medium unterwirft, um eine Verbindung der Formel (XI)

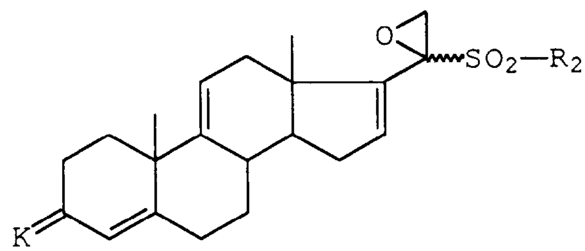

(XI)

zu erhalten, bei der man die Epoxid-Funktion im basischen Medium und in Anwesenheit von Ionen $R_1O^-$, worin $R_1$ wie oben definiert ist, öffnet, um eine Verbindung der Formel (XII)

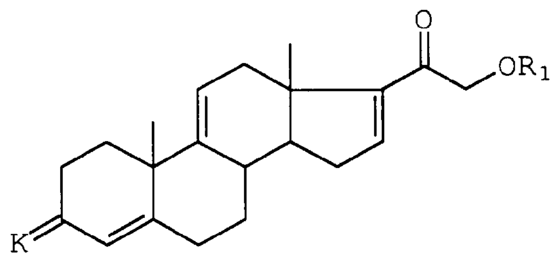

(XII)

zu erhalten, bei der man die Schutzgruppe von der 3-Oxo-Funktion entfernt, um die erwartete Verbindung der Formel (A) oder auch die Verbindung der Formel (XI) zu erhalten, die man dann mit einem Alkalihalogenid behandelt, um die Verbindung der Formel (XV)

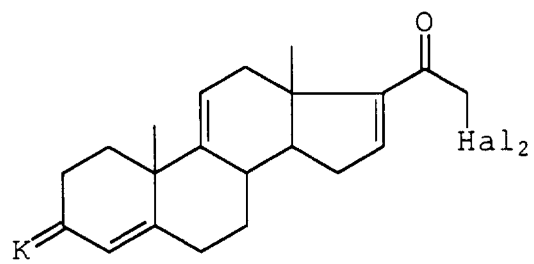

(XV)

zu erhalten, in der $Hal_2$ ein Atom von Chlor, Brom oder Iod darstellt und K wie vorstehend definiert ist, die man danach der Einwirkung eines Mittels zur Acyloxylierung im alkalischen Medium unterwirft, um die wie oben definierte Verbindung der Formel (XII) zu erhalten, und man anschließend die Synthese wie oben beschrieben fortsetzt,

- oder mit einem wie vorstehend definierten Alkalisulfinat der Formel $R_2SO_2Q$ in Anwesenheit einer Base behandelt, um eine Verbindung der Formel (XIII)

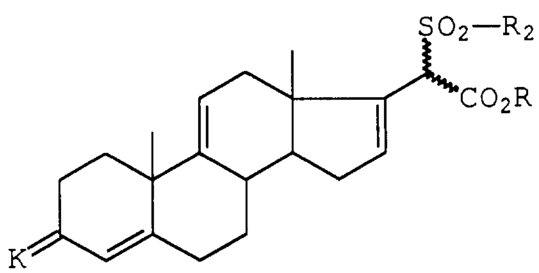

(XIII)

zu erhalten, in der R, $R_2$ und K wie vorstehend definiert sind, die man mit einem Halogenierungsmittel in Anwesenheit einer Base behandelt, und die man anschließend verseift und decarboxyliert, um eine Verbindung der Formel (XIV)

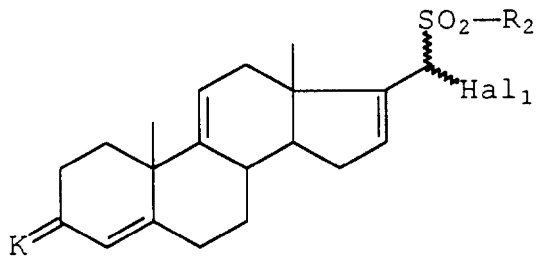

(XIV)

zu erhalten, in der $Hal_1$ ein Halogenatom darstellt, die man danach mit Formaldehyd in Anwesenheit einer Base behandelt, um die wie oben definierte Verbindung der Formel (XI) zu erhalten, die man weiter wie oben beschrieben behandelt, um die gewünschte Verbindung der Formel (A) zu erhalten,

- oder mit einem Reduktionsmittel behandelt, um eine Verbindung der Formel (XVI)

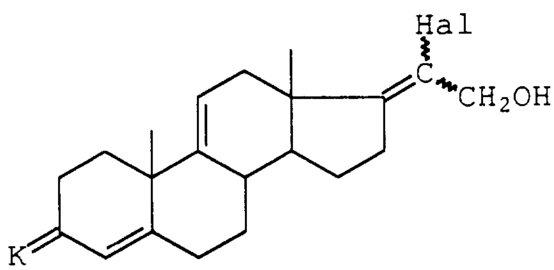

(XVI)

zu erhalten, in der K und Hal wie in Anspruch 1 definiert sind, die man der Einwirkung eines Oxidationsmittels unterzieht, um eine Verbindung der Formel (XVII)

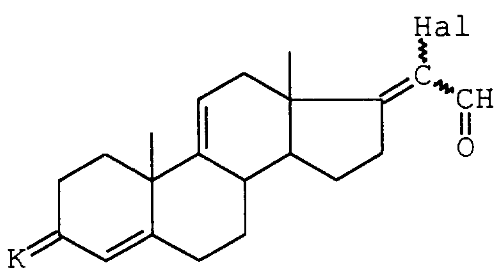

(XVII)

zu erhalten, die man anschließend der Einwirkung eines Mittels zur Acyloxylierung unterwirft, um eine wie oben definierte Verbindung der Formel (XII) zu erhalten, und man anschließend die Synthese wie oben beschrieben fortsetzt, um die erwartete Verbindung der Formel (A) zu erhalten.

**6.** Verwendung nach Anspruch 5, dadurch gekennzeichnet, daß

- $R_a$ und $R_b$ ein Wasserstoffatom, einen Hydroxyrest oder einen Methylrest darstellen,
- das Acylierungsmittel Essigsäureanhydrid oder Acetylchlorid ist,
- das Alkalisulfinat das Natrium-toluolsulfinat ist,
- die Öffnung des Epoxides in Anwesenheit von Acetationen durchgeführt wird,
- das Alkalihalogenid Lithiumbromid ist,
- das Mittel zur Acyloxylierung Kaliumacetat ist,
- das verwendete Halogenierungsmittel das Hypochlorit oder das Hypobromit von Natrium ist.

**7.** Als industrielle Produkte

- die Verbindungen der Formel (F)

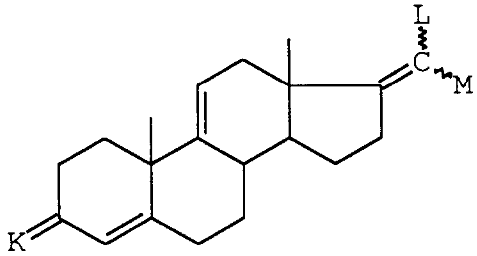

(F)

in der K wie in Anspruch 1 definiert ist, und
entweder L eine Gruppe

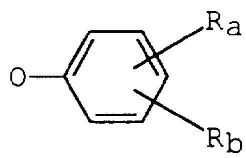

darstellt, worin $R_a$ und $R_b$, gleich oder verschieden, ein Wasserstoffatom, einen Alkylrest mit 1 bis 4 Kohlenstoffatomen, einen Alkoxyrest mit 1 bis 4 Kohlenstoffatomen oder einen Hydroxyrest bedeuten, M einen Rest $CH_2OH$ oder $CO_2R$ darstellt und R einen Alkylrest mit 1 bis 6 Kohlenstoffatomen, einen Aralkylrest mit 7 bis 15 Kohlenstoffatomen oder einen Rest Trialkylsilyl, Triphenylsilyl oder Diphenyl-tert.-butylsilyl bedeutet,
oder L ein Chloratom oder Bromatom darstellt und M einen Rest $CH_2OH$ bedeutet,
und die Wellenlinien irgendeine der isomeren Formen oder ihre Mischungen symbolisieren.

**8.** Als industrielle Produkte die Produkte der Formel (G)

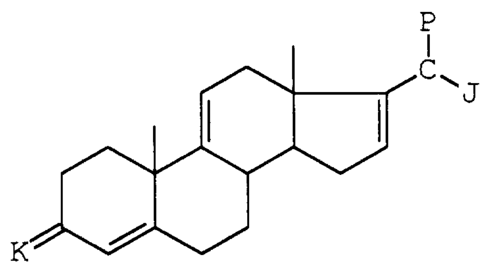

(G)

in der K wie in Anspruch 1 definiert ist, die Gruppe C-P eine Gruppe
$CH_2$, $C{=}CH_2$ oder

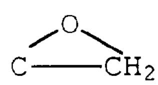

darstellt und die Gruppe C-J eine Gruppe $C\text{-}SO_2R_2$ bedeutet,
oder die Gruppe C-P eine Gruppe

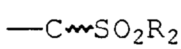

darstellt und die Gruppe C-J eine Gruppe

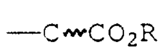

oder C-$Hal_1$ bedeutet,

$R_2$ einen Rest Methyl, Phenyl, Tolyl oder Xylyl darstellt,

R einen Alkylrest mit 1 bis 6 Kohlenstoffatomen, einen Aralkylrest mit 7 bis 15 Kohlenstoffatomen oder einen Rest Trialkylsilyl, Triphenylsilyl oder Diphenyl-tert.-butylsilyl bedeutet, $Hal_1$ ein Halogenatom darstellt, insbesondere ein Chloratom oder Bromatom,
und die Wellenlinien irgendeine der isomeren Formen oder ihre Mischungen symbolisieren.

## Claims

### Claims for the following Contracting States : AT, BE, CH, DE, DK, FR, GB, IT, LI, LU, NL, PT, SE

**1.** The compounds of formula (I):

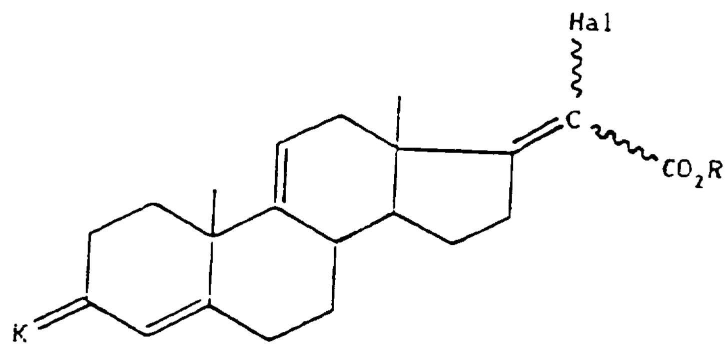

(I)

in which Hal represents a chlorine or bromine atom, R represents an alkyl radical containing 1 to 6 carbon atoms, an aralkyl radical containing 7 to 15 carbon atoms or a trialkylsilyl, triphenylsilyl or diphenyl tert-butyl silyl remainder, K represents a protective group of the oxo radical of formula:

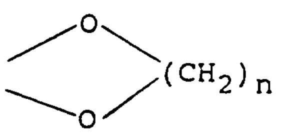 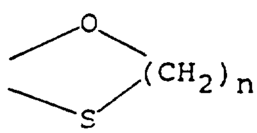 or 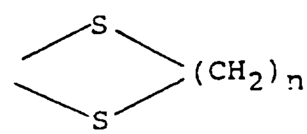

in which n is equal to 2 or 3 and the wavy lines symbolize any one of the isomer forms or their mixtures.

**2.** The compounds of formula (I), as defined in claim 1, in which Hal represents a chlorine atom, R represents an alkyl radical containing 1 to 3 carbon atoms and K represents a group of formula

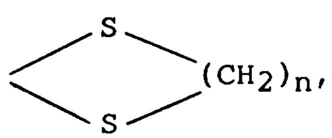,

in which n is equal to 2 or 3.

**3.** A compound according to claim 1, of which the name follows:
methyl 20-chloro-3,3-[(1,2-ethanediyl)-bis(thio)]-pregna-4,9(11),17(20)-trien-21-oate.

**4.** The preparation process for compounds of formula (I) as defined in claim 1, characterized in that the compound of formula (II):

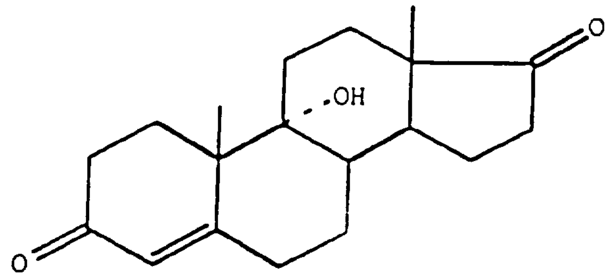

(II)

is treated with acetic anhydride in the presence of a strong acid then with an acid hydrolysis agent, in order to obtain the compound of formula (III):

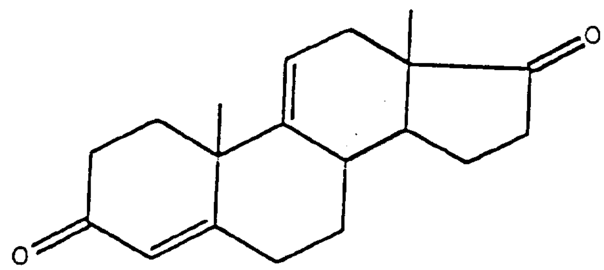

(III)

the 3-oxo function of which is selectively blocked by the action of a diol, a thiol or a dithiol of formula HO-$(CH_2)_n$-OH, HO-$(CH_2)_n$-SH or HS-$(CH_2)_n$-SH in which n is defined as in claim 1 in order to obtain a compound of formula (IV):

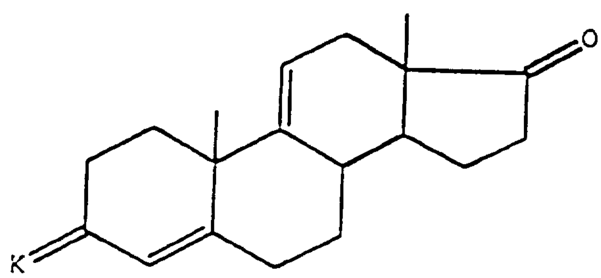

(IV)

in which K is defined as in claim 1, on which a compound of formula:

$$Hal_3C\text{-}CO_2R$$

in which Hal and R are defined as in claim 1, is reacted in the presence of zinc and a Lewis acid in order to obtain the expected compound of formula (I).

**5.** Process according to claim 4, characterized in that:

- the strong acid in the presence of which the acetic anhydride is reacted on the compound of formula (II) is paratoluene sulphonic acid,
- the acid hydrolysis agent is formic acid,
- the blocking agent of the 3-oxo function is ethane dithiol, used in the presence of a catalytic quantity of concentrated hydrochloric or hydrobromic acid,
- the Lewis acid is titanium tetrachloride.

6. Use of the compounds of formula (I) as defined in claim 1, for the preparation of the compounds of formula (A):

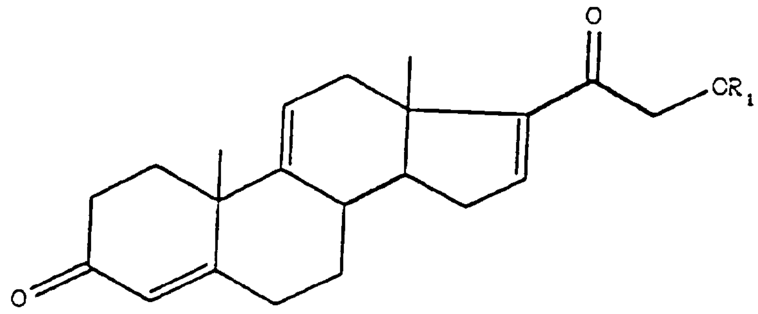

(A)

in which $R_1$ represents an acyl radical containing 1 to 8 carbon atoms, characterized in that a compound of formula (I) is treated, in a basic medium, by a phenol of formula:

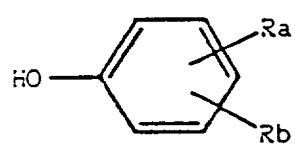

in which $R_a$ and $R_b$, identical or different, represent a hydrogen atom, an alkyl radical containing 1 to 4 carbon atoms, an alkoxy radical containing 1 to 4 carbon atoms, or a hydroxy radical, in order to obtain a compound of formula (V):

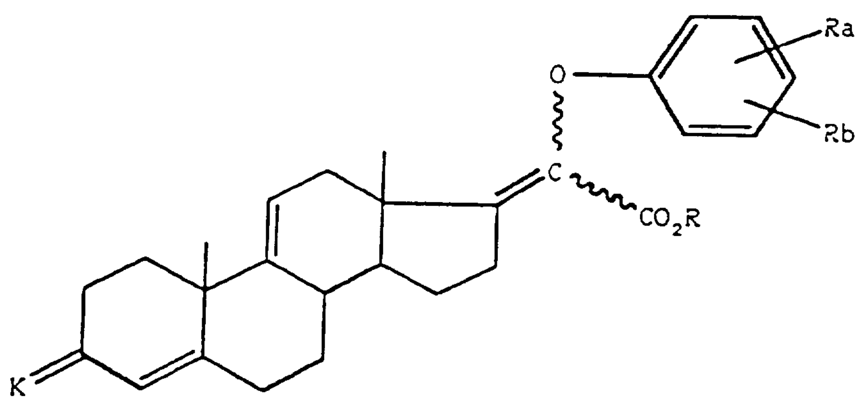

(V)

in which K and R are defined as in claim 1 and $R_a$ and $R_b$ are defined as previously, which is subjected to the action of a reducing agent, in order to obtain a compound of formula (VI):

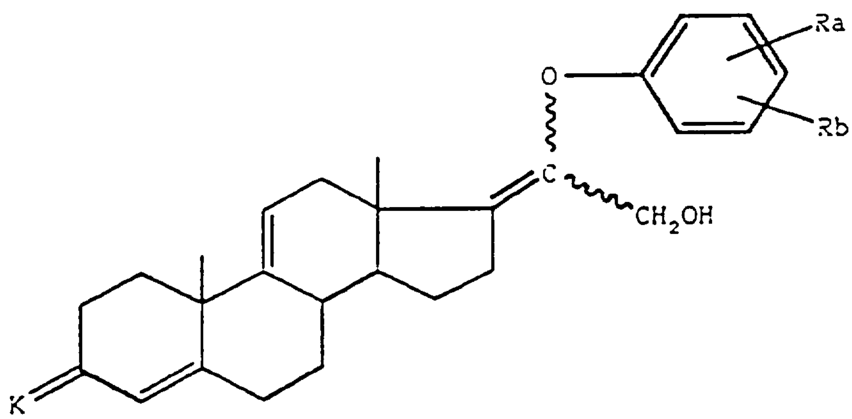

(VI)

the 3-oxo function of which is deprotected, which is then treated by an epoxidation agent, in order to obtain the corresponding epoxide in position 17,20, which is hydrolyzed in an acid medium, in order to obtain the compound of formula (VII):

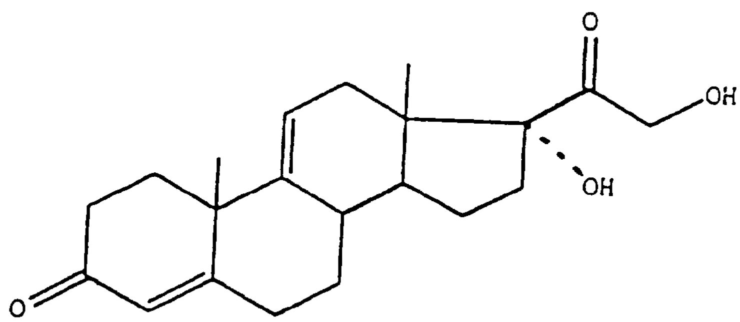

(VII)

the hydroxy functions of which are acylated in order to obtain the compound of formula (VIII):

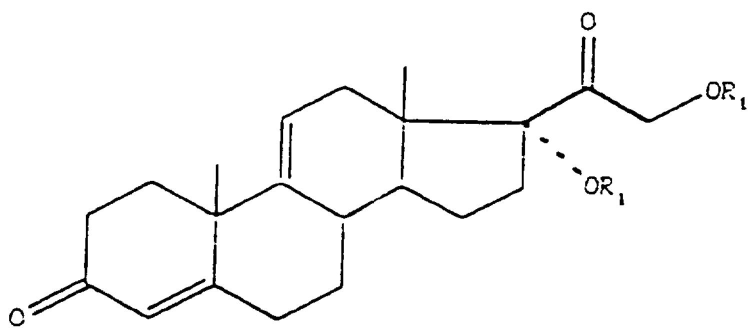

(VIII)

in which $R_1$ is defined as previously, which is subjected to the action of an elimination agent of the 17alpha-$OR_1$ radical, in order to obtain the expected compound of formula (A).

**7.** Use according to claim 6, characterized in that:

- $R_a$ and $R_b$ represent a hydrogen atom, a hydroxy radical or a methyl radical,
- the acylation agent is acetic anhydride or acetyl chloride.

**8.** Use of the compounds of formula (I) as defined in claim 1, for the preparation of the compounds of formula (A), as defined in claim 6, characterized in that a compound of formula (I) is treated, in a basic medium, by an alkaline sulphinate of formula:

$$R_2\text{-}SO_2\text{-}Q$$

in which $R_2$ represents a methyl, phenyl, tolyl or xylyl radical and Q represents an alkali metal, in the presence of a base, in order to obtain, after saponification and decarboxylation, a compound of formula (IX):

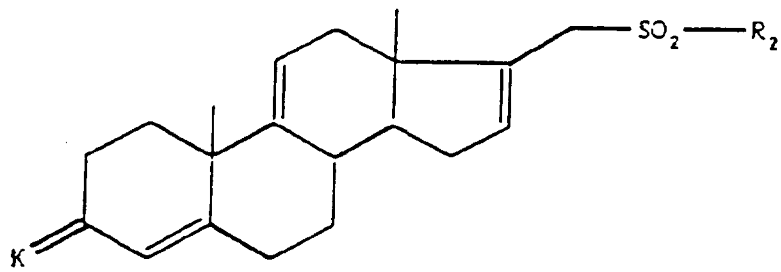

(IX)

in which K and $R_2$ are defined as previously, which is subjected to the action of formaldehyde in the presence of a base, in order to obtain a compound of formula (X):

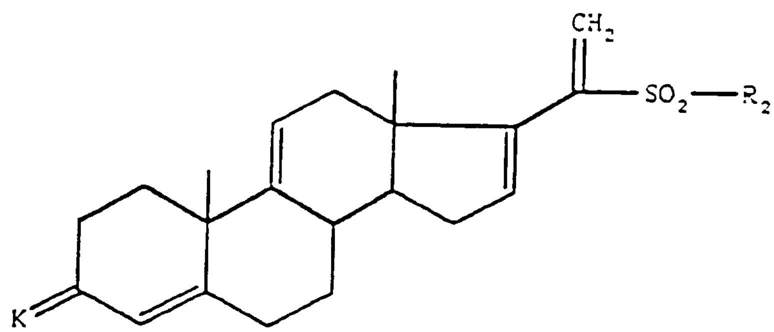

(X)

which is subjected to the action of an epoxidation agent in an alkaline medium, in order to obtain a compound of formula (XI):

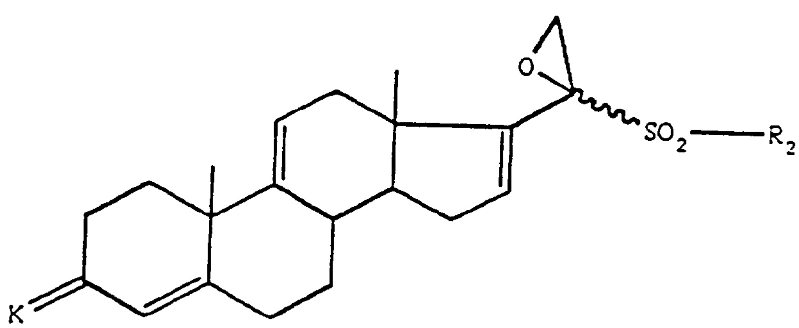

(XI)

the epoxide function of which is opened in a basic medium and in the presence of $R_1O^{\theta}$ ions, in which $R_1$ is defined as in claim 6, in order to obtain a compound of formula (XII):

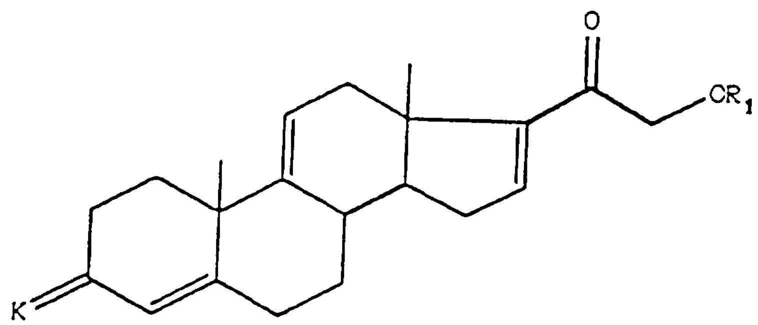

(XII)

the 3-oxo function of which is deprotected, in order to obtain the expected compound of formula (A).

**9.** Use according to claim 8, characterized in that:

- the alkaline sulphinate is sodium toluenesulphinate,
- the opening of the epoxide is carried out in the presence of acetate ions.

**10.** Use of the compounds of formula (I) as defined in claim 1, for the preparation of the compounds of formula (A), as defined in claim 6, characterized in that a compound of formula (I) is treated by an alkaline sulphinate of formula $R_2SO_2Q$, as defined previously, in the presence of a base, in order to obtain a compound of formula (XIII):

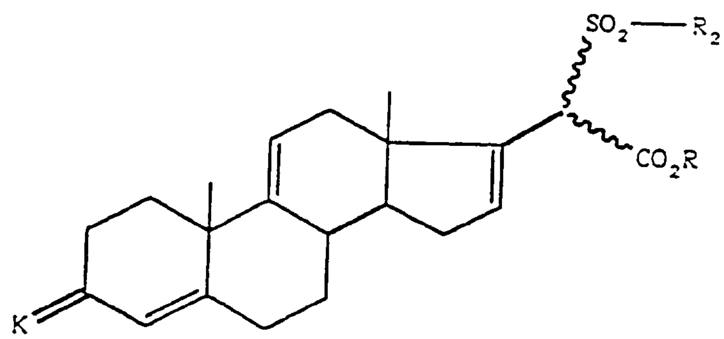

(XIII)

in which R, $R_2$ and K are defined as previously, which is treated with a halogenation agent in the presence of a base, then is saponified and decarboxylated in order to obtain a compound of formula (XIV):

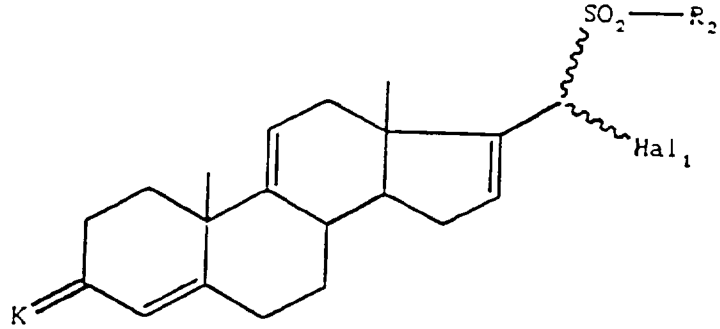

(XIV)

in which $Hal_1$ represents a halogen atom, which is treated with formaldehyde in the presence of a base, in order to obtain the compound of formula (XI) as defined in claim 8, which is treated as described in claim 8, in order to

obtain the desired compound of formula (A).

**11.** Use according to claim 10, characterized in that:

- the alkaline sulphinate used is sodium toluenesulphinate,
- the halogenation agent used is sodium hypochlorite or hypobromite,
- the opening of the epoxide is carried out in the presence of acetate ions.

**12.** Use of the compounds of formula (I) as defined in claim 1, for the production of the compounds of formula (A), characterized in that the compound of formula (XI) obtained from the compounds of formula (I) according to the methods described in claims 8 or 10 is treated with an alkaline halide in order to obtain the compound of formula (XV):

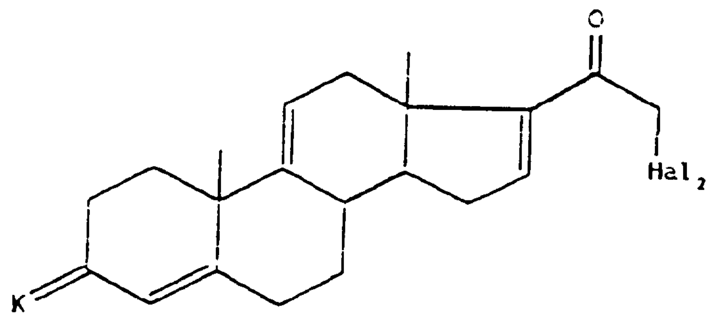

(XV)

in which $Hal_2$ represents a chlorine, bromine or iodine atom and K is defined as previously, which is subjected to the action of an acyloxylation agent in an alkaline medium, in order to obtain the compound of formula (XII) as defined in claim 8, then the synthesis is continued as described in claim 8.

**13.** Use according to claim 12, characterized in that:

- the alkaline halide is lithium bromide,
- the acyloxylation agent is potassium acetate.

**14.** Use of the compounds of formula (I) as defined in claim 1, for the preparation of the compounds of formula (A), as defined in claim 6, characterized in that a compound of formula (I) is treated with a reducing agent, in order to obtain a compound of formula (XVI):

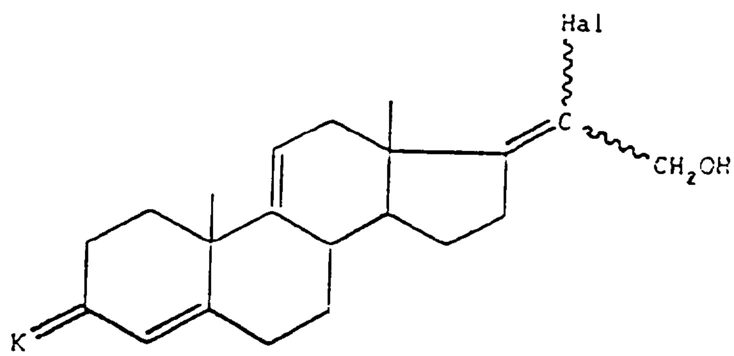

(XVI)

in which K and Hal are defined as in claim 1, which is subjected to the action of an oxidizing agent, in order to obtain a compound of formula (XVII):

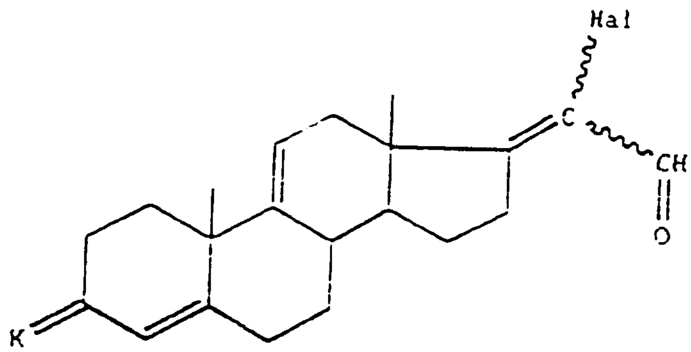

(XVII)

which is subjected to the action of an acyloxylation agent in order to obtain a compound of formula (XII) as defined in claim 8, then the synthesis is continued as described in claim 8.

**15.** As industrial products:

- the compounds of formula (F):

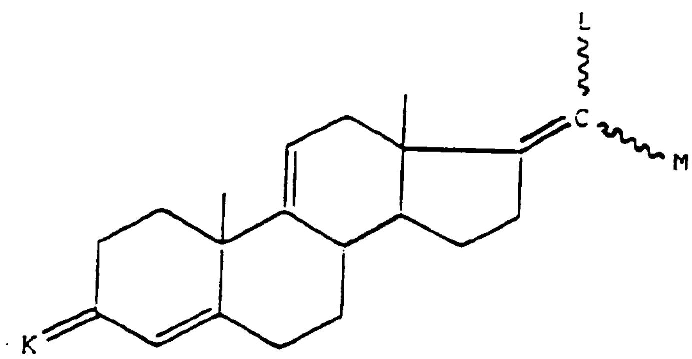

(F)

in which K is defined as in claim 1, and

either L represents a

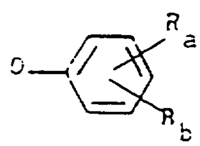

group,
$R_a$ and $R_b$, identical or different, representing a hydrogen atom, an alkyl radical containing 1 to 4 carbon atoms, an alkoxy radical containing 1 to 4 carbon atoms or a hydroxy radical, M represents a $CH_2OH$ or $CO_2R$ radical, R representing an alkyl radical containing 1 to 6 carbon atoms, an aralkyl radical containing 7 to 15 carbon atoms or a trialkylsilyl, triphenylsilyl or diphenyl tert-butyl silyl remainder,
or L represents a chlorine or bromine atom and M represents a $CH_2OH$ radical,
and the wavy lines symbolize any one of the isomer forms or their mixtures.

**16.** As industrial products, the products of formula (G):

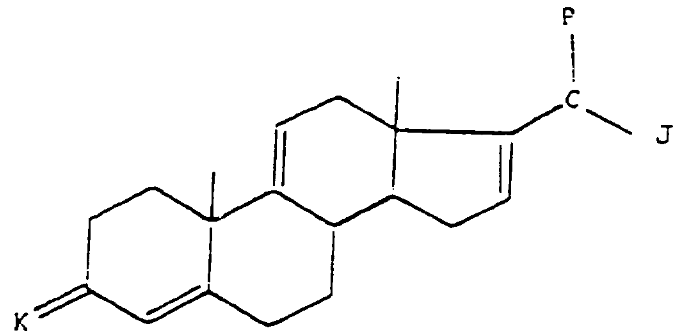

(G)

in which K is defined as in claim 1, the C-P group represents a $CH_2$, $C{=}CH_2$ or

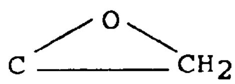

group and the C-J group represents a $C\text{-}SO_2R_2$ group, or the C-P group represents a

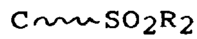

group and the C-J group represents a

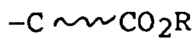

or C-$Hal_1$ group, $R_2$ representing a methyl, phenyl, tolyl or xylyl radical, R representing an alkyl radical containing 1 to 6 carbon atoms, an aralkyl radical containing 7 to 15 carbon atoms or a trialkylsilyl, triphenylsilyl or diphenyl tert-butyl silyl remainder, Hall represents a halogen atom, notably a chlorine or bromine atom, the wavy lines symbolizing any one of the isomer forms or their mixtures.

**Claims for the following Contracting State : ES**

**1.** Process for preparing the compounds of formula (I):

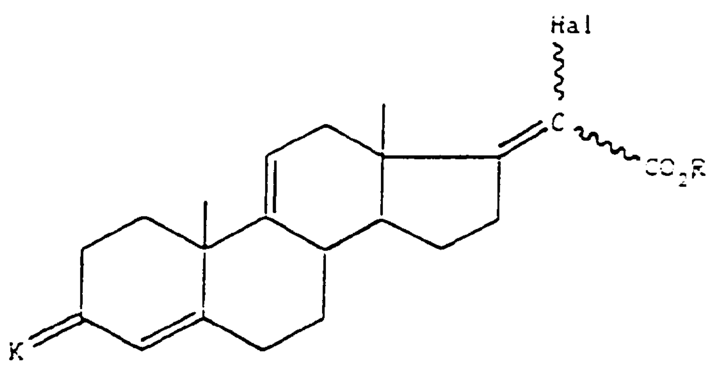

(I)

in which Hal represents a chlorine or bromine atom, R represents an alkyl radical containing 1 to 6 carbon atoms, an aralkyl radical containing 7 to 15 carbon atoms or a trialkylsilyl, triphenylsilyl or diphenyl tert-butyl silyl remainder, K represents a protective group of the oxo radical of formula:

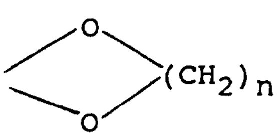 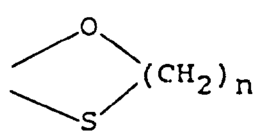 or 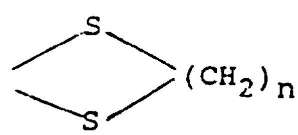

in which n is equal to 2 or 3 and the wavy lines symbolize any one of the isomer forms or their mixtures, characterized

in that the compound of formula (II):

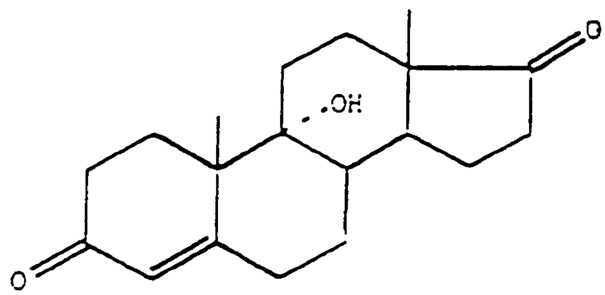

(II)

is treated with acetic anhydride in the presence of a strong acid then with an acid hydrolysis agent, in order to obtain the compound of formula (III):

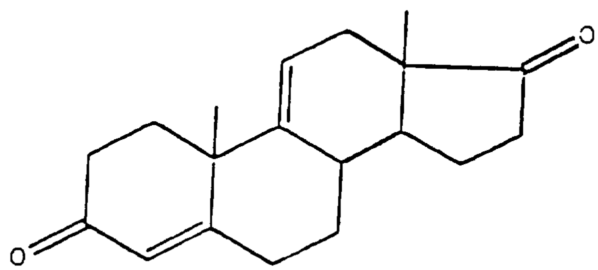

(III)

the 3-oxo function of which is selectively blocked by the action of a diol, a thiol or a dithiol of formula HO-$(CH_2)_n$-OH, HO-$(CH_2)_n$-SH or HS-$(CH_2)_n$-SH in which n is defined as above, in order to obtain a compound of formula (IV):

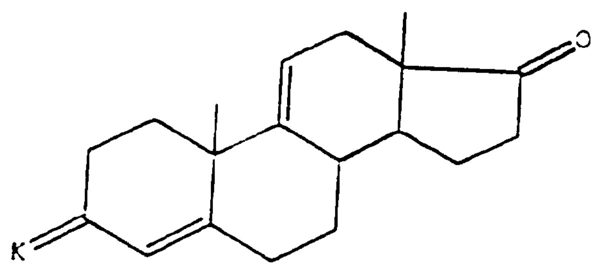

(IV)

in which K is defined as above, on which a compound of formula:

$$Hal_3C\text{-}CO_2R$$

in which Hal and R are defined as above, is reacted in the presence of zinc and a Lewis acid in order to obtain the expected compound of formula (I).

2. Process according to claim 1, characterized in that a dithiol of formula HS-$(CH_2)_n$-SH in which n is equal to 2 or 3 is used as the blocking agent of the 3-oxo function and in that the compound of formula $Cl_3C\text{-}CO_2R$ in which R represents an alkyl radical containing 1 to 3 carbon atoms is reacted on the compound of formula (IV).

3. Process according to claim 1 or 2, characterized in that:

- the strong acid in the presence of which the acetic anhydride is reacted on the compound of formula (II) is

paratoluene sulphonic acid,
- the acid hydrolysis agent is formic acid,
- the blocking agent of the 3-oxo function is ethane dithiol, used in the presence of a catalytic quantity of concentrated hydrochloric or hydrobromic acid,
- the Lewis acid is titanium tetrachloride.

**4.** Process according to any one of claims 1 to 3, characterized in that ethanethiol is used as the blocking agent of the oxo function then methyl trichloroacetate is reacted on the compound of formula (IV).

**5.** Use of the compounds of formula (I) as defined in claim 1, for the preparation of the compounds of formula (A):

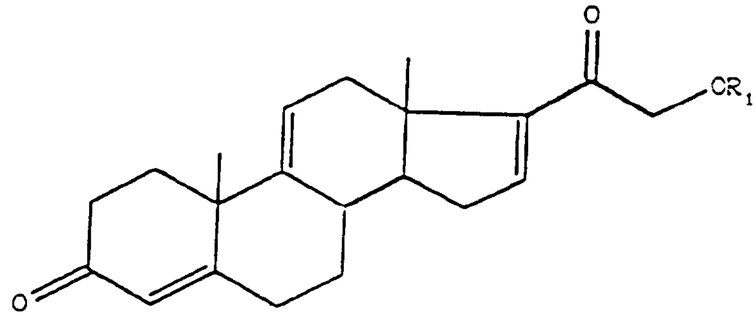

(A)

in which $R_1$ represents an acyl radical containing 1 to 8 carbon atoms, characterized in that said compound of formula (I) is treated

- either in a basic medium, by a phenol of formula:

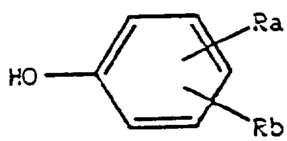

in which $R_a$ and $R_b$, identical or different, represent a hydrogen atom, an alkyl radical containing 1 to 4 carbon atoms, an alkoxy radical containing 1 to 4 carbon atoms, or a hydroxy radical, in order to obtain a compound of formula (V):

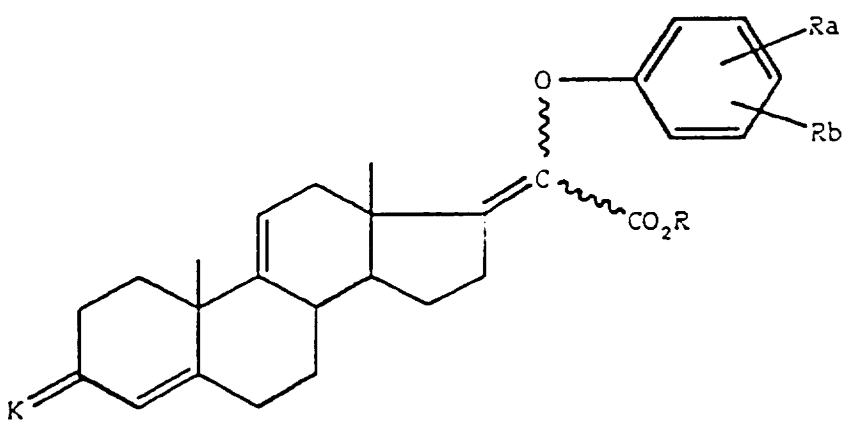

(V)

in which K and R are defined as in claim 1 and $R_a$ and $R_b$ are defined as previously, which is subjected to the action of a reducing agent, in order to obtain a compound of formula (VI):

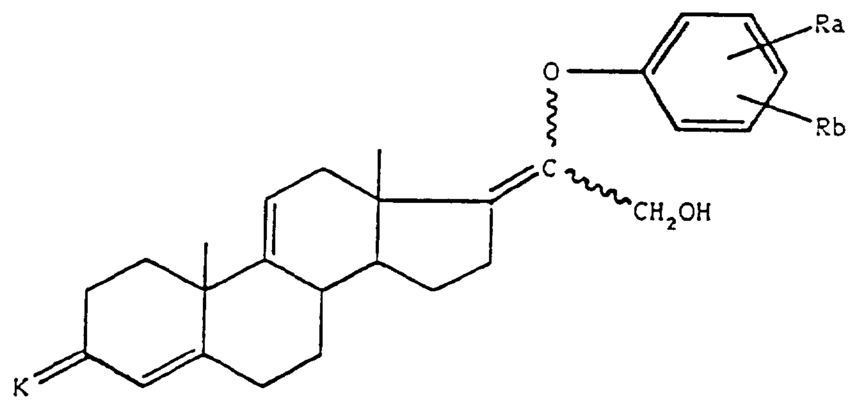

(VI)

the 3-oxo function of which is deprotected, which is then treated by an epoxidation agent, in order to obtain the corresponding epoxide in position 17,20, which is hydrolyzed in an acid medium, in order to obtain the compound of formula (VII):

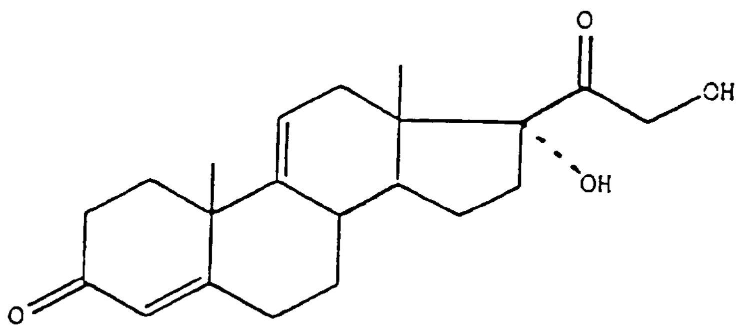

(VII)

the hydroxy functions of which are acylated in order to obtain the compound of formula (VIII):

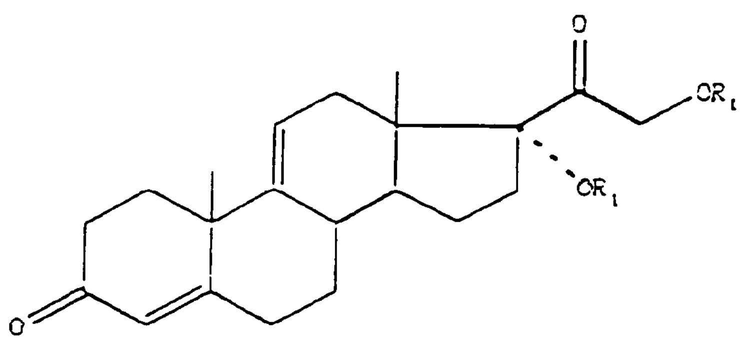

(VIII)

in which $R_1$ is defined as previously, which is subjected to the action of an elimination agent of the 17alpha-$OR_1$ radical, in order to obtain the expected compound of formula (A),

- or in a basic medium, by an alkaline sulphinate of formula:

$$R_2\text{-}SO_2\text{-}Q$$

in which $R_2$ represents a methyl, phenyl, tolyl or xylyl radical and Q represents an alkali metal, in the presence of a base, in order to obtain, after saponification and decarboxylation, a compound of formula (IX):

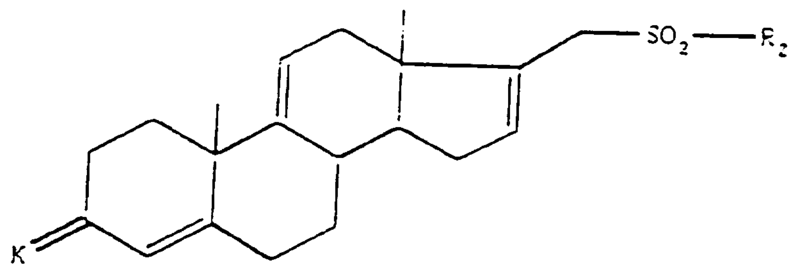

(IX)

in which K and $R_2$ are defined as previously, which is subjected to the action of formaldehyde in the presence of a base, in order to obtain a compound of formula (X):

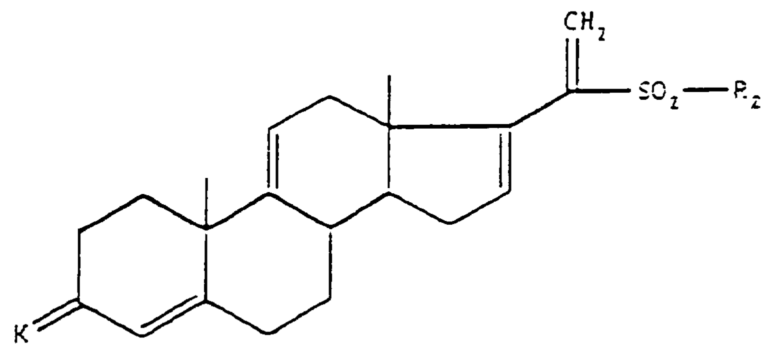

(X)

which is subjected to the action of an epoxidation agent in an alkaline medium, in order to obtain a compound of formula (XI):

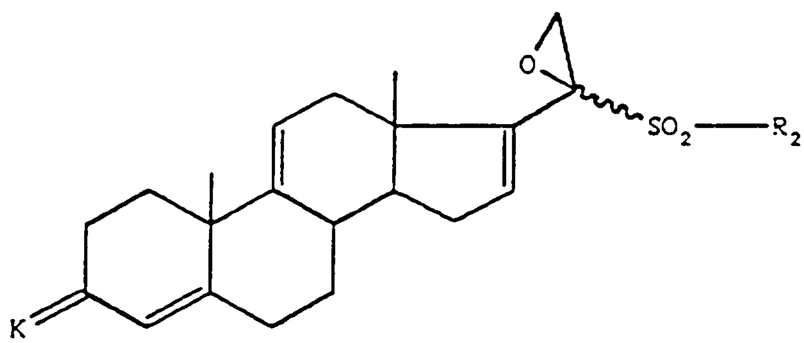

(XI)

the epoxide function of which is opened in a basic medium and in the presence of $R_1O^{\theta}$ ions, in which R1 is defined as above in order to obtain a compound of formula (XII):

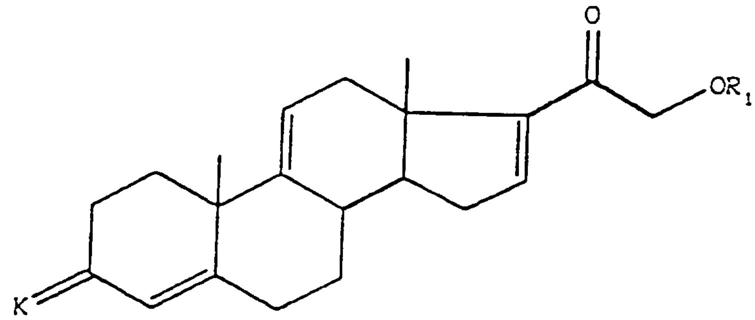

(XII)

the 3-oxo function of which is deprotected, in order to obtain the expected compound of formula (A) or a compound of formula (XI) which is treated with an alkaline halide in order to obtain the compound of formula (XV):

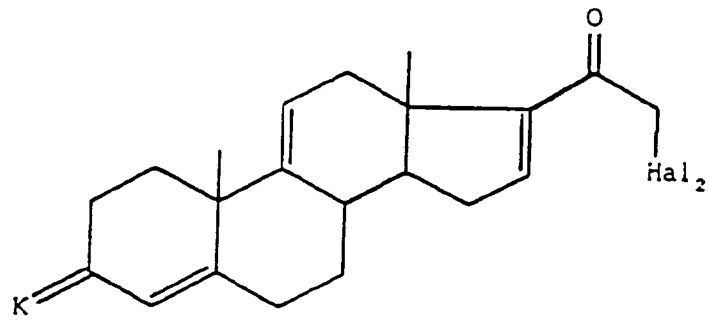

(XV)

in which $Hal_2$ represents a chlorine, bromine or iodine atom and K is defined as previously, which is subjected to the action of an acyloxylation agent in an alkaline medium, in order to obtain the compound of formula (XII) as defined above, then the synthesis is continued as described above,

- or by an alkaline sulphinate of formula $R_2SO_2Q$, as defined previously, in the presence of a base, in order to obtain a compound of formula (XIII):

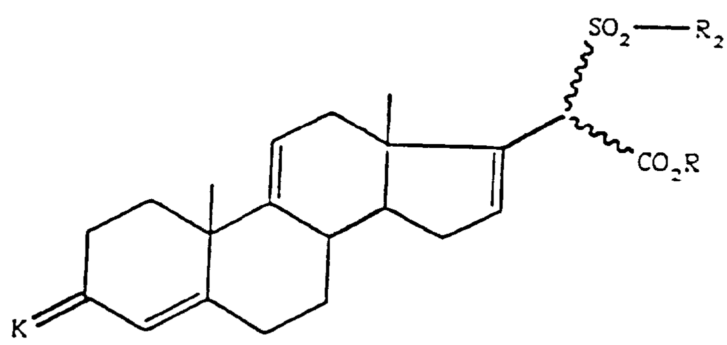

(XIII)

in which R, $R_2$ and K are defined as previously, which is treated with a halogenation agent in the presence of a base, then is saponified and decarboxylated in order to obtain a compound of formula (XIV):

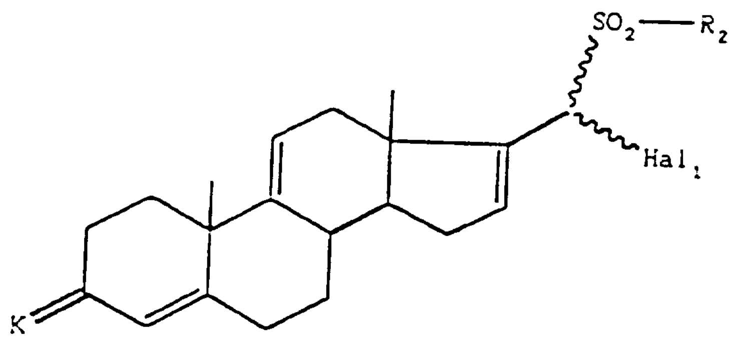

(XIV)

in which $Hal_1$ represents a halogen atom, which is treated with formaldehyde in the presence of a base, in order to obtain the compound of formula (XI) as defined above, which is treated as described above, in order to obtain the desired compound of formula (A),

- or by a reducing agent, in order to obtain a compound of formula (XVI):

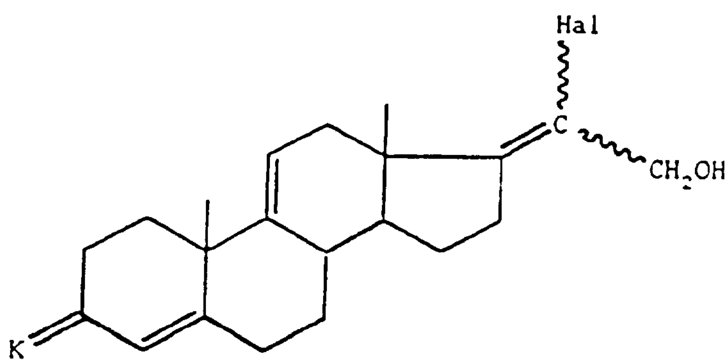

(XVI)

in which K and Hal are defined as in claim 1, which is subjected to the action of an oxidizing agent, in order to obtain a compound of formula (XVII):

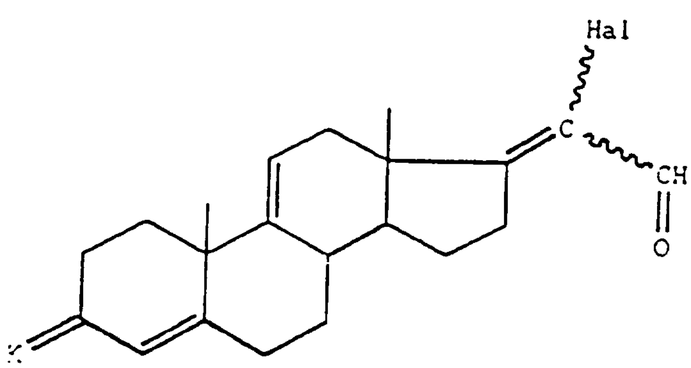

(XVII)

which is subjected to the action of an acyloxylation agent in order to obtain a compound of formula (XII) as defined above, then the synthesis is continued as described above in order to obtain the expected compound of formula (A).

**6.** Use according to claim 5, characterized in that:

- $R_a$ and $R_b$ represent a hydrogen atom, a hydroxy radical or a methyl radical,
- the acylation agent is acetic anhydride or acetyl chloride,
- the alkaline sulphinate is sodium toluenesulphinate,

- the opening of the epoxide is carried out in the presence of acetate ions,
- the alkaline halide is lithium bromide,
- the acyloxylation agent is potassium acetate,
- the halogenation agent used is sodium hypochlorite or hypobromite.

**Claims for the following Contracting State : GR**

**1.** Process for preparing the compounds of formula (I):

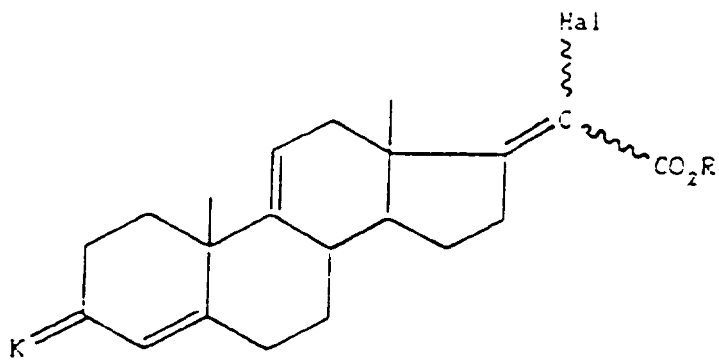

(I)

in which Hal represents a chlorine or bromine atom, R represents an alkyl radical containing 1 to 6 carbon atoms, an aralkyl radical containing 7 to 15 carbon atoms or a trialkylsilyl, triphenylsilyl or diphenyl tert-butyl silyl remainder, K represents a protective group of the oxo radical of formula:

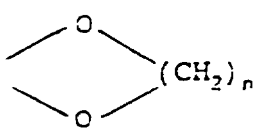 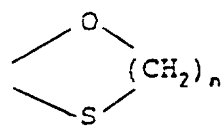 or 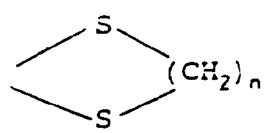

in which n is equal to 2 or 3 and the wavy lines symbolize any one of the isomer forms or their mixtures, characterized in that the compound of formula (II):

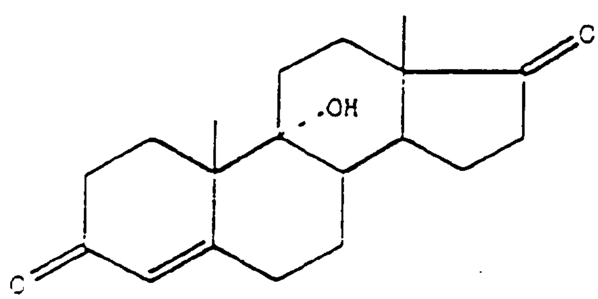

(II)

is treated with acetic anhydride in the presence of a strong acid then with an acid hydrolysis agent, in order to obtain the compound of formula (III):

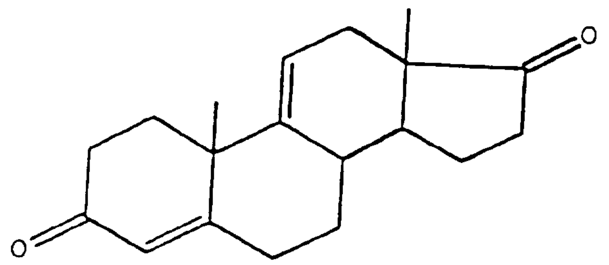

(III)

the 3-oxo function of which is selectively blocked by the action of a diol, a thiol or a dithiol of formula $HO-(CH_2)_n-OH$, $HO-(CH_2)_n-SH$ or $HS-(CH_2)_n-SH$ in which n is defined as above, in order to obtain a compound of formula (IV):

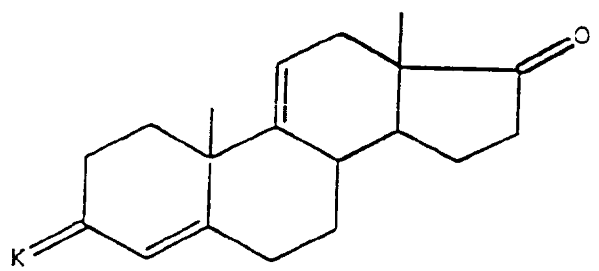

(IV)

in which K is defined as above, on which a compound of formula:

$$Hal_3C-CO_2R$$

in which Hal and R are defined as above, is reacted in the presence of zinc and a Lewis acid in order to obtain the expected compound of formula (I).

**2.** Process according to claim 1, characterized in that a dithiol of formula $HS-(CH_2)_n-SH$ in which n is equal to 2 or 3 is used as the blocking agent of the 3-oxo function and in that the compound of formula $Cl_3C-CO_2R$ in which R represents an alkyl radical containing 1 to 3 carbon atoms is reacted on the compound of formula (IV).

**3.** Process according to claim 1 or 2, characterized in that:

- the strong acid in the presence of which the acetic anhydride is reacted on the compound of formula (II) is paratoluene sulphonic acid,
- the acid hydrolysis agent is formic acid,
- the blocking agent of the 3-oxo function is ethane dithiol, used in the presence of a catalytic quantity of concentrated hydrochloric or hydrobromic acid,
- the Lewis acid is titanium tetrachloride.

**4.** Process according to any one of claims 1 to 3, characterized in that ethanethiol is used as the blocking agent of the oxo function then methyl trichloroacetate is reacted on the compound of formula (IV).

**5.** Use of the compounds of formula (I) as defined in claim 1, for the preparation of the compounds of formula (A):

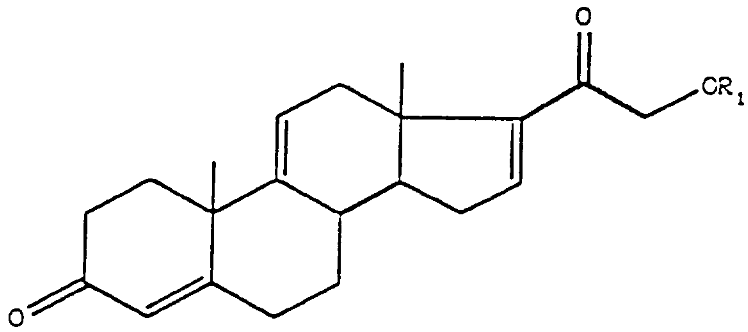

(A)

in which $R_1$ represents an acyl radical containing 1 to 8 carbon atoms, characterized in that said compound of formula (I) is treated

- either in a basic medium, by a phenol of formula:

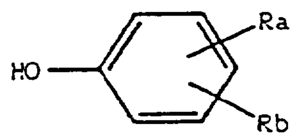

in which $R_a$ and $R_b$, identical or different, represent a hydrogen atom, an alkyl radical containing 1 to 4 carbon atoms, an alkoxy radical containing 1 to 4 carbon atoms, or a hydroxy radical, in order to obtain a compound of formula (v):

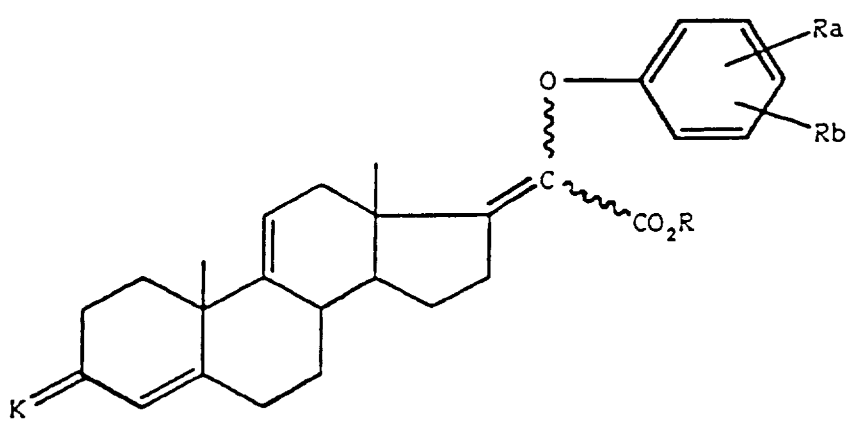

(V)

in which K and R are defined as in claim 1 and $R_a$ and $R_b$ are defined as previously, which is subjected to the action of a reducing agent, in order to obtain a compound of formula (VI):

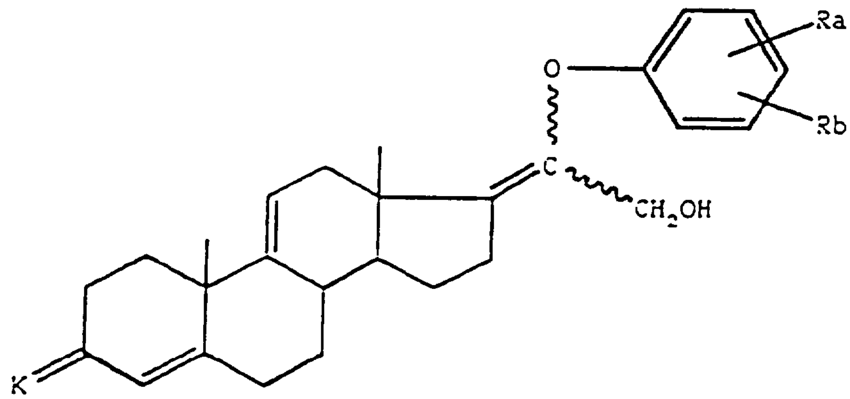

(VI)

the 3-oxo function of which is deprotected, which is then treated by an epoxidation agent, in order to obtain the corresponding epoxide in position 17,20, which is hydrolyzed in an acid medium, in order to obtain the compound of formula (VII):

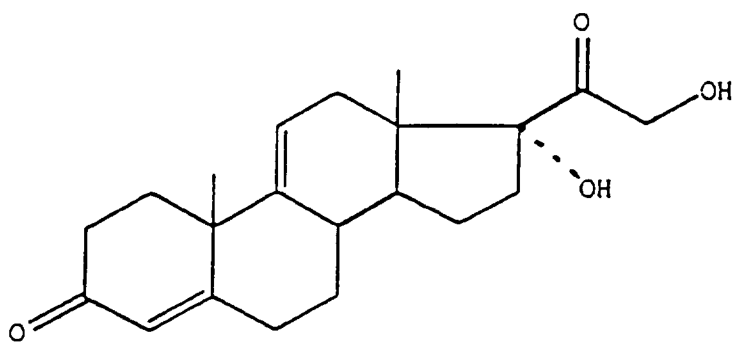

(VII)

the hydroxy functions of which are acylated in order to obtain the compound of formula (VIII):

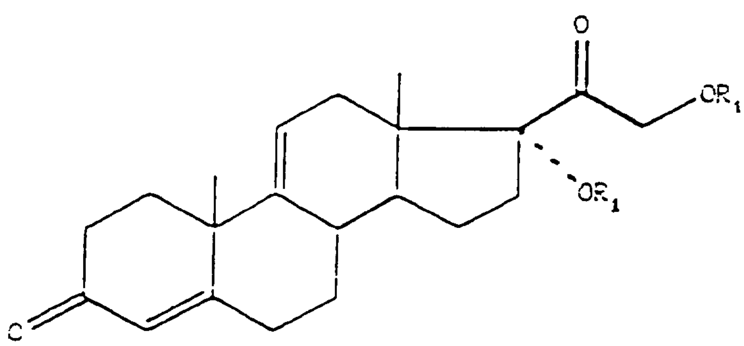

(VIII)

in which $R_1$ is defined as previously, which is subjected to the action of an elimination agent of the 17alpha-$OR_1$ radical, in order to obtain the expected compound of formula (A),

- or in a basic medium, by an alkaline sulphinate of formula:

$$R_2\text{-}SO_2\text{-}Q$$

in which $R_2$ represents a methyl, phenyl, tolyl or xylyl radical and Q represents an alkali metal, in the presence of a base, in order to obtain, after saponification and decarboxylation, a compound of formula (IX):

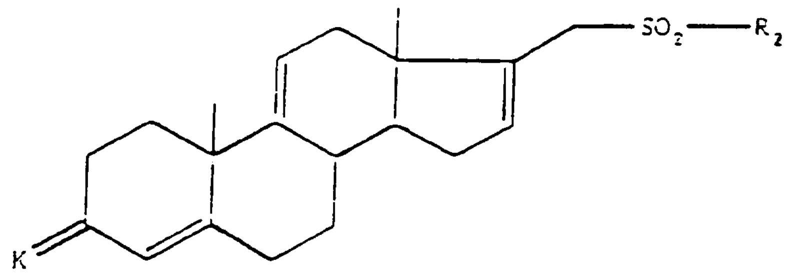

(IX)

in which K and $R_2$ are defined as previously, which is subjected to the action of formaldehyde in the presence of a base, in order to obtain a compound of formula (X):

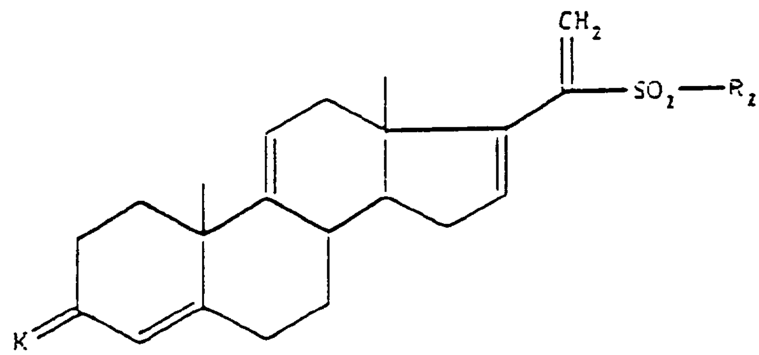

(X)

which is subjected to the action of an epoxidation agent in an alkaline medium, in order to obtain a compound of formula (XI):

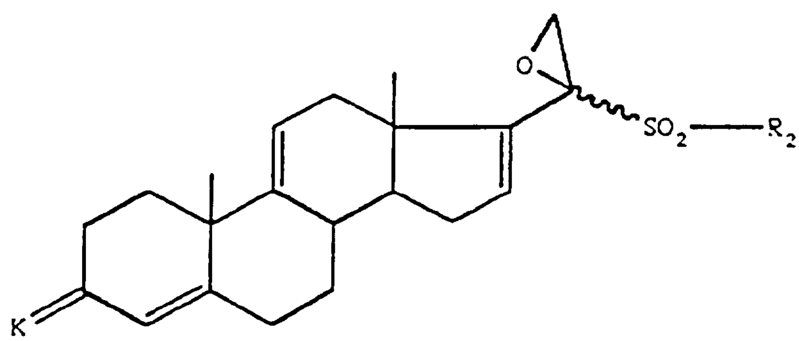

(XI)

the epoxide function of which is opened in a basic medium and in the presence of $R_1O^{\theta}$ ions, in which $R_1$ is defined as above, in order to obtain a compound of formula (XII):

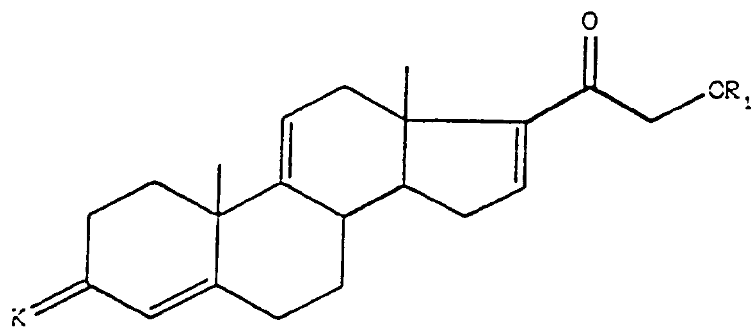

(XII)

the 3-oxo function of which is deprotected, in order to obtain the expected compound of formula (A) or a compound of formula (XI) which is treated with an alkaline halide in order to obtain the compound of formula (XV):

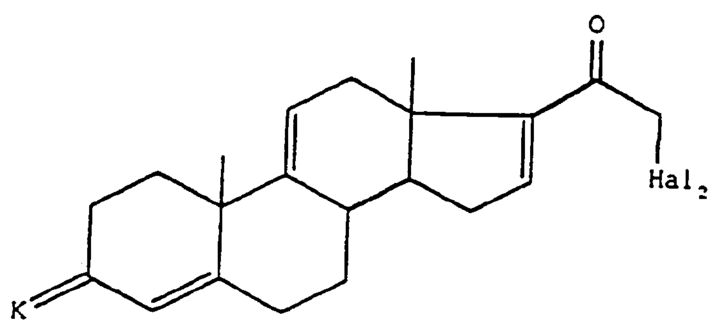

(XV)

in which $Hal_2$ represents a chlorine, bromine or iodine atom and K is defined as previously, which is subjected to the action of an acyloxylation agent in an alkaline medium, in order to obtain the compound of formula (XII) as defined above, then the synthesis is continued as described above,

- or by an alkaline sulphinate of formula $R_2SO_2Q$, as defined previously, in the presence of a base, in order to obtain a compound of formula (XIII):

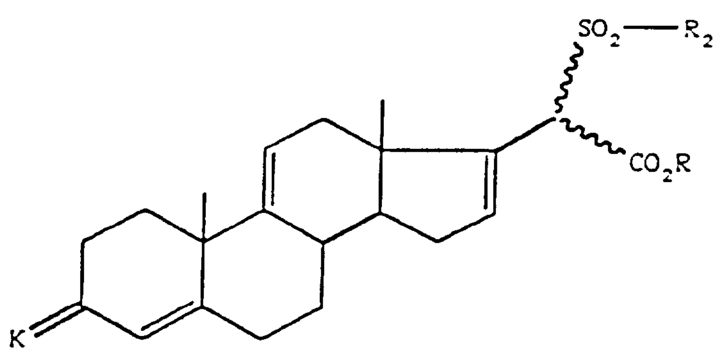

(XIII)

in which R, $R_2$ and K are defined as previously, which is treated with a halogenation agent in the presence of a base, then is saponified and decarboxylated in order to obtain a compound of formula (XIV):

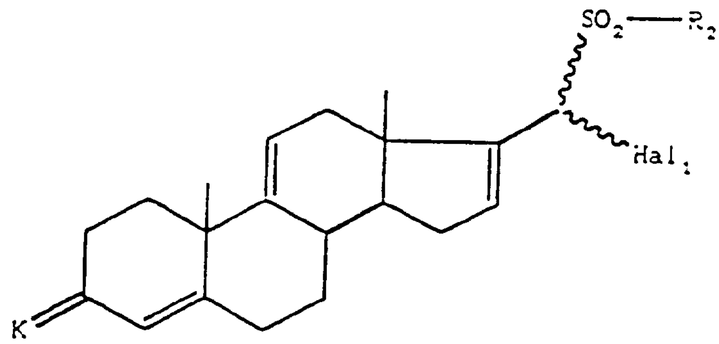

(XIV)

in which $Hal_1$ represents a halogen atom, which is treated with formaldehyde in the presence of a base, in order to obtain the compound of formula (XI) as defined above, which is treated as described above, in order to obtain the desired compound of formula (A),

- or by a reducing agent, in order to obtain a compound of formula (XVI):

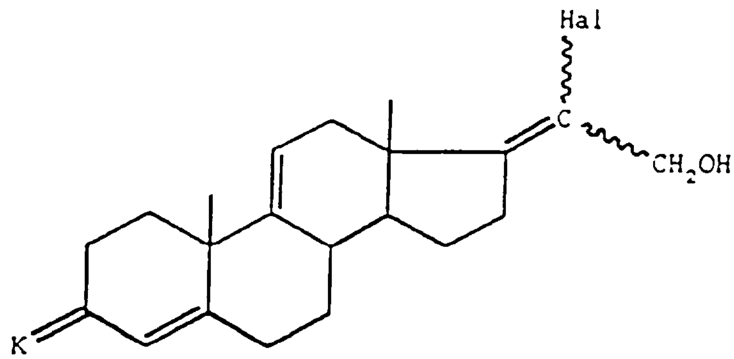

(XVI)

in which K and Hal are defined as in claim 1, which is subjected to the action of an oxidizing agent, in order to obtain a compound of formula (XVII):

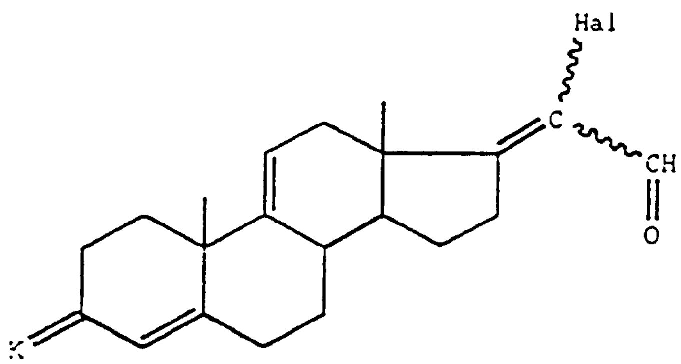

(XVII)

which is subjected to the action of an acyloxylation agent in order to obtain a compound of formula (XII) as defined above, then the synthesis is continued as described above in order to obtain the expected compound of formula (A).

**6.** Use according to claim 5, characterized in that:

- $R_a$ and $R_b$ represent a hydrogen atom, a hydroxy radical or a methyl radical,
- the acylation agent is acetic anhydride or acetyl chloride,
- the alkaline sulphinate is sodium toluenesulphinate,
- the opening of the epoxide is carried out in the presence of acetate ions,

- the alkaline halide is lithium bromide,
- the acyloxylation agent is potassium acetate,
- the halogenation agent used is sodium hypochlorite or hypobromite.

**7.** As industrial products:

- the compounds of formula (F):

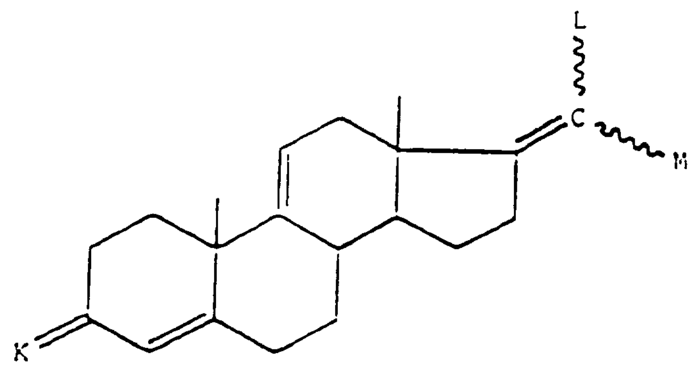

(F)

in which K is defined as in claim 1, and

either L represents a

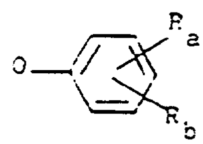

group,
$R_a$ and $R_b$, identical or different, representing a hydrogen atom, an alkyl radical containing 1 to 4 carbon atoms, an alkoxy radical containing 1 to 4 carbon atoms or a hydroxy radical, M represents a $CH_2OH$ or $CO_2R$ radical, R representing an alkyl radical containing 1 to 6 carbon atoms, an aralkyl radical containing 7 to 15 carbon atoms or a trialkylsilyl, triphenylsilyl or diphenyl tert-butyl silyl remainder,
or L represents a chlorine or bromine atom and M represents a $CH_2OH$ radical,
and the wavy lines symbolize any one of the isomer forms or their mixtures.

**8.** As industrial products, the products of formula (G):

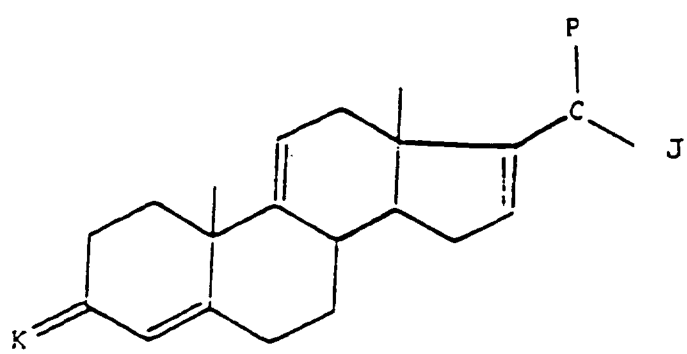

(G)

in which K is defined as in claim 1, the C-P group represents a $CH_2$, $C=CH_2$ or

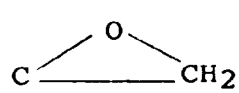

group and the C-J group represents a $C\text{-}SO_2R_2$ group, or the C-P group represents a

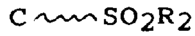

group and the C-J group represents a

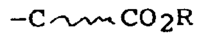

or C-Hal$_1$ group, $R_2$ representing a methyl, phenyl, tolyl or xylyl radical, R representing an alkyl radical containing 1 to 6 carbon atoms, an aralkyl radical containing 7 to 15 carbon atoms or a trialkylsilyl, triphenylsilyl or diphenyl tert-butyl silyl remainder, Hal$_1$ represents a halogen atom, notably a chlorine or bromine atom, the wavy lines symbolizing any one of the isomer forms or their mixtures.